# EUROPEAN PATENT SPECIFICATION

(11) **EP 2 013 183 B1**
(45) Date of publication and mention of the grant of the patent: **02.11.2011**
(21) Application number: 07724531.4
(22) Date of filing: 24.04.2007
(51) Int. Cl.: C07D 215/40, C07D 401/12, C07C 215/04, C07C 275/62, A61K 31/435, A61K 31/075

(54) **DERIVATIVES OF 4-(2-AMINO-1-HYDROXIETHYL)PHENOL AS AGONISTS OF THE BETA2 ADRENERGIC RECEPTOR**
DERIVATE VON 4-(2-AMINO-1-HYDROXYETHYL)PHENOL ALS AGONISTEN DES BETA2-ADRENERGEN REZEPTORS
DÉRIVÉS DE 4-(2-AMINO-1-HYDROXYÉTHYL)PHÉNOL EN TANT QU'AGONISTES DU RÉCEPTEUR BETA2 ADRÉNERGIQUE

(30) Priority: 27.04.2006 ES 200601082
(43) Date of publication of application: 14.01.2009
(73) Proprietor: Almirall, S.A., 08022 Barcelona (ES)
(72) Inventor: PUIG DURAN, Carlos, E-08024 Barcelona (ES); PRAT QUINONES, Maria, E-08003 Barcelona (ES); PEREZ CRESPO, Daniel, E-8024 Barcelona (ES)
(74) Representative: Srinivasan, Ravi Chandran
(86) International application number: PCT/EP2007/003601
(87) International publication number: WO 2007/124898

(56) References cited:
- WO-A-03/099764
- WO-A-2005/030678

## Description

### FIELD OF THE INVENTION

The present invention is directed to novel β2 adrenergic receptor agonists. The invention is also directed to pharmaceutical compositions comprising such compounds, the compounds for use in treating diseases associated with β2 adrenergic receptor activity, and processes and intermediates useful for preparing such compounds.

### BACKGROUND OF THE INVENTION

β2 adrenergic receptor agonists are recognized as effective drugs for the treatment of pulmonary diseases such as asthma and chronic obstructive pulmonary disease (including chronic bronchitis and emphysema). β2 adrenergic receptor agonists are also useful for treating pre-term labor, glaucoma and are potentially useful for treating neurological disorders and cardiac disorders.

In spite of the success that has been achieved with certain β2 adrenergic receptor agonists, current agents possess less than desirable potency, selectivity, onset, and/or duration of action. Thus, there is a need for additional β2 adrenergic receptor agonists having improved properties. Preferred agents may possess, among other properties, improved potency, selectivity, onset, improved safety margins, improved therapeutic window and/or duration of action.

WO 2005/030678 describes amino-substituted ethylamino B2 adrenergic receptor agonists. WO 03/099764 describes alkoxy aryl β₂ adrenergic receptor agonists.

### SUMMARY OF THE INVENTION

The invention provides novel compounds that possess β2 adrenergic receptor agonist activity. Accordingly, there is provided a compound of the invention which is a compound of formula (I): 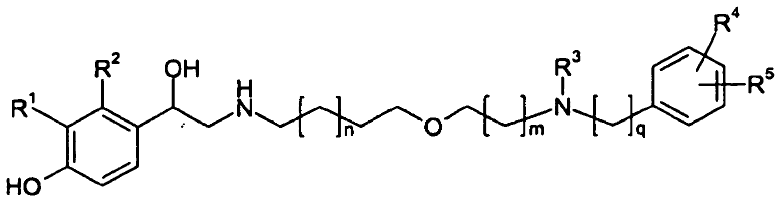 wherein:
- R¹ is a group selected from -CH₂OH, -NHC(O)H and
- R² is a hydrogen atom; or
- R¹ together with R² form the group -NH-C(O)-CH=CH- wherein the nitrogen atom is bound to the carbon atom in the phenyl ring holding R¹ and the carbon atom is bound to the carbon atom in the phenyl ring holding R²
- R³ is selected from hydrogen atoms and C₁₋₄alkyl groups
- R⁴ is selected from hydrogen and halogen atoms or groups selected from -SO-R⁶, -SO₂-R⁶, -NR⁷-CO-NHR⁸, -CO-NHR⁷, hydantoino, C₁₋₄alkyl, C₁₋₄alkoxy and -SO₂NR⁹R⁸
- R⁵ is selected from hydrogen atoms, halogen atoms and C₁₋₄alkyl groups
- R⁶ is a C₁₋₄alkyl group or C₃₋₈ cycloalkyl
- R⁷ is independently selected from hydrogen atoms and C₁₋₄alkyl groups
- R⁸ is independently selected from hydrogen atoms and C₁₋₄alkyl groups
- or R⁷ and R⁸ form the group -CH=CH-C(O)- wherein the carbon atom forming part of the ethylenic bond is bound to the nitrogen atom which is also bound to the carbon atom in the phenyl ring and the carbon atom of the carbonyl group is bound to the nitrogen atom which is bound to the hydrogen atom
- R⁹ is independently selected from hydrogen atoms and C₁₋₄alkyl groups
- m is 1 or 2
- n, is 0, 1, 2, 3 or 4
- q is 0, 1 or 2
or a pharmaceutically-acceptable salt or solvate or stereoisomer thereof.

The invention also provides a pharmaceutical composition comprising a therapeutically effective amount of a compound of the invention and a pharmaceutically-acceptable carrier and optionally further comprising a therapeutically effective amount of one or more other therapeutic agents.

The invention further provides combinations comprising a compound of the invention and one or more other therapeutic agents.

In separate and distinct aspects, the invention also provides synthetic processes and intermediates described herein, which are useful for preparing compounds of the invention.

The invention also provides a compound of the invention as described herein for use in treating a disease or pathological condition in a mammal associated with β2 adrenergic receptor activity (e.g. a pulmonary disease, such as asthma or chronic obstructive pulmonary disease, pre-term labor, glaucoma, a neurological disorder, a cardiac disorder, or inflammation) in a mammal.

The invention also provides use of a compound, composition or combination of the invention in the manufacture of a medicament for the treatment of a said disease or pathological condition.

### DETAILED DESCRIPTION OF THE INVENTION

When describing the compounds, compositions and methods of the invention, the following terms have the following meanings, unless otherwise indicated.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition associated with β2 adrenergic receptor activity" includes all disease states and/or conditions that are acknowledged now, or that are found in the future, to be associated with β2 adrenergic receptor activity. Such disease states include, but are not limited to, pulmonary diseases, such as asthma and chronic obstructive pulmonary disease (including chronic bronchitis and emphysema), as well as neurological disorders and cardiac disorders. β2 adrenergic receptor activity is also known to be associated with pre-term labor (see International Patent Application Publication Number WO 98/09632), glaucoma and some types of inflammation (see International Patent Application Publication Number WO 99/30703 and Patent Application Publication Number EP 1 078 629).

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, fumaric, gluconic, glutamic, hydrobromic, hydrochloric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

The term "solvate" refers to a complex or aggregate formed by one or more molecules of a solute, i.e. a compound of the invention or a pharmaceutically-acceptable salt thereof, and one or more molecules of a solvent. Such solvates are typically crystalline solids having a substantially fixed molar ratio of solute and solvent. Representative solvents include by way of example, water, methanol, ethanol, isopropanol, acetic acid, and the like. When the solvent is water, the solvate formed is a hydrate.

It will be appreciated that the term "or a pharmaceutically-acceptable salt or solvate of stereoisomer thereof" is intended to include all permutations of salts, solvates, and stereoisomers, such as a solvate of a pharmaceutically-acceptable salt of a stereoisomer of a compound of formula (I).

The term "amino-protecting group" refers to a protecting group suitable for preventing undesired reactions at an amino nitrogen. Representative amino-protecting groups include, but are not limited to, formyl; acyl groups, for example alkanoyl groups, such as acetyl; alkoxycarbonyl groups, such as tert-butoxycarbonyl (Boc); arylmethoxycarbonyl groups, such as benzyloxycarbonyl (Cbz) and 9-fluorenylmethoxycarbonyl (Fmoc); arylmethyl groups, such as benzyl (Bn), trityl (Tr), and 1,1-di-(4'-methoxyphenyl)methyl; silyl groups, such as trimethylsilyl (TMS) and tert-butyidimethylsilyl (TBS), and the like.

The term "hydroxy-protecting group" refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl; arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

The compounds of the invention contain at least a chiral center. Accordingly, the invention includes racemic mixtures, enantiomers, and mixtures enriched in one or more stereoisomer. The scope of the invention as described and claimed encompasses the racemic forms of the compounds as well as the individual enantiomers, diastereomers, and stereoisomer-enriched mixtures.

In an embodiment in formula (I) defining compounds of the present invention m+n has a value of 4, 5 or 6, more preferably 4 or 5.

In another embodiment in formula (I) defining compounds of the present invention m is 1.

In still another embodiment in formula (I) defining compounds of the present invention n is 3.

In still another embodiment in formula (I) defining compounds of the present invention q is 0 or 1, more preferably 1.

In still another embodiment in formula (I) defining compounds of the present invention R¹ is a group -CH₂OH and R² is a hydrogen atom; or R¹ together with R² form the group -NH-C(O)-CH=CH- wherein the nitrogen atom is bound to the carbon atom in the phenyl ring holding R¹ and the carbon atom is bound to the carbon atom in the phenyl ring holding R². In still another embodiment in formula (I) defining compounds of the present invention R³ is a methyl group.

In still another embodiment in formula (I) defining compounds of the present invention R⁴ is selected from halogen atoms or groups selected from -SO-R⁶, -SO₂-R⁶, -NR⁷-CO-NHR⁸, -CO-NHR⁷, hydantoino and -SO₂NR⁹R⁸ more preferably selected from -NH-CO-NH₂, -CO-NH₂ groups.

In still another embodiment in formula (I) defining compounds of the present invention the group R⁴ is in a meta position with respect to the rest

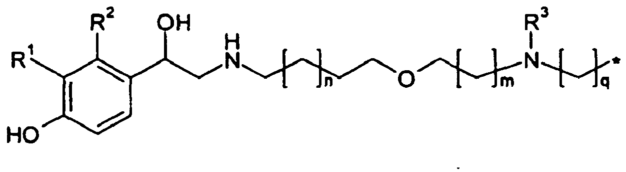

In still another embodiment in formula (I) defining compounds of the present invention R⁵ is a hydrogen atom.

Of particular interest are the compounds:
1. 3-[{2-[(6-{[2-hydroxy-2-(8-hydroxy-2-oxo-1,2-dihydroquinolin-5-yl)ethyl]amino}hexyl)oxy]ethyl}(methyl)amino]benzamide
2. 4-{2-[(6-{2-[(2,6-dichlorobenzyl)(methyl)amino]ethoxy}hexyl)amino]-1-hydroxyethyl}-2-(hydroxymethyl)phenol
3. 3-[(2-{[6-({2-hydroxy-2-[4-hydroxy-3-(hydroxymethyl)phenyl]ethyl}amino)hexyl]oxy}ethyl)(methyl) amino]benzamide
4. 5-{2-[(6-{2-[(2,6-dichlorobenzyl)(methyl)amino]ethoxy}hexyl)amino]-1-hydroxyethyl}-8-hydroxyquinolin-2(1H)-one
5. N-(3-{[{2-[(6-{[2-hydroxy-2-(8-hydroxy-2-oxo-1,2-dihydroquinolin-5-yl)ethyl]amino}hexyl)oxy]ethyl}(methyl)amino]methyl} phenyl)urea
6. N-(3-{[{2-[(6-{[(2R)-2-hydroxy-2-(8-hydroxy-2-oxo-1,2-dihydroquinolin-5-yl)ethyl]amino}hexyl)oxy]ethyl}(methyl)amino]methyl} phenyl)urea
7. N-(3-{[(2-{[6-({2-hydroxy-2-[4-hydroxy-3-(hydroxymethyl)phenyl]ethyl}amino)hexyl]oxy}ethyl)(methyl)amino]methyl}p henyl)urea
8. 4-{2-[(6-{3-[(2,6-dichlorobenzyl)(methyl)amino]propoxy}hexyl)amino]-1-hydroxyethyl}-2-(hydroxymethyl)phenol
9. 3-[(3-{[6-({2-hydroxy-2-[4-hydroxy-3-(hydroxymethyl)phenyl]ethyl}amino) hexyl]oxy}propyl)(methyl)amino]benzamide
10. 3-[(3-{[6-({(2R)-2-hydroxy-2-[4-hydroxy-3-(hydroxymethyl)phenyl]ethyl}amino)hexyl]oxy}propyl)(methyl)amino]benza mide
11. 3-{[{2-[(6-{[2-hydroxy-2-(8-hydroxy-2-oxo-1,2-dihydroquinolin-5-yl)ethyl]amino}hexyl)oxy]ethyl}(methyl)amino]methyl}benzamide
12. 3-{[(2-{[6-({2-hydroxy-2-[4-hydroxy-3-(hydroxymethyl)phenyl]ethyl}amino)hexyl]oxy}ethyl)(methyl)amino]methyl}b enzamide
13. 1-(3-{[{2-[(6-{[2-hydroxy-2-(8-hydroxy-2-oxo-1,2-dihydroquinolin-5-yl)ethyl]amino}hexyl)oxy]ethyl}(methyl)amino]methyl}phenyl)pyrimidine-2,4(1H,3H)-dione
14. 5-{(1R)-2-[(6-{2-[[3-(Cyclopentylsulfonyl)benzyl](methyl)amino]ethoxy}hexyl)amino]-1-hydroxyethyl}-8-hydroxyquinolin-2(1H)-one
15. 5-[2-({6-[2-(benzylamino)ethoxy]hexyl}amino)-1-hydroxyethyl]-8-hydroxyquinolin-2(1H)-one
16. 3-[(3-{[6-({(2R)-2-[3-(Formylamino)-4-hydroxyphenyl]-2-hydroxyethyl}amino)hexyl]oxy}propyl)(methyl)amino]benzamide
17. 4-[2-({6-[2-(Benzylamino)ethoxy]hexyl}amino)-1-hydroxyethyl]-2-(hydroxymethyl)phenol
18. 1-(3-{[{2-[(6-{[(2R)-2-hydroxy-2-(8-hydroxy-2-oxo-1,2-dihydroquinolin-5-yl)ethyl]amino}hexyl)oxy]ethyl}(methyl)amino]methyl}phenyl)pyrimidine-2,4(1H,3H)-dione
19. N-(tert-Butyl)-3-{[{2-[(6-{[(2R)-2-hydroxy-2-(8-hydroxy-2-oxo-1,2-dihydroquinolin-5-yl)ethyl]amino}hexyl)oxy]ethyl}(methyl)amino]methyl}benzenesulfonamide
20. 8-Hydroxy-5-{(1R)-1-hydroxy-2-[(5-{2-[methyl(2-phenylethyl)amino]ethoxy}pentyl)amino]ethyl}quinolin-2(1H)-one
21. 5-{(1R)-2-[(6-{2-[[3-(Cyclopentylsulfinyl)benzyl](methyl)amino]ethoxy}hexyl)amino]-1-hydroxyethyl}-8-hydroxyquinolin-2(1H)-one
22. 3-(3-{[(2-{[6-({(2R)-2-Hydroxy-2-[4-hydroxy-3-(hydroxymethyl)phenyl]ethyl}amino)hexyl]oxy}ethyl)(methyl)amino]methyl}p henyl)imidazolidine-2,4-dione
and pharmaceutically-acceptable salts and solvates thereof.

In one aspect the invention comprises also pharmaceutical compositions comprising a therapeutically effective amount of a compound as hereinabove defined and a pharmaceutically acceptable carrier.

In an embodiment of the present invention the pharmaceutical composition further comprises a therapeutically effective amount of one or more other therapeutic agents. Among the therapeutically agents it is preferred to use corticosteroids, antichlolinergic agents, or a PDE4 inhibitors.

It is also an embodiment of the present invention that the pharmaceutical composition is formulated for administration by inhalation.

The compounds of the present invention as hereinabove defined may also be combined with one or more other therapeutic agents, in particular one or more drugs selected from the group consisting of corticosteroids, an antichlolinergic agents and PDE4 inhibitors.

In a preferred embodiment of the present invention the combination comprises a compound of formula (I) as hereinabove defined and a drug selected from the group consisting of fluticasone propionate, 6α,9α-difluoro-17α-[-(2-furanylcarbonyl)oxy]-11β-hydroxy-16α-methyl-3-oxo-androsta-1,4-diene-17β-carbothioic acid S-fluoromethyl ester, and 6α,9α-difluoro-11β-hydroxy-16α-methyl-3-oxo-17.alpha.-propionyloxy-androsta-1,4-diene-17β-carbothioic acid S-(2-oxo-tetrahydro-furan-3S-yl) ester.

### General Synthetic Procedures

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal, are described in T. W. Greene and G. M. Wuts, Protecting Groups in Organic Synthesis, Third Edition, Wiley, New York, 1999, and references cited therein.

Processes for preparing compounds of the invention are provided as further embodiments of the invention and are illustrated by the procedures below.

In general the compounds of formula (I) are obtained following the synthetic route shown in Scheme 1

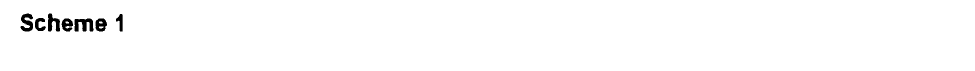

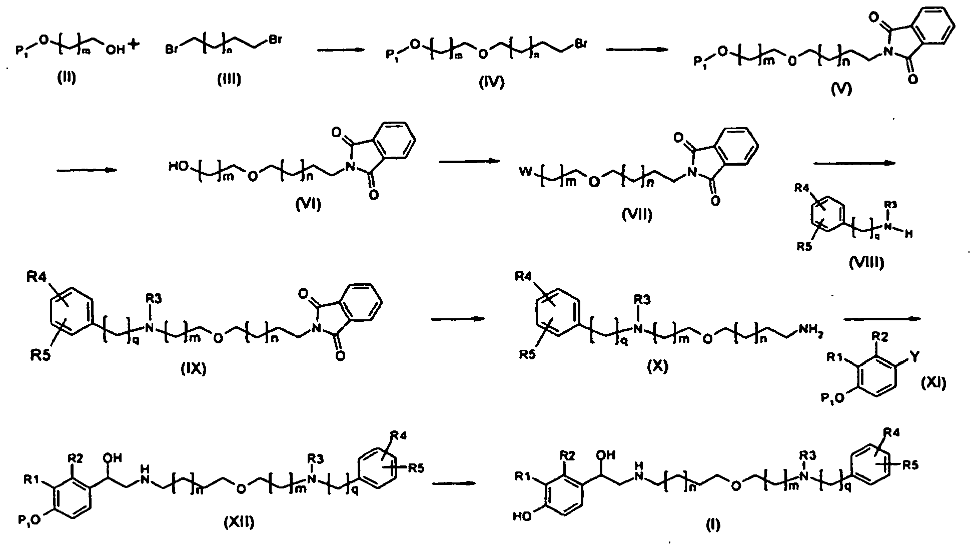

Compounds of formula (IV) may be obtained by reaction of alcohols of formula (II) (where P₁ is a protecting group such as, for example, a benzyl group) with dibromoderivatives of formula (III). The reaction may be carried out with a base such as sodium hydroxide, potassium hydroxide or sodium hydride, optionally in the presence of a phase transfer catalyst such as tetrabutylammonium bromide, with a solvent such as water, dimethylformamide, dimethylsulfoxide or diethylene glycol dimethyl ether, and at a temperature from 20° to 100°C.

Bromoderivatives of formula (IV) may be reacted with potassium phtalimide to give compounds of formula (V). The reaction may be carried out in a solvent such as dimethylformamide, dimethylsulfoxide, acetonitrile or tetrahydrofuran, optionally with a catalyst such as (n-hexadecyl)tri-n-butylphosphonium bromide, and at a temperature from room temperature to the boiling point of the solvent.

Removal of the protecting group P₁ from the derivatives of formula (V) yields the compounds of formula (VI). When the protecting group P¹ is a benzyl group the debenzylation is carried out with hydrogen and a hydrogenation catalyst like palladium on charcoal. This step is achieved using a variety of solvents such as lower alcohols, and in neutral or slightly acidic media. The pressure of hydrogen may conveniently lie between 0,067 and 0,28 MPa and the temperature between 10° and 30°C.

In the next step, the alcohol functionality of compound (VI) is transformed into a good leaving group W (compound VII) such as a methanesulphonate group. This may be achieved by reaction with methanesulfonyl chloride, in the presence of a base such as triethylamine, diisopropylethylamine or pyridine, with a solvent such as methylene chloride, chloroform or tetrahydrofuran, and at a temperature from 0°C to the boiling point of the solvent.

Compound (VII) is then reacted with the corresponding amine (VIII) to give compounds (IX). This reaction may be carried out in the presence of an acid scavenger, such as a tertiary amine or sodium bicarbonate, in a variety of solvents such as dioxane, DMF, dimethylsulfoxide or also without solvent, in a range of temperatures between 60° and 140° C. At this stage it is possible to modify if necessary the substituent R⁴ on the terminal phenyl group. One example of such a modification is the conversion of a group - S-R⁶ into a group -SO-R⁶. Such a conversion may be carried out in a solvent such as methanol by addition of an oxidising agent such as an IO₄Na aqueous solution.

The reaction of compounds of formula (IX) with hydrazine in a solvent such as methanol, ethanol, isopropyl alcohol or tetrahydrofuran, and at a temperature from room temperature to the boiling point of the solvent gives amines of formula (X).

These amines (X) are coupled with the phenylethanol moiety by means of an intermediate (XI), where Y represents a protected bromohydrine group (-CHOP₂CH₂Br; wherein P₂ is an oxygen-protecting group such as a silyl ether), a glyoxal group (-COCHO), or an oxyrane group ( 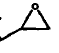 ). The conditions for the reaction depend on the nature of the group Y. Thus, for the bromohydrine, the reaction may be carried out in the presence of an acid scavenger, such as a tertiary amine or sodium bicarbonate, in a variety of solvents such as dioxane, dimethylsulfoxyde or also without solvent, in a range of temperatures between 60 and 140° C. The removal of the protecting group P₂, usually a silyl ether, is effected by means of the fluoride anion, for example in the form of a quaternary ammonium salt like tetrabutylammonium fluoride, to give the intermediate (XII). For the glyoxals the reaction consists of a reductive alkylation process This step may be effected in a variety of solvents, such as THF, alcohols such as methanol, ethanol or isopropyl alcohol, as well as mixtures of solvents such as methanol/THF or dimethylsulfoxyde/methanol, the temperature range being between 5 and 100° C; more specifically between 15 and 70° C.

The reducing agent may be a hydride such as sodium borohydride or cyanoborohydride as well as hydrogen plus a hydrogenation catalyst such as palladium on carbon. When the intermediate (XI) is an oxyrane, the reaction may be carried out in many solvents such as alcohols, tetrahydrofurane or without solvents at all, in a range of temperatures between 20 and 140 °C.

The intermediate (XII) is deprotected to the target compound (I) by conventional methods. If the protecting group P₁ is a benzyl group the debenzylation is carried out with hydrogen and a hydrogenation catalyst such as palladium on carbon. This step is carried out using a variety of solvents such as alcohols, THF or mixtures of both in neutral or slightly acidic media. The pressure of hydrogen lies between 0,067 and 0,28 MPa and the temperature between 10 and 30°C. If R₁ together with P₁ form the acetonyl group (when R₁ = CH₂OH) the deprotection is effected in acidic media, for example by treatment with acetic acid in water at a temperature between 60 and 80°C.

An alternative route for the preparation of the compounds of formula (I) is depicted iln synthetic scheme 2

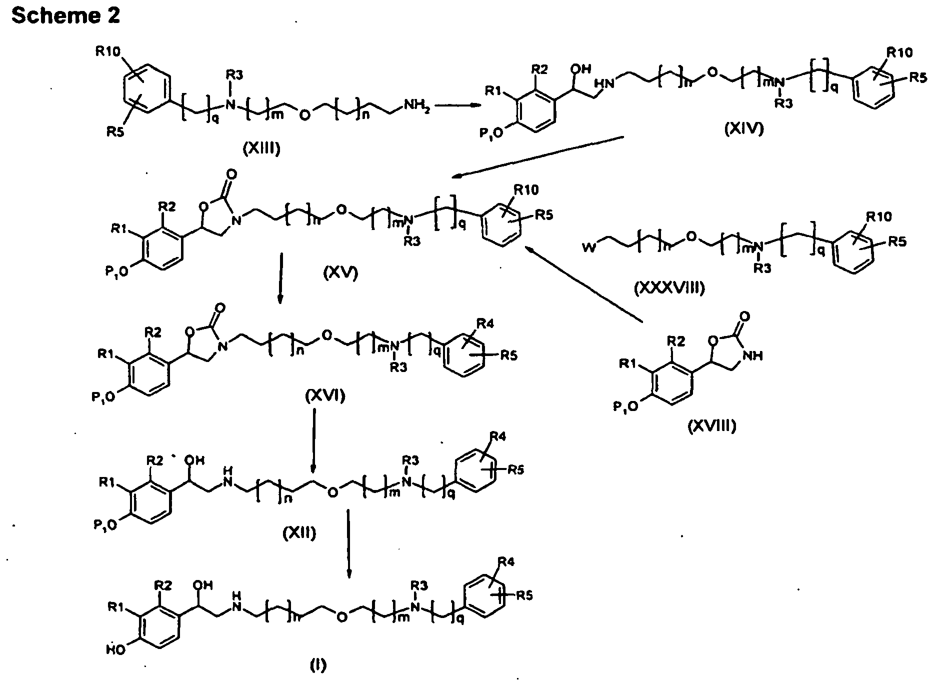

The intermediates (XIII) and (XIV) are prepared in a similar way to that described for the homologous compounds (X) and (XII) in synthetic scheme 1.

The amino alcohol moiety of (XIV) is then protected to yield compound (XV) by means of an oxazolidinone group. The introduction of such protecting group may be effected by treatment of (XIV) with an acylating agent such as 1,1'-carbonyldiimidazole. This reaction may be carried out in a variety of solvents, such us THF, dichloromethane or chloroform, with the optional addition of a tertiary amine such as triethylamine, in a range of temperatures from room temperature to the boiling point.

Alternatively, the compound of formula (XV) may be obtained by alkylation of the oxazolidinones (XVIII) with the intermediates (XXXVIII), which are in turn prepared in a similar way as compounds (XXXVII) in Scheme 6.

This alkylation step may be carried out in a variety of solvents, preferably polar aprotic ones such as DMF, by generating first the anion by treatment with a base, preferably sodium hydride.

The reaction of the compounds of formula (XV) to yield the compounds of formula (XVI) involves a transformation of the phenyl substituent group R₁₀ into a group R₄ and may involve, for example, the reduction of a nitro group and subsequent transformation of the corresponding aniline to an urea group.

The deprotection of the oxazolidinone (XVI) to give the intermediate (XII) may be carried out both in alkaline (with, for example, potassium trimethylsilanolate in THF or THF/DMSO) or acidic (with, for ex., dilute hydrochloric acid with alcohols) media in a wide range of temperatures from 0 to 100°C.

The conversion of compound (XII) into the final compound (I) is performed under the same condition explained for the last step of the synthetic scheme I.

Scheme 3 shows an alternative approach to the preparation of compounds (I).

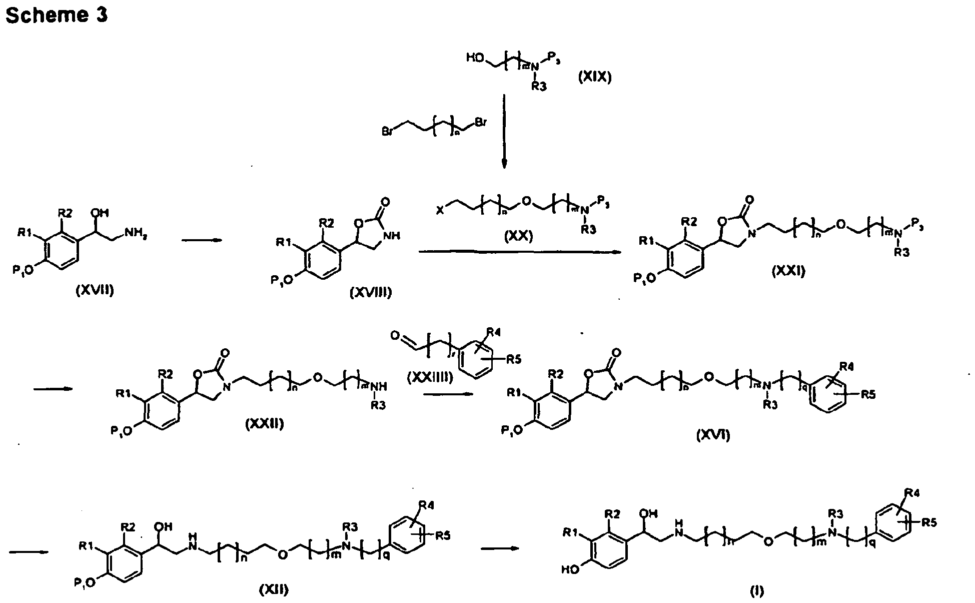

It starts from the oxazolidinones (XVIII), which are, in turn, prepared from the amino alcohols (XVII) described in the literature (for ex., see J. Med. Chem., 1976, 19(9), 1138, compound 19; DE 2461861, example 24). This synthesis can be carried out in a single step, by treatment with 1,1'-carbonyldiimidazole (in a manner analogue to that described for the preparation of (XV) from (XIV) in scheme 2); or in a two step sequence involving first the conversion of the amine (XVII) into the corresponding BOC derivative, then the treatment of this BOC derivative with sodium hydride (as described in WO 02/066422, example 1, part iv).

The oxazolidinones (XVIII) are alkylated with the intermediates (XX), which are in turn prepared from the protected aminoalcohols (XIX) by alkylation with α,ω-alkyldibromides in a similar way to that described for the intermediates (IV) of scheme 1.

The synthesis of (XXI) may be carried out in a variety of solvents, preferably polar aprotic ones such as DMF, by generating first the anion by treatment with a base, preferably sodium hydride.

The removal of the protecting group P₃ of (XXI) can be effected in a variety of ways, such as hydrogenation with a catalyst like palladium on charcoal if P₃ is a benzyl group, the process being carried out in a variety of solvents such as THF, alcohols o mixtures thereof in a range of temperatures covering from room temperature to 60°C. When P₃ is a BOC group it may also be removed by acidic hydrolysis, for example by treatment of (XI) with trifluoroacetic acid in a solvent such as dichloromethane at room temperature.

The preparation of the intermediate (XVI) from (XXII) involves a reductive alkylation step using the aldehyde (XXIII) as carbonyl component. This step is carried out in a solvent such as alcohols, THF or mixtures thereof, using as reductive agent a hydride such as sodium borohydride or sodium cyanoborohydride or by hydrogenation using a catalyst such as palladium on charcoal, usually at room temperature.

The transformation of the oxazolidinone (XVI) to the intermediate (XII) can be effected by acidic hydrolysis, for example, by treatment with hydrogen chloride in a solvent like dioxane or THF in a range of temperatures from 0 to 50°C.

The conversion of compound (XII) into the final compound (I) is performed under the same condition explained for the last step of the synthetic scheme 1.

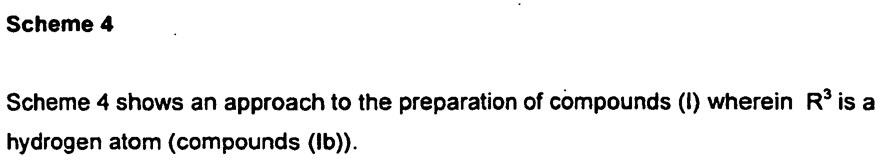

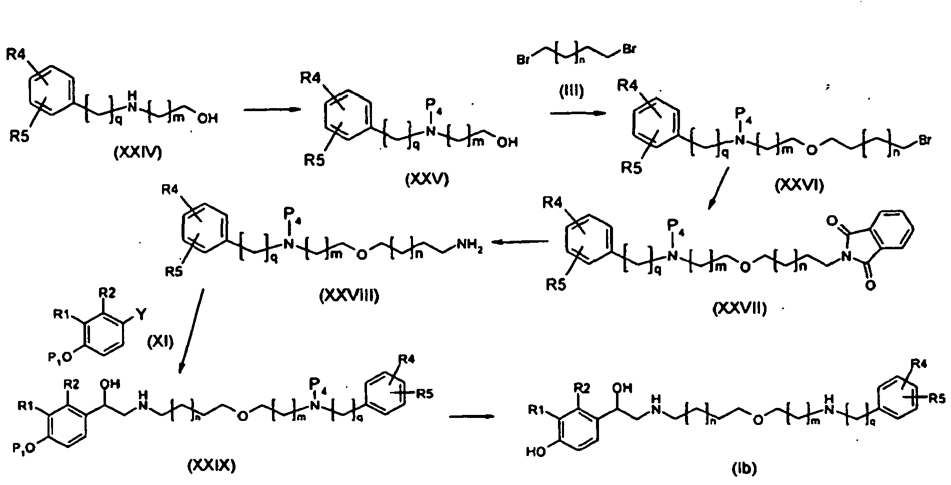

Aminoalcohols (XXIV) are protected with a protecting group P₄ group, which may be an acid-labile protecting group such as the BOC group. This transformation can be effected in a conventional way by treatment of (XXIV) with diterbutyl dicarbonate in neutral or basic media using a solvent like THF or dioxane at a range of temperatures between -10°C and room temperature.

The resulting alcohols (XXV) may be alkylated with dibromo derivatives (III) using similar conditions to that described for the preparation of compounds (IV) in Synthetic Scheme 1.

Bromoderivatives of formula (XXVI) may be reacted with potassium phtalimide to give compounds of formula (XVII). The reaction may be carried out in a solvent such as dimethylformamide, dimethylsulfoxide, acetonitrile or tetrahydrofuran, optionally with a catalyst such as (n-hexadecyl)tri-n-butylphosphonium bromide, and at a temperature from room temperature to the boiling point of the solvent.

The reaction of compounds of formula (XXVII) with hydrazine in a solvent such as methanol, ethanol, isopropyl alcohol or tetrahydrofuran, and at a temperature from room temperature to the boiling of the solvent gives amines of formula (XXVIII).

These amines (XXVIII) are coupled with the phenylethanol moiety by means of an intermediate (XI), where Y has the same description as shown in Scheme 1. The conditions for the reaction depend on the nature of the group Y. Thus, for the bromohydrine, the reaction may be carried out in the presence of an acid scavenger, such as a tertiary amine or sodium bicarbonate, in a variety of solvents such as dioxane, dimethylsulfoxyde or also without solvent, in a range of temperatures between 60 and 140° C. The removal of the protecting group P₂, usually a silyl ether, is effected by means of the fluoride anion, for example in the form of a quaternary ammonium salt like tetrabutylammonium fluoride, to give the intermediate (XXIX). For the glyoxals the reaction consists of a reductive alkylation process This step may be effected in a variety of solvents, such as THF, alcohols such as methanol, ethanol or isopropyl alcohol, as well as mixtures of solvents such as methanol/THF or dimethylsulfoxyde/methanol, the temperature range being between 5 and 100° C; more specifically between 15 and 70° C. The reducing agent may be a hydride such as sodium borohydride or cyanoborohydride as well as hydrogen plus a hydrogenation catalyst such as palladium on carbon. When the intermediate (XI) is an oxyrane, the reaction may be carried out in many solvents such as alcohols, tetrahydrofurane or without solvents at all, in a range of temperatures between 20 and 140 °C.

The removal of the protecting groups P₁ and P₄ to the final compounds (Ib) is effected according to the nature of both groups and can be run in the same or in separated synthetic steps. If both groups are easily removed in acidic medium, as is the case when P₁ is a 4-methoxybenzyl or acetonyl group (when R₁ = CH₂OH) and P₄ is a BOC group, the treatment with a strong acid like trifluoroacetic or hydrochloric acid without or with a solvent like dichloromethane at room temperature gives the target structures (Ib). 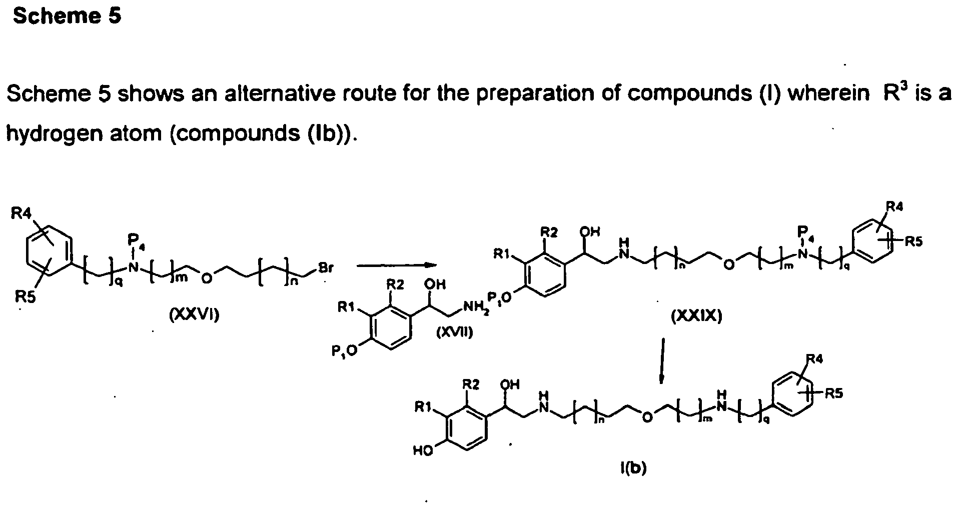 Intermediates of formula (XXIX) may be obtained by reaction of a bromoderivative of formula (XXVI) with an Intermediate of formula (XVII) in the presence of a base, such as a tertiary amine, NaHCO₃ or K₂CO₃, in a variety of solvents such as dioxane, acetonitrile, THF, DMF, DMSO, or also without solvent, in a range of temperatures between room temperature and 140°C. The removal of the protecting groups P₁ and P₄ is effected according to the procedures described in Scheme 4.

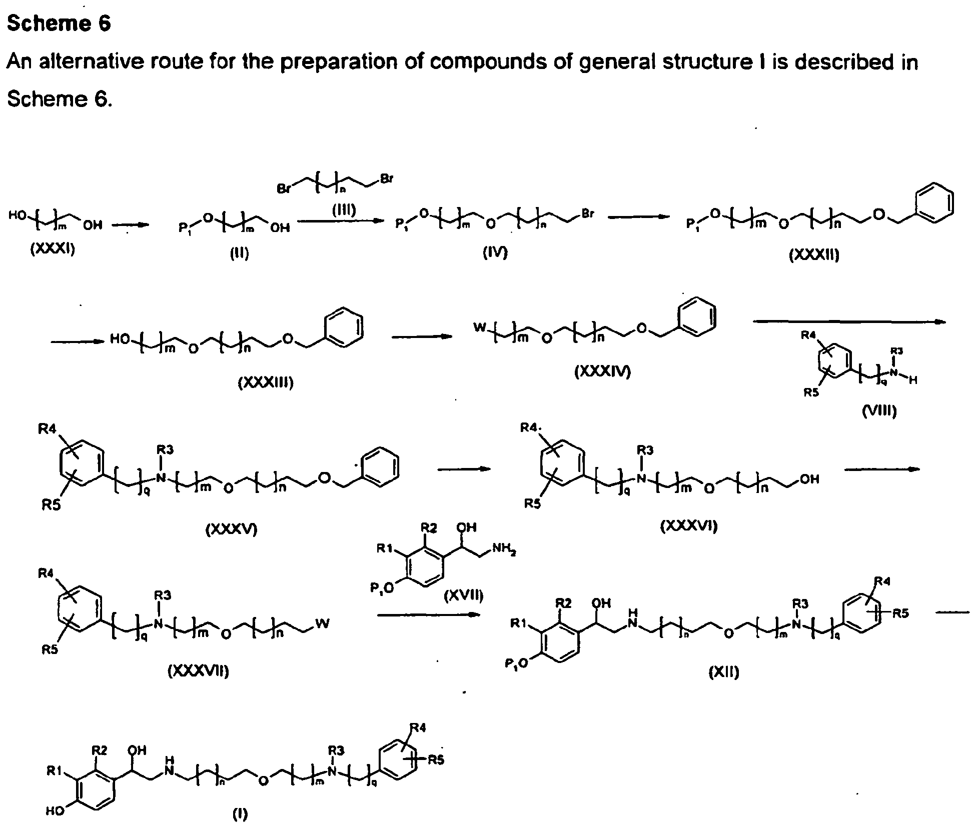

The alcohols of formula (II) may be obtained by reaction of a diol of formula (XXXI) with a sililating agent (for example ter-butylchlorodimethylsilane) previous treatment with a base like sodium hydride in a solvent like dioxane or tetrahydrofuran, usually at room temperature.

Compounds of formula (IV) where P₁ is a silyl protecting group (for example a tert-butyldimethylsilyl group) may be obtained by reaction of the corresponding alcohols of formula (II) with dibromoderivatives of formula (III) using similar procedures as those described in synthetic Scheme 1.

Compounds of formula (XXXII) may be obtained by reaction of bromoderivatives of formula (IV) with benzyl alcohol. The reaction may be carried out in the presence of a base such as sodium hydroxide, potassium hydroxide or sodium hydride, optionally in the presence of a phase transfer catalyst such as tetrabutylammonium bromide, in a solvent like water, dimethylformamide or tetrahydrofuran, and at a temperature from 20°C to 100°C.
The removal of the protecting group P₁ to obtain the intermediates of formula (XXXIII), is effected using tetrabutylammonium fluoride in a solvent such as THF at a temperature between room temperature and 50°C.

In the next step, the alcohol functionality is transformed into a suitable leaving group W (compounds XXXIV) such as the methanesulphonate group. This is achieved by reaction with methanesulfonyl chloride, in the presence of a base such as triethylamine or diisopropylethylamine, in a solvent such as methylene chloride, chloroform or tetrahydrofuran, and at a temperature from 0°C to the boiling point of the solvent.

In the next step the intermediates of formula (XXXIV) are reacted with the corresponding amine (VIII), in the presence of an acid scavenger such as triethylamine or NaHCO₃, in a variety of solvents like dimethylformamide, dimethylsulfoxide, acetonitrile, dioxane or tetrahydrofurane or also without solvent in a range of temperatures between room temperature and 140°C.

The debenzylation of intermediates of formula (XXXV) may be carried out with hydrogen and a hydrogenation catalyst like palladium on charcoal. This step may be carried out using a lower alcohol as a solvent in acidic media. The temperature of the reaction is usually 20°C-30°C.

The alcohol functionality of intermediates of formula (XXXVI) is transformed into a suitable leaving group W, for example a methanesulphonate group. This is achivied by using similar reaction conditions as described for compounds (XXXIV).

The compounds of formula (XXXVII) are coupled with the phenylethanol moiety by means of an intermediate (XVII). This step may be carried out in a variety of conditions, for example as is described in Scheme 5 for the reaction of compounds of formula (XXVI) with compounds of formula (XVII), or optionally substituting the base for a catalyst such as tetrabutylammonium bromide, in a range of temperatures between room temperature to the boiling point of the solvent.

The intermediates of formula (XII) are deprotected to yield the target compounds (I) by conventional methods as is described in the synthetic Scheme 1.

### EXAMPLES

**General.** Reagents, starting materials, and solvents were purchased from commercial suppliers and used as received. Concentration refers to evaporation under vacuum using a Büchi rotatory evaporator. Reaction products were purified, when necessary, by flash chromatography on silica gel (40-63 µm) with the solvent system indicated. Spectroscopic data were recorded on a Varian Gemini 300 spectrometer and a Varian Inova 400 spectrometer. Melting points were recorded on a Büchi 535 apparatus.

### Intermediate 1

### ({2-[(6-bromohexyl)oxy]ethoxy}methyl)benzene.

A mixture of 31.4 g-(206.3 mmol) of 2-benzyloxyethanol, 99.8 ml of 1,6-dibromohexane, 78.3 mi of 50 % w/v NaOH and 1.3 g of tetrabutylammonium bromide is vigorously stirred for 5 hr at room temperature. Excess of water and hexane are added and the organic phase is washed thoroughly with water, dried over magnesium sulphate and concentrated. The residue is distilled at 0.2 mm Hg giving a first fraction, b.p.: 50-70°C consisting mainly of dibromohexane, and a second one, b.p.: 125-140°C, consisting in the title compound (50.4 g; 78%).

### Intermediate 2

### 2-{6-[2-(benzyloxy)ethoxy]hexyl}-1H-isoindole-1,3(2H)-dione.

A mixture of 50.4 g (159.8 mmol) of the Intermediate, 34.0 g (183.5 mmol) of potassium phtalimide and 0.1 g of (N-hexadecyl)tri-N-butylphosphonium bromide in 230 ml of dimethylformamide is stirred at 75°C for 3 hr. The solvent is evaporated in vacuo and the residue fractionated in ethyl ether/water. The organic layer is washed with water, dried over magnesium sulfate and concentrated giving 59.9 g (98%) of the title compound (95% content by HPLC).

### Intermediate 3

### 2-[6-(2-hydroxyethoxy)hexyl]-1H-isoindole-1,3(2H)-dione.

3.0 g of 10 % palladium on charcoal catalyst is added to a solution of 59.9 g (157.0 mmol) of the Intermediate 2 in 500 ml methanol, and the whole is hydrogenated at 0,276 MPa for 2 hr at room temperature. The catalyst is filtered and the solvent eliminated in vacuo giving 45.4 g (99%) of the title compound as a colorless oil.

### Intermediate 4

### 2-{[6-(1,3-dioxo-1,3-dihydro-2H-isoindol-2-yl)hexyl]oxy}ethyl methanesulfonate.

To a stirred solution of 20.0 g (68.6 mmol)of Intermediate 3 and 10.5 ml (7.5 g; 74.7 mmol) of triethylamine is added dropwise a solution of 5.56 ml (8.2 g; 71.8 mmol) of methanesulphonyl chloride in 80 ml of dichloromethane. After stirring overnight at room temperature the solution is washed successively with water, 4 % sodium bicarbonate and water. The solution is dried over magnesium sulphate and concentrated, giving 24.4 g (96%) of the title compound as a colorless oil.

### Intermediate 5

### 3-[(2-{[6-(1,3-dioxo-1,3-dihydro-2H-isoindol-2-yl)hexyl]oxy}ethyl)(methyl)amino] benzamide.

A mixture of 1.24 g (3.35 mmol) of Intermediate 4, 0.75 g (5.00 mmol) of 3-(methylamino)benzamide and 1.15 ml (0.853 g; 6.6 mmol) of diisopropylethylamine is stirred at 120°C overnight. The residue is partitioned between a mixture of ethyl acetate/dichloromethane 9/1 and water. The organic layer is washed successively with 1 % citric acid and water, and is dried and concentrated giving 1.2 g of the title compound pure enough to continue with the next step.

### Intermediate 6

### 3-[{2-[(6-aminohexyl)oxy]ethyl}(methyl)amino]benzamide.

A mixture of 1.2 g (2.8 mmol) of the Intermediate 5 and 0.17 ml (0.17 g; 8.7 mmol) of hydrazine monohydrate in 12 ml of ethanol is stirred 16 hr at room temperature. An additional quantity of 0.042 ml of hydrazine is added and the stirring prosecuted for 6 hr at room temperature. The solvent is evaporated in vacuo and the residue purified by column chromatography eluting with dichloromethane/methanol/aq.ammonia 40/811, yielding 0.33 g (39%) of the title compound.

### Intermediate 7

### 8-(benzyloxy)-5-(2-bromo-1-hydroxyethyl)quinolin-2(1H)-one

A suspension of 5.0 g (13.4 mmol) of 8-(benzyloxy)-5-(2-bromo-1-hydroxyethyl)quinolin-2(1H)-one (see, for example, US 20040059116) in a mixture of 50 ml of tetrahydrofuran and 50 ml of methanol is stirred in an ice/water bath whilst adding, in small portions, 0.5 g (13.21 mmol) of sodium borohydride. The stirring with the ice bath is prosecuted for 30' and then 200 ml of 0.5 N hydrochloric acid is added dropwise slowly whilst maintaining the ice bath. After 1 hr the solid is filtered, washed with water and dried in a desiccator. Yield: 4.5 g (89%) of the title compound.

### Intermediate 8

### 8-(benzyloxy)-5-(2-bromo-1-{[tert-butyl(dimethyl)silyl]oxy}ethyl)quinolin-2(1H)-one

A solution of 2.48 g (16.4 mmol) of dimethylterbutylsilyl chloride in 15 ml of dimethylformamide is added dropwise into a stirred solution of 3.08 g (8.2 mmol) of the Intermediate 7 and 1.12 g (16.4 mmol) of imidazole in 30.8 ml of dimethylformamide. After a 24 hr stirring at room temperature a 50 % excess of imidazole and chlorosilane are added and the whole is stirred for 48 additional hours. 135 ml of cyclohexane and 45 ml of water are added and the organic layer is washed several times with water, filtered, dried and concentrated. The excess of chlorosilane is eliminated in a vacuum pump at 70°C for 2-3 hr. The resulting solid is treated with a little cold heptane and filtered, giving 3.4 g (85%) of the title product as a low melting point solid.

### Intermediate 9

### 3-[(2-{[6-({2-[8-(benzyloxy)-2-oxo-1,2-dihydroquinolin-5-yl]-2-hydroxyethyl}amino) hexyl]oxy}ethyl)(methyl)amino]benzamide.

A mixture of 0.55 g (1.12 mmol) of the Intermediate 8, 0.33 g (1.12 mmol) of the Intermediate 6, 0.25 g of sodium iodide and 0.29 g of sodium hydrogencarbonate in 1.25 ml of dimethylsulphoxyde is stirred at 140°C in a nitrogen atmosphere for 2 hr. The residue is taken in ethyl acetate/tetrahydrofuran 9/1 and water, the organic layer washed with water, dried and concentrated giving 0.8 g. This residue is dissolved in 8 ml of tetrahydrofuran and 0.64 g (2.45 mmol) of tetrabutylammonium fluoride are added. After 16 hr of stirring at room temperature, excess of ethyl acetate and water are added. The organic layer is washed with water, dried and concentrated. The residue is purified through column chromatography eluting with dichloromethane/methanol/aq. ammonia 40/4/0.2 giving 0.11 g (16%) of the title compound.

### Example 1

### 3-[{2-[(6-{[2-hydroxy-2-(8-hydroxy-2-oxo-1,2-dihydroquinolin-5-yl)ethyl]amino}hexyl) oxy]ethyl}(methyl)amino]benzamide

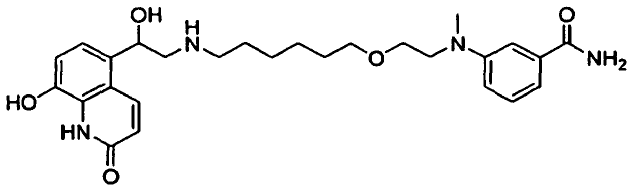

0.07 g of 10 % palladium on charcoal catalyst are added to a solution of 0.11 g (0.18 mmol) of the Intermediate 9 in 11 ml of methanol. The whole is stirred in a hydrogen atmosphere for 16 hr at room temperature. The catalyst is filtered off and the solution is concentrated. The residue is purified by column chromatography eluting with dichloromethane/methanol/aq ammonia 40/8/1 giving 0.026 g (27%) of the title compound.

¹H-NMR (d₆-DMsO): 1.07-1.44 m (8H); 2.59-2.74 m (2H); 2.93 s (3H); 3.32-3.64 m (8H); 6.39-6.52 m (2H); 6.81-6.87 m (2H); 7.11-7.26 m (4H); 7.93 s (1H); 8.14-8.17 m (2H).

### Intermediate 10

### (2,6-Dichloro-benzyl)-methyl-amine

17.5 ml (128 mmol) of a 33 % w/v solution of methylamine in ethanol is added dropwise into a stirred solution of 3.0 g (15.7 mmol) of 2,6-dichlorobenzyl bromide in 10 ml of ethanol. The whole is refluxed smoothly in a closed atmosphere for 4 hr. After concentrating, the residue is partitioned between ethyl ether/water. The organic layer is successively extracted with 2N hydrochloric acid, washed with ethyl ether, basified with 2N NaOH and extracted with dichloromethane. After drying and concentrating 2.2 g (94 %) of the title compound (92% HPLC purity) is obtained as a colourless oil.

### Intermediate 11

### 2-(6-{2-[(2,6-dichlorobenzyl)(methyl)amino]ethoxy}hexyl)-1H-isoindole-1,3(2H)-dione

A mixture of 3.57 g (9.67 mmol) of the Intermediate 4, 2.2 g (11.57 mmol) of the Intermediate 10 and 1.82 ml (1.35 g; 10.44 mmol) of diisopropylethylamine are stirred at 45°C for 16 hr. The residue is partitioned between ethyl ether/water and the organic layer is washed thoroughly with water and extracted with 2N hydrochloric acid. The acid layer is extracted with dichloromethane, which is washed successively with with 5N NaOH and water. After drying and concentrating, 3.7 g (83%) of the title compound are obtained pure enough to continue with the next step.
MS (M+): 462.

### Intermediate 12

### 6-{2-[(2,6-dichlorobenzyl)(methyl)amino]ethoxy} hexan-1-amine

A solution of 3.7 g (7.98 mmol) of the Intermediate 11 and 0.47 ml (9.57 mmol) of hidrazine hydrate in 37 ml ethanol is stirred 72 hr at room temperature. 74 ml of diisopropyl ether are added and, after 10 min. stirring, the solid is filtered and the filtrate concentrated. The residue is dissolved in ethyl ether and washed successively with 2N NaOH and water. The organic layer is extracted with 2N HCl and the later washed, in turn, with dichloromethane. After basification with 32 % w/v NaOH the product is extracted with ethyl ether, which is dried and concentrated. The residue is purified through column chromatography eluting first with dichloromethane/methanol/aq NH₃ 40/4/0.2 then 40/8/1. 1.6 g (60%) of the title product are thus obtained.

### Intermediate 13

### 2-bromo-1-(2,2-dimethyl-4H-1,3-benzodioxin-6-yl)ethanol

10 g (35.0 mmol) of 2-bromo-1-(2,2-dimethyl-4H-1,3-benzodioxin-6-yl)ethanone (DE 3513885) are dissolved in 300 ml MeOH. The solution is cooled in an ice/water bath and, while stirring, 1.33 g (35.0 mmol) of sodium borohydride are added, in portions. After stirring for 30 min at the ice bath temperature, 400 ml of saturated ammonium chloride solution is added slowly while cooling. 300 additional ml of water are added and the whole is extracted 3 times with ethyl ether. The organic layer is washed with water, dried and concentrated to a small volume. The solid is filtered and washed with a little ethyl ether. 7.93 g (79%) of the pure title compound as an off white solid are thus obtained.

### Intermediate 14

### [2-bromo-1-(2,2-dimethyl-4H-1,3-benzodioxin-6-yl)ethoxy](tert-butyl)dimethylsilane

9.5 g (33.1 mmol) of the Intermediate 13 are dissolved in 96 ml of dry DMF. 4.5 g (66.2 mmol) of imidazole are added and, in a nitrogen atmosphere, a solution of 10.0 g (66.2 mmol) of terbutyldimethylsilyl chloride in 48 ml dry DMF is added slowly. After 20 hr of stirring at room temperature the solvent is evaporated in vacuo. The residue is treated with ethyl ether and filtered. The filtrate is washed with water and brine. After drying and evaporation the excess of chlorosilane is eliminated in a vacuum pump at room temperature for 2-3 hr. The title compound (13.0 g, 98%) is obtained as a colorless oil.

### Intermediate 15

### N-(2,6-dichlorobenzyl)-5-(2,2-dimethyl-4H-1,3-benzodioxin-6-yl)-N,2,2,3,3-pentamethyl-4,14-dioxa-7-aza-3-silahexadecan-16-amine

A mixture of 1.3 g (3.23 mmol) of Intermediate 14, 1.07 g (3.23 mmol) of Intermediate 12, 0.67 g of potassium carbonate and 13 ml dioxane is stirred at 105°C (nitrogen atmosphere) for 16 hr. Excess water and petroleum ether are added. The insoluble material is discarded and the organic layer is washed with water, dried and concentrated to give 1.97 g of an oil. This compound is dissolved in 19 ml tetrahydrofuran and 1.7 g (6.5 mmol) of tetrabutylammonium fluoride trihydrate added. The solution is stirred at 45°C for 3 hr. The solution is concentrated and the residue partitioned between ethyl ether and water. The organic layer is washed with water, dried and concentrated giving 1.3 g of title compound.

### Example 2

### 4-{2-[(6-{2-[(2,6-dichlorobenzyl)(methyl)amino]ethoxy}hexyl)amino]-1-hydroxyethyl}-2-(hydroxymethyl)phenol

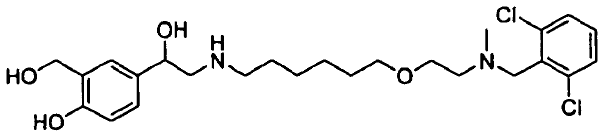

A solution of 1.3 g (2.40 mmol) of the Intermediate 16 in 63 ml of acetic acid and 15 ml of water is stirred at 75°C for 30 min. The solvent is evaporated in vacuo and the remaining water eliminated by coevaporation with ethanol then cyclohexane. Chromatographic purification eluting with DCM/MeOH/aq NH3 40/4/0.2 gives 0.18 g (15%) of the pure title compound as an oil. This is dissolved in 6 ml of isopropanol and 0.084 g (1 equivalent) of fumaric acid are added. The solvent is eliminated in vacuo and the residue taken in ethyl ether/ethyl acetate, filtered and dried.

¹H-NMR (d₆-DMSO): 1.02-1.05 m (1H); 1.28 bs (3H); 1.46 bs (2H); 1.59 bs (2H); 2.18 s (3H); 2.60-2.63 t (2H); 2.84-2.89 m (3H); 2.95-3.01 m (1H); 3.32-3.38 t (2H); 3.45-3.51 t (2H); 3.73 s (2H); 4.48 s (2H); 4.74-4.80 m (1H); 6.52 s (2H); 6.73-6.76 m (1H); 7.03-7.05 m (1H); 7.30-7.34 m (2H); 7.44-7.46 m (2H).

### Intermediate 16

### 3-[(2-{[6-((2-[4-(benzyloxy)-3-(hydroxymethyl)phenyl]-2-hydroxyethyl}amino)hexyl] oxy}ethyl)(methyl)amino]benzamide

A solution of 0.33 g (1.10 mmol) of Intermediate 6 and 0.30 g (1.10 mmol) of 4-benzyloxy-3-hydroxymethylphenylglioxal (US 4,753,962; description 54) in 3 ml THF and 3 ml methanol is stirred at room temperature for 1 hr. After cooling with an ice/water bath, 0.1 g (2.6 mmol) of sodium borohydride are added in portions and the stirring prosecuted for 2 hr at room temperature. After concentrating, the residue is partitioned in ethyl acetate/water, the organic layer washed with 2N NaOH and more water. The solution is dried and concentrated to give 0.54 g of the title compound that is used per se in the next step.

### Example 3

### 3-[(2-{[6-({2-hydroxy-2-[4-hydroxy-3-(hydroxymethyl)pheny]ethyl}amino)hexyl]oxy} ethyl)(methyl) amino]benzamide

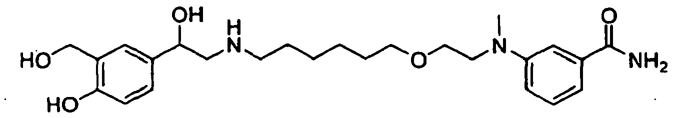

0.54 g (0.98 mmol) of the Intermediate 16 are dissolved in 60 ml of methanol. After adding 0.5 g of 10 % palladium on carbon catalyst the whole is hydrogenated at 0,069 MPa for 5 hr. The catalyst is filtered, the filtrate concentrated and the residue purified chromatographically eluting with DCM/MeOH/aq NH₃ 40/8/1. By this way 0.12 g (28%) of the title compound are obtained as an oil, which is dissolved in 6 ml of isopropanol and 0.030 g (1 equivalent) of fumaric acid are added. The solvent is eliminated in vacuo and the residue taken in ethyl ether/ethyl acetate, filtered and dried.

¹H-NMR (d₆-DMSO): 1.03-1.10 m (1H); 1.26 bs (3H); 1.43-1.58 m (4H); 2.75-2.87 m (3H) 2.94 s (2H); 3.34-3.39 m (4H); 3.51 bs (4H); 4.47 s (2H); 4.72-4.73 m (1H); 6.45 s (2H); 6.72-6.74 m (1H); 6.81-6.84 m (1H); 7.02-7.04 m (1H); 7.09-7.1 m (1H); 7.16-7.22 m (2H); 7.30 bs (1H); 7.86 bs (1H).

### Intermediate 17

### 8-(benzyloxy)-5-{1-{[tert-butyl(dimethyl)silyl]oxy}-2-[(6-{2-[(2,6-dichlorobenzyl) (methyl)amino]ethoxy}hexyl)amino]ethyl}quinolin-2(1H)-one

A mixture of 0.7 g (1.43 mmol) of Intermediate 8, 0.57 g (1.71 mmol) of Intermediate 12, 0.32 g of sodium iodide and 0.36 g of sodium hydrogen carbonate in 1.6 ml DMSO are stirred at 140°C (nitrogen atmosphere) for 2 hr. Excess water and ethyl acetate are added. The organic layer is washed thoroughly with water, dried and concentrated, giving 1.5 g of an oil which is dissolved in 10 ml THF. 0.8 g (3.0 mmol) of tetrabutylammonium fluoride are added and the system is stirred at 45°C for 3 hr. The solvent is evaporated in vacuo and the residue is partitioned between ethyl acetate and water. The organic layer is washed with water and extracted with 0.1 N hydrochloric acid. The acid layer is washed with ethyl ether, neutralized with sodium hydrogen carbonate and extracted with ethyl acetate. Once dried and concentrated, 0.7 g of the title compound pure enough to continue with the next step are obtained.

### Example 4

### 5-{2-[(6-{2-[(2,6-dichlorobenzyl)(methyl) amino]ethoxy}hexyl)amino]-1-hydroxyethyl} -8-hydroxyquinolin-2(1H)-one

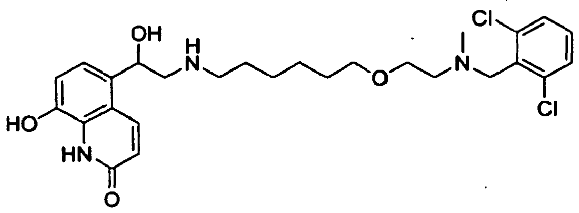

0.7 g (1.11 mmol) of the Intermediate 17 are dissolved in 24 ml MeOH and 0.15 g of 10 % palladium on charcoal catalyst are added. The whole is hydrogenated at 0,206 MPa for 6 hr. The catalyst is filtered and the filtrate evaporated. The residue is chromatographically purified eluting with DCM/MeOH/aq NH₃ 50/6/0.4 then 60/8/0.7 then 70/10/1, giving 0.2 g (33%) of the title compound as an oil. It is dissolved in 10 ml of ethanol and 0.095 g (1 equivalent) of fumaric acid are added. The solvent is eliminated in vacuo and the residue taken in ethyl acetate, filtered and dried.

¹H-NMR (d₆-DMSO): 1.25-1.30 bs (4H); 1.41-1.63 m (4H); 2.18 s (3H); 2.60-2.63 m (2H); 2.81-3.05 m (4H); 3.37 t (2H); 3.48 t (2H); 3.72 s (2H); 5-29-5.31 m (1H); 6.53 s (2H); 6-52-6.55 m (2H); 6.97-6.99 m (1H); 7.12-7.14 m (1H); 7.29-7.33 m (1H); 7.43-7.45 m (2H); 8.17s(1H);8.19s(1H).

### Intermediate 18

### N-methyl-N-(3-nitrobenzyl)amine

5 g (29.14 mmol) of 3-nitrobenzyl chloride are dissolved in 20 ml THF. 87 ml (174 mmol) of a 2M solution of methylamine in THF are added with stirring and the whole is stirred overnight at room temperature. The solvent is eliminated and a saturated solution of hydrogen chloride in dioxane is added to the residue until the pH is in the acidic range. The solid salt is taken in diisopropylether, filtered and washed with more diisopropyl ether.

The residue is partitioned between diethyl ether/1N HCl and the aqueous layer is alkalinized by adding solid potassium carbonate, extracted with DCM, dried and concentrated. 4.26 g of an oil (84% purity by 1H-NMR) of the title compound, which is enough pure to continue, are thus obtained.

### Intermediate 19

### 2-(6-{2-[methyl(3-nitrobenzyl)amino]ethoxy}hexyl)-1H-isoindole-1,3(2H)-dione

A mixture of 7.27 g (19.67 mmol) of Intermediate 4, 4.26 g (21.53 mmol) of the Intermediate 18 and 3.7 ml of diisopropylethylamine are stirred at 50°C overnight and two additional hours at 65°C. The residue is partitioned between ethyl ether and water. The organic layer is washed several times with water and then extracted with 2N HCl. The acid layer is extracted with DCM, which is washed with 2N NaOH, water, dried and concentrated giving 5.43 g (62%) of the title compound.

### Intermediate 20

### {2-[(6-aminohexyl)oxy]ethyl}methyl(3-nitrobenzyl)amine

A mixture of 5.4 g (12.28 mmol) of the Intermediate 19 and 0.72 ml (0.74g; 14.8 mmol) of hydrazine hydrate in 54 ml of ethanol is stirred at room temperature overnight. 0.36 additional ml of hydrazine hydrate are added and the system is refluxed for 4 hr. The whole is cooled in an ice/water bath while 108 ml of diisopropyl ether are added drop by drop. After stirring for 10' the solid is filtered. The filtrate is concentrated to dryness and the residue partitioned in ethyl ether/2N NaOH. The ethereal solution is washed successively with more 2N NaOH and water, and is extracted with 2N HCl. The aqueous layer is washed with DCM, basified with concentrated NaOH and extracted with diethyl ether, which is in turn washed with water, dried and concentrated, giving 2.84 g (70%) of the title compound (95 % purity by HPLC/MS).

### Intermediate 21

### 8-(benzyloxy)-5-{1-hydroxy-2-[(6-{2-[methyl(3-nitrobenzyl)amino]ethoxy}hexyl)amino]ethyl}quinolin-2(1H)-one

0.16 g (0.52 mmol) of 8-(benzyloxy)-5-(dihydroxyacetyl)quinolin-2(1H)-one (EP 0147719, Example 2(1)) are dissolved in 1.6 ml of DMSO. 0.16 g (0.52 mmol) of intermediate 20 are added and the system is stirred at room temperature for 2 hr. 1.6 ml of MeOH are added and the solution is cooled in an ice/water bath. 0.079 g (1 eq) of sodium borohydride is added and the stirring is prosecuted for 2 hr. After adding excess of water the system is extracted with ethyl acetate, the organic layer washed with water, dried and concentrated. The residue is chromatographically purified eluting with DCM/MeOH/aq NH₃ 40/4/0.2. 0.14 g (44%) of the title compound as an oil are thus obtained.

### Intermediate 22

### 8-(benzytoxy)-5-[3-(6-{2-[methyl(3-nitrobenzyl)amtno]ethoxy}hexyl)-2-oxo-1,3-oxazolidin-5-yl]quinolin-2(1H)-one

1.84 g (3.02 mmol) of the Intermediate 21 are dissolved in 3.0 ml of chloroform. 0.65 ml (0.47 g; 4.66 mmol) of triethylamine and 0.74 g (4.58 mmol) of carbonylbisimidazole are added. The solution is stirred at room temperature for 72 hr. Water and ethyl acetate are added and the organic layer is washed with water, dried and concentrated. The residue is chromatographically purified eluting with DCM then diethyl ether giving 0.36 g (19%) of the title compound.

### Intermediate 23

### 5-[3-(6-{2-[(3-aminobenzyl)(methyl)amino]ethoxy}hexyl)-2-oxo-1,3-oxazolidin-5-yl]-8-(benzyloxy)quinolin-2(1H)-one

0.33 g (0.52 mmol) of the Intermediate 22 are dissolved in 11.8 ml of ethanol and 0.47 g (2.08 mmol) of tin dichloride dihydrate are added. The system is refluxed in a nitrogen atmosphere for 2 hr. The solvent is evaporated and the residue is partitioned in DCM/6N NaOH. The organic layer is washed with water, dried and concentrated giving 0.29 g (92%) of the pure title compound.

### Intermediate 24

### N-(3-([{2-[(6-{5-[8-(benzyloxy)-2-oxo-1,2-dihydroquinolin-5-yl]-2-oxo-1,3-oxazolidin-3-yl}hexyl)oxy]ethyl}(methyl)amino]methyl}phenyl)urea

0.29 g (0.48 mmol) of the Intermediate 23 are dissolved in 2 ml acetic acid. 1 ml of water is added, and the system is cooled in an ice/water bath. With stirring, a solution of 0.16 g (1.95 mmol) of potassium cyanate in 2 ml of water is added dropwise slowly. The stirring is prosecuted for 2 hr at room temperature and excess water is added. The product is extracted with ethyl acetate, which is washed with sodium hydrogen carbonate solution, water, dried and concentrated. The residue is chromatographically purified eluting with DCM/MeOH/aq NH₃ 40/4/0.2. 0.11 g (35%) of the title compound are thus obtained.

### Intermediate 25

### N-(3-{[{2-[(6-{5-[8-(benxyloxy)-2-oxo-1,2-dihydroquinolin-5-yl]-2-oxo-1,3-oxazolidin-3-yl}hexyl)oxy]ethyl} (methyl)amino]methyl}phenyl)urea

0.11 g (0.17 mmol) of the Intermediate 24 are dissolved in THF and 0.10 g (0.78 mmol) of potassium trimethylsilanolate are added. The system is refluxed for 2 hr in a nitrogen atmosphere. 5 ml of DMSO are added in order to ensure complete solution of the components and the reflux is prosecuted for 2 hrs. After adding 0.10 additional g of silanolate and refluxing 2 hrs and repeating once more this operation the reaction is complete. Excess THF and saturated ammonium chloride solution are added. The organic layer is dried and concentrated giving 0.06 g (57%) of the title compound pure enough to continue with the next step.

### Example 5

### N-(3-{[{2-[(6-{[2-hydroxy-2-(8-hydroxy-2-oxo-1,2-dihydroquinolin-5-yl)ethyl]amino} hexyl)oxy]ethyl}(methyl)amino]methyl} phenyl)urea.

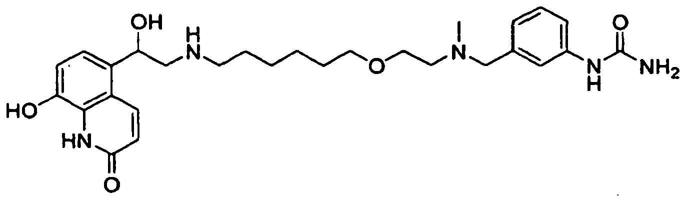

0.51 g (0.82 mmol) of the Intermediate 25 are dissolved in 50 ml MeOH. 0.5 g of 10 % palladium on charcoal catalyst are added and the system is hydrogenated at low pressure for 4 hr at room temperature. After filtering the catalyst and concentrating the filtrate the residue is chromatographically purified eluting with DCM/MeOH/aq NH₃ 80/20/2 then 70/30/3. 0.22 g (51%) of the title compound are thus obtained.

¹H-NMR (d₆-DMSO): 1.22-1.30 bs (4H); 1.36-1.48 m (4H); 2.14 s (3H); 2.47-2.56 m (4H); 2.64-2.70 m (2H); 3.33 t (2H); 3.41 s (2H); 3.46 t (2H); 5.00-5.04 m (1H); 5.81 s (2H); 6.48-6.51 m (1H); 6.79-6.82 m (2H); 6.89-6.92 m (1H); 7.05-7.15 m (2H); 7.28-7.30 m (2H); 8.15-8.18 m (1H); 8.52 s (1H).

### Intermediate 26

### Methyl-(3-nitro-benzyl)-carbamic acid benzyl ester

12.8 g (77.02 mmol) of the Intermediate 18 are dissolved in 400 ml of DCM. 12.8 ml (9.21 g; 94.5 mmol) of triethylamine are added. The solution is cooled in an ice/water bath and a solution of 13.12 ml (15.67 g; 91.9 mmol) of benzyl chloroformiate in 60 ml DCM is added dropwise. The stirring is prosecuted for 15 minutes at this temperature and 1 hr at room temperature. The solution is washed several times with water, dried and concentrated to give 23.1 g of the title compound as a colorless oil.

### Intermediate 27

### (3-Amino-benzyl)-methyl-carbamic acid benzyl ester

A mixture of 23.1 g (76.9 mmol) of the Intermediate 26 and 69.5 g (307.5 mmol) of tin dichloride dihydrate in 500 ml of ethanol is refluxed for 2 hr. The solvent is evaporated in vacuo and the residue is redissolved in 250 ml DCM. The solution is washed with 500 + 250 ml of 2N NaOH, water, and is dried and concentrated giving 20.7 g (quantitative yield) of the title compound.

### Intermediate 28

### Methyl-(3-ureido-benzyl)-carbamic acid benzyl ester

20.5 g (75.8 mmol) of the Intermediate 27 are dissolved in 150 ml of acetic acid. 50 ml of water are added and the solution is cooled in an ice/water bath. A solution of 27.1 g (334.0 mmol) of potassium cyanate in 50 ml of water is added dropwise slowly. When the addition is finished, the stirring is prosecuted for 30 min at room temperature. Excess water is added and the product extracted with ethyl acetate. The organic layer is washed with 4 % sodium hydrogen carbonate solution and water, dried and concentrated. The residue crystallises by treatment with ethyl ether. The solid is filtered and recrystallised from a little DCM/diisopropyl ether. 15.9 g (61 %) of the title compound are thus obtained.

### Intermediate 29

### (3-Methylaminomethyl-phenyl)-urea

15.9 g (50.74 mmol) of the Intermediate 28 are dissolved in 450 ml of methanol. 0.8 g of 10 % palladium on carbon catalyst are added and the system is hydrogenated at 0,276 MPa for 4 hr. After filtering the catalyst the filtrate is concentrated and the residue treated with ethyl ether. The crystalline solid is filtered and washed with a little diethyl ether. 8.2 g (90%) of the title compound is thus obtained.

### Intermediate 30

### N-(3-{[(2-{[6-(1,3-dioxo-1,3-dihydro-2H-isoindol-2-yl)hexyl]oxy}ethyl)(methyl)amino] methyl}phenyl)urea

A mixture of 8.2 g (45.75 mmol) of the Intermediate 29, 15.47 g (41.8 mmol) of the Intermediate 4 and 4.15 g of sodium hydrogen carbonate in 81 ml of DMF is stirred at 70°C for 72 hr. The solvent is eliminated in vacuo and the residue partitioned in ethyl acetate/water. The organic layer is washed with water, dried and concentrated to give an oil. This is dissolved in 150 ml of ethyl acetate and 150 ml of ethyl ether is added. A coloured oil separates and is discarded. The solution is concentrated to give 16.0 g (83%) of the title compound as an oil enough pure to continue with the next step.

### Intermediate 31

### N-(3-{[{2-[(6-aminohexyl)oxy]ethyl}(methyl) amino]methyl}phenyl)urea

A mixture of 16.o g (35.35 mmol) of the Intermediate 30 and 2.56 ml (2.64 g; 52.7 mmol) of hydrazine monohydrate in 190 ml of ethanol is refluxed for 2 hr and stirred at room temperature overnight. The solvent is evaporated in vacuo and the residue is partitioned in 0.2N NaOH (150 ml)/ethyl ether (100 ml). The aqueous layer is washed with 100 ml of ethyl ether and extracted with DCM. By adding saturated solution of potassium carbonate to the aqueous layer more product is extracted with DCM. The extracts are dried and concentrated giving 8.9 g (78%) of the title product as an oil.

### Intermediate 32

### N-(3-{(14R)-14-[8-(benzyloxy)-2-oxo-1,2-dihydroquinolin-5-yl]-2,16,16,17,17-penta-methyl-5,15-dioxa-2,12-diaza-16-silaoctadec-1-yl}phenyl)urea

A mixture of 3.0 g (6.14 mmol) of 8-(benzyloxy)-5-((1R)-2-bromo-1-{[tert-butyl(dimethyl)silyl]oxy}ethyl)quinolin-2(1H)-one (US20040059116), 2.0 g (6.20 mmol) of Intermediate 31, 1.4 g (9.33 mmol) of sodium iodide and 1.56 g (18.5 mmol) of sodium hydrogen carbonate in 7.5 ml of DMSO is stirred at 85°C for 20 hr. A further 0.5 g (1.02 mmol) of the bromoderivative are added ant the stirring at 85°C is prosecuted for 18 hr. Excess water and ethyl acetate are added and the organic layer is washed with water, dried and concentrated. The residue is chromatographically purified eluting with DCM/MeOH/aq NH₃ 40/2.5/0.2 to give 0.80 g (18%) of the title compound.

### Intermediate 33

### N-(3-{[(2-{[6-({(2R)-2-[8-(benzyloxy)-2-oxo-1,2-dihydroquinolin-5-yl]-2-hydroxyethyl} amino)hexyl]oxy}ethyl)(methyl) amino]methyl}phenyl)urea

0.8 g (1.09 mmol) of the Intermediate 32 are dissolved in 12 ml of THF and 0.50 g (1.89 mmol) of ammonium tetrabutyl fluoride are added and the system is stirred at room temperature for 20 hr. After concentration, the residue is partitioned in ethyl acetate/water, the organic layer washed with water, dried and concentrated. The residue is chromatographically purified eluting with DCM/MeOH/aq NH₃ 40/4/0.2 to give 0.45 g (66%) of the title compound.

### Example 6

### N-(3-{[{2-[(6-{[(2R)-2-hydroxy-2-(8-hydroxy-2-oxo-1,2-dihydroquinolin-5-yl)ethyl] amino}hexyl)oxy]ethyl}(methyl)amino]methyl} phenyl)urea.

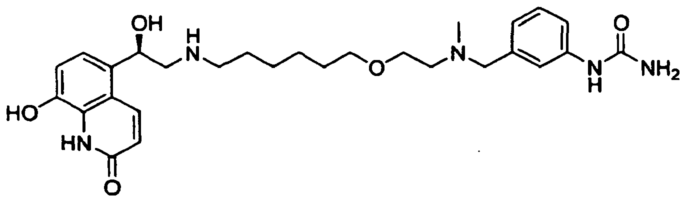

0.45 g (0.73 mmol) of the Intermediate 33 are dissolved in 45 ml of methanol. 0.45 g of 10 % palladium on charcoal catalyst are added and the system is hydrogenated at low pressure for 20 hr. After filtering the catalyst the solvent is evaporated in vacuo and the residue is chromatographically purified eluting with DCM/MeOH/aq NH₃ 40/8/1 to give 0.22 g (57%) of the title compound.

¹H-NMR (d₆-DMSO): 1.25-1.32 bs (4H); 1.39-1.53 m (4H); 2.14 s (3H); 2.47-2.60 m (4H); 2.71-2.73 m (2H); 3.33 t (2H); 3.42 s (2H); 3.46 t (2H); 5.03-5.08 m (1H); 5.83 s (2H); 6.49-6.52 m (1H); 6.80-6.82 m (1H); 6.90-6.93 m (1H); 7.06-7.16 m (2H); 7.28-7.31 m (2H); 8.16-8.19 m (1H); 8.55 s (1H).

### Intermediate 34

### N-(3-{[(2-{[6-({2-[4-(benzyloxy)-3-(hydroxymethyl)phenyl]-2-hydroxyethyl}amino)hexyl]oxy}ethyl)(methyl)amino]methyl} phenyl)urea

A solution of 0.8 g (2.48 mmol) of Intermediate 31 and 0.70 g (2.59 mmol) of 4-benzyloxy-3-hydroxymethylphenylglioxal (US 4,753,962; description 54) in 10 ml THF and 10 ml methanol is stirred at room temperature for 1.5 hr. After cooling with an ice/water bath, 0.2 g (5.2 mmol) of sodium borohydride are added in portions and the stirring prosecuted for 1.5 hr at room temperature. 80 ml of DCM are added and the solution is washed with 20 ml of water. The water solution, after adding ammonium chloride, is reextracted with more DCM. The combined DCM extracts are dried and concentrated. The residue is chromatographically purified eluting with DCM/MeOH/aq NH₃ 40/4/0.2 to give 0.30 g (21%) of the title compound.

### Example 7

### N-(3-{[(2-{[6-({2-hydroxy-2-[4-hydroxy-3-(hydroxymethyl)phenyl]ethyl}amino)hexyl]oxy}ethyl)(methyl)amino]methyl}phenyl) urea

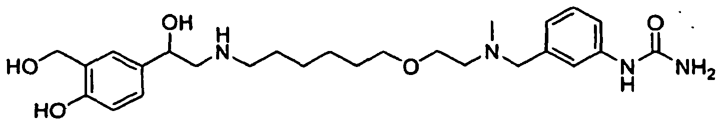

0.3 g (0.52 mmol) of the Intermediate 34 are dissolved in 12 ml of MeOH. 0.015 g of 10 % palladium on charcoal catalyst are added and the system is hydrogenated at low pressure for 16 hr at room temperature. After filtering the catalyst and concentrating the residue is chromatographically purified eluting with DCM/MeOH/aq NH₃ 40/4/0.2 to give 0.14 g (60%) of the title compound. It is dissolved in 5 ml of methanol and 0.033 g (1 equivalent) of fumaric acid dissolved in methanol are added. The solvent is eliminated in vacuo and the residue taken in ethyl acetate, filtered and dried.

¹H-NMR (d₆-DMSO): 1.29 bs (4H); 1.46-1.65 bs (4H); 2.16 s (3H); 2.49-2.53 m (2H); 2.86-2.92 m (2H); 2.98-3.03 m (1H); 3.34 t (2H); 3.44 s (2H); 3.48 t (2H); 4.48 s (2H); 4.79-4.82 m (1H); 5.97 bs (2H); 6.50 s (2H); 6.74-6.82 m (2H); 7.03-7.16 m (2H); 7.29-7.35 m (3H); 8.86 s (1H).

### Intermediate 35

### ({3-[(6-bromohexyl)oxy]propoxy}methyl)benzene

A mixture of 3-(benzyloxy)propan-1-ol (25 ml, 26.1 g, 0.157 mol), 1,6-dibromohexane (85 ml, 134.8 g, 0.552 mol), tetrabutylammonium bromide (1.0 g, 0.003 mol) and 60 ml of aqueous NaOH solution (50% weight), was vigorously stirred at room temperature for 20 hours. The reaction mixture was diluted with 200 ml of water and extracted with hexane (3 x 100 ml). The organic extracts were combined, washed with water and brine, dried (MgSO4), and concentrated. The obtained residue was purified by distillation under reduced pressure. The title compound was obtained as a colourless oil (23.6 g, 45%), b.p.: 150°C-156°C, 0.3 mm Hg).

### Intermediate 36

### 2-{6-[3-(benzyloxy)propoxy]hexyl}-1H-isoindole-1,3(2H)-dione

To a solution of 100.0 g (0.304 mol) of Intermediate 35 in 220 ml of dimethylformamide, 64.7 g (0.349 mol) of potassium phtalimide and 0.2 g (0.4 mmol) of hexadecyltributyl-phosphonium bromide were added. The mixture was stirred at 75°C for 4 hours and at room temperature overnight. After this reaction time the solvent was removed under reduced pressure. The obtained residue was diluted with water and the obtained solution was extracted with diethyl ether (x3). The organic extracts were combined, washed with water (x2) and brine, dried (MgSO₄), and concentrated under reduced pressure. The title compound was obtained as an oil. (115.1g, 96%).

### Intermediate 37

### 2-[6-(3-hydroxypropoxy)hexyl]-1H-isoindole-1,3(2H)-dione

To a solution of 42.5 g (0.107 mol) of Intermediate 36 in 200 ml of methanol and 150 ml of ethyl acetate , 2.0 g of Pd/C (10%) were added. The mixture was hydrogenated at room temperature at 0,262 MPa. for 4 hours. The catalyst was filtered and the filtrate was concentrated under reduced pressure. The title compound was obtained as an oil (30.5 g, 93%).

### Intermediate 38

### 3-{[6-(1,3-dioxo-1,3-dihydro-2H-isoindol-2-yl)hexyl]oxy}propyl methanesulfonate

A solution of 2.67 ml (3.95 g , 0.0345 mol) of methanesulfonyl chloride in 40 ml of dichloromethane was added to a solution of 10.0 g (0.0327 mol) of Intermediate 37 and 5 ml (3.63 g, 0.0359 mol) of triethylamine in 65 ml of dichloromethane. The mixture was stirred at room temperature for 4 hours. After this reaction time, 500 µl (3.59 mmol) of triethylamine and 267 µl (3.45 mmol) of methanesulfonyl chloride were added and the reaction mixture was stirred at room temperature overnight. Excess dichloromethane was added and the obtained solution was washed with water, NaHCO₃ (4% solution) (x2), dried (MgSO₄), and concentrated under reduced pressure. The obtained residue was purified by column chromatography on silica gel using hexane/ethylacetate (70:30→40:60) as eluents. The title compound was obtained as an oil. (8.94 g, 71%).

### Intermediate 39

### 2-(6-{3-[(2,6-dichlorobenzyl)(methyl)amino]propoxy}hexyl)-1H-isoindole-1,3(2H)-dione

A mixture of 3.23 g (8.4 mmol) of Intermediate 38, 2.0 g (10.5 mmol) of Intermediate 10 and 1.59 ml (1.18g, 9.1 mmol) of N,N-diisopropylethylamine was vigorously stirred at 45°C for 18 h. The crude reaction was diluted with 150 ml of ethyl ether and washed with water (2 x 50 ml). The organic phase was extracted with 2N HCl (2x 50 ml), the acidic solutions were combined and extracted with methylene chloride (3x 50 ml). The methylene chloride solutions were combined, washed with 6N NaOH (to obtain the free base) and water (x2), dried (MgSO₄) and the solvent was removed under reduced pressure. The title compound was obtained as an ambered oil (3.15g, 78%) and was used in the next step without further purification.

### Intermediate 40

### {3-[(6-aminohexyl)oxy]propyl}(2,6-dichlorobenzyl)methylamine

To a solution of 3.15 g (6.60 mmol) of Intermediate 39 in 30 ml of ethanol, 0.38 ml (7.83 mmol) of hydrazine monohydrate were added. The mixture was stirred at room temperature for 28h. After this time 63 µl (1.32 mmol) of hydrazine monohydrate were added and the reaction mixture was stirred at room temperature for 72 h. A white solid was formed during the process. The reaction mixture was treated with 60 ml of isopropyl ether and filtered. The solid was discarded and the filtrate was concentrated under reduced pressure. The obtained residue was diluted with ethyl ether and washed with 2N NaOH and water. The organic phase was extracted with 2N HCl (x2). The acidic extracts were combined, washed with methylene chloride and basified with solid K₂CO₃. The aqueous basic solution was extracted with ethyl ether (x3). The ether extracts were combined, washed with brine, dried (MgSO₄), and the solvent was removed under reduced pressure. The obtained residue was purified by column chromatography on silica gel, eluting with chloroform/methanol/ammonium hydroxide (90:10:0.5 → 90:10:1 → 80:20:2). The title compound was obtained as a colourless oil (1.05 g, 45%).

### Intermediate 41

### N-(2,6-dichlorobenzyl)-5-(2,2-dimethyl-4H-1,3-benzodioxin-6-yl)-N,2,2,3,3-pentamethyl-4,14-dioxa-7-aza-3-silaheptadecan-17-amine

To a solution of 1.05 g (3.02 mmol) of Intermediate 40 and 1.21 g (3.02 mmol) of Intermediate 14 in 12 ml of dioxane, 0.52 g (3.78 mmol) of solid potassium carbonate were added. The mixture was stirred at 105°C, under nitrogen atmosphere, for 20h. The reaction mixture was cooled at room temperature and the solvent was removed under reduced pressure. The obtained residue was treated with water and extracted with ethyl acetate (x2). The organic extracts were combined, washed with water and brine, dried (MgSO₄), and concentrated under reduced pressure. The obtained brown oil was purified by column chromatography on silica gel eluting with chloroform/methanol (35:1). The title compound was obtained as an ambered oil (715 mg, 35%).

### Intermediate 42

### 2-[(6-{3-[(2,6-dichlorobenzyl)(methyl)amino]propoxy}hexyl)amino]-1-(2,2-dimethyl-4H-1,3-benzodioxin-6-yl)ethanol

To a solution of 610 mg (0.913 mmol) of Intermediate 41 in 14 ml of anhydrous tetrahydrofuran , 1.159 g (1.16-1.74 mmol) of tetrabutyl ammonium fluoride on SiO₂ (capacity (F⁻):1.0-1.5 mmol /g) were added. The mixture was stirred at room temperature, under nitrogen atmosphere, for 19 h. The reaction mixture was filtered and the filtrate was concentrated under reduced pressure. The obtained residue was purified by column chromatography on silica gel, eluting with chloroform/methanol (9:1) → chloroform/methanol/ammonium hydroxide (90:10:0.5). The title compound was obtained as an ambered oil (430 mg, 85%).

### Example 8

### 4-{2-[(6-{3-[(2,6-dichlorobenzyl)(methyl)amino]propoxy}hexyl)amino]-1-hydroxyethyl}-2-(hydroxymethyl)phenol

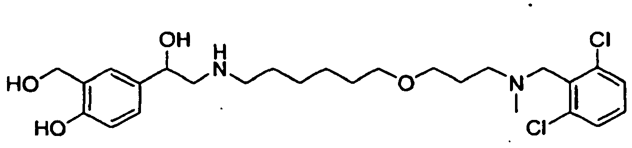

A mixture of 480 mg (0.87 mmol) of Intermediate 42, 23.2 ml of glacial acetic acid and 5.9 ml of water was stirred at 70°C for 30 min. The reaction mixture was cooled and concentrated under reduced pressure. The obtained residue was diluted with cyclohexane and concentrated (x2), the residue was diluted with chloroform (ethanol free) and concentrated again. The oil obtained was purified by column chromatography on silica gel eluting with chloroform/methanol/ammonium hydroxide (90:10:1→90:15:1.5). The title compound was obtained as an oil (345 mg, 77%).

¹H-NMR (400 MHz, Cl3CD) δ ppm: 1.28-1.35 (m, 4H), 1.43-1.56 (m, 4H), 1.80-3.0 (br.s., 4H), 1.77-1.85 (m, 2H), 2.24 (s, 3H), 2.54-2.82 (m, 6H), 3.36 (t, *J*=6.6 Hz, 2H), 3.44 (t, *J*=6.4 Hz, 2H), 3.74 (s, 2H), 4.58 (dd, *J*=8.7, 3.7 Hz, 1H), 4.80 (s, 2H), 6.82 (d, *J*=8.3 Hz, 1H), 6.99-7.02 (m, 1H), 7.10-7.15 (m, 2H), 7.25-7.31 (m, 2H).
MS: 513 (2 Cl) [M+1]⁺

### Intermediate 43

### 3-(methylamino)benzamide

To a solution of 16.26 g (119 mmol) of 3-aminobenzamide in 445 ml of dry tetrahydrofuran, 11.71 g (125 mmol) of potassium carbonate were added. A solution of 11.83 ml (15.77 g, 125 mmol) of dimethyl sulphate in 30 ml of dry tetrahydrofuran was added dropwise to the obtained suspension while stirring. The stirring was continued for 66 hours at room temperature. After this time the solvent was removed under reduced pressure. The residue was diluted with water, the obtained solution saturated with solid potassium carbonate and extracted with ethyl ether several times. The ether extracts were combined, washed with a small volume of brine, dried (MgSO₄), and concentrated under reduced pressure. The obtained residue was purified by chromatography on silica gel performing a gradient elution using mixtures of chloroform/methanol (from 50:1 to 15:1) as eluents. Appropriate fractions were combined and concentrated. The title compound was obtained as an sticky gum that gave a solid after treatment with isopropyl ether (6.74 g, 38%).

### Intermediate 44

### 3-[(3-{[6-(1,3-dioxo-1,3-dihydro-2H-isoindol-2-yl)hexyl]oxy}propyl)(methyl)amino] benzamide

A mixture of 3.06 g (8.0 mmol) of Intermediate 38, 1.50 g (10.0 mmol) of Intermediate 43 and 2.8 ml (2.08 g, 16.0 mmol) of N,N-diisopropylethylamine was vigorously stirred at 120°C for 5 hours. The reaction mixture was cooled at room temperature, treated with water (100 ml) and extracted with ethyl acetate (3 x 50 ml). The ether extracts were combined, washed with a solution of 10% citric acid, washed with brine, dried (MgSO₄) and concentrated. The obtained residue was purified by column chromatography on silica gel eluting with chloroform/methanol 50:1→30:1. The title compound was obtained as an oil (3.0 g, 86%).

### Intermediate 45

### 3-[{3-[(6-aminohexyl)oxy]propyl}(methyl)amino]benzamide

To a solution of 3.0 g (6.86 mmol) of Intermediate 44 in 30 ml of Ethanol, 0.4 ml (8.25 mmol) of hydrazine monohydrate were added. The reaction mixture was stirred at room temperature for 22 hours. A white solid was formed during the process. The reaction mixture was filtered, the solid was discarded and the filtrate was concentrated under reduced pressure. The obtained residue was purified by column chromatography on silica gel eluting with chloroform/methanol/ammonium hydroxide 90:10:1→80:20:2. The title compound was obtained as an oil (1.27 g, 60%).

### Intermediate 46

### 3-[(3-{[6-({2-[4-(benzyloxy)-3-(hydroxymethyl)phenyl]-2-hydroxyethyl}amino)hexyl] oxy}propyl)(methyl)amino]benzamide

To a suspension of 435 mg (1.609 mmol) of 4-benzyloxy-3-hydroxymethylphenylglioxal (US 4,753,962; description 54) in 4.5 ml of dry tetrahydrofuran, a solution of 500 mg (1.626 mmol) of Intermediate 45 in 4.5 ml of methanol was added. The mixture was stirred at room temperature for 1 hour. After this time the reaction mixture was cooled to 0°C-5°C and 145 mg (3.833 mmol) of NaBH₄ were added in several portions. The mixture was warmed to room temperature and the stirring was continued for 2 hours. The crude reaction was filtered and the filtrate concentrated under reduced pressure. The obtained residue was treated with water and extracted with ethyl acetate (x2). The organic extracts were combined, washed with 2N NaOH, water and brine, dried (MgSO₄), and the solvent was removed under reduced pressure. The title product was obtained as an oil. (770 mg, 85%).

### Example 9

### 3-[(3-{[6-({2-hydroxy-2-[4-hydroxy-3-(hydroxymethyl)phenyl]ethyl}amino) hexyl]oxy} propyl)(methyl)amino]benzamide

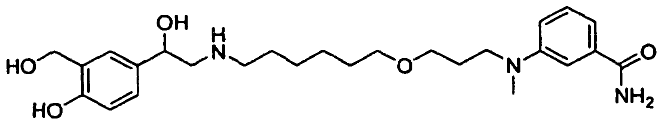

770 mg (1.366 mmol) of Intermediate 46 were dissolved in 77 ml of MeOH and 155 mg of Pd/C (10%) were added. The mixture was hydrogenated at room temperature at 0,069 MPa for 5 hours. The catalyst was filtered and the filtrate was concentrated under reduced pressure. The obtained residue was purified by column chromatography on silica gel using chloroform/methanol/ammonium hydroxide 90:10:1→80:20:0.5 as eluents. The title compound was obtained as a dry foam (395 mg, 61%).

¹H-NMR (400 MHz, d₆-DMSO) δ ppm: 1.23-1.35 (m, 4H), 1.35-1.44 (m, 2H), 1.45-1.55 (m, 2H), 1.69-1.75 (m, 2H), 2.47-2.62 (m, 4H), 2.90 (s, 3H), 3.28-3.44 (m, 6H), 4.46 (s, 2H), 4.47-4.52 (m, 1H), 4.84-5.11 (bs, 2H), 6.68 (d, *J*=8.2 Hz, 1H), 6.82 (dd, *J*=8.2, 2.3 Hz, 1H), 6.98 (dd, *J*=8.0, 2.2 Hz, 1H), 7.09 (d, *J*=7.8 Hz, 1H), 7.13-7.16 (m, 1H), 7.18 (d, *J*=7.8 Hz, 1H), 7.21 (bs, 1H), 7.25 (d, *J*=2.0 Hz, 1H), 7.83 (bs, 1H), 8.86-9.36 (bs, 1H).
MS: 474 [M+1]⁺

### Intermediate 47

### 3-[[(13R)-13-(2,2-dimethyl-4H-1,3-benzodioxin-6-yl)-15,15,16,16-tetramethyl-4,14-dioxa-11-aza-15-silaheptadec-1-yl](methyl)amino]benzamide

To a solution of 499 mg (1.623 mmol) of Intermediate 45 and 652 mg (1.624 mmol) of [(1R)-2-bromo-1-(2,2-dimethyl-4H-1,3-benzodioxin-6-yl)ethoxy](tert-butyl)dimethylsilane (US 2004167167, Preparation 43) in 6.5 ml of dioxane, 0.281 g (2.033 mmol) of solid potassium carbonate were added. The mixture was stirred at 105°C, under nitrogen atmosphere, for 23.5 h and then for 16 h at room temperature. The solvent was removed under reduced pressure. The obtained residue was treated with water and extracted with ethyl acetate (x2). The organic extracts were combined, washed with water and brine, dried (MgSO₄), and concentrated under reduced pressure. The obtained residue was purified by column chromatography on silica gel performing a gradient elution using chloroform → chloroform/methanol (150:1) → chloroform/methanol/ammonium hydroxide (90:10:1→80:20:2) as eluents. Appropiate fractions were combined and concentrated. The title compound was obtained as an oil (515 mg, 50%).

### Intermediate 48

### 3-[{3-[(6-{[(2R)-2-(2,2-dimethyl-4H-1,3-benzodioxin-6-yl)-2-hydroxyethyl]amino} hexyl)oxy]propyl}(methyl)amino]benzamide

To a solution of 620 mg (0.987 mmol) of Intermediate 47 in 14.9 ml of anhydrous tetrahydrofuran, 1.257 g (1.26-1.88 mmol) of tetrabutyl ammonium fluoride on SiO₂ (capacity (F⁻):1.0-1.5 mmol /g) were added. The mixture was stirred at room temperature, under nitrogen atmosphere, for 16h.
The stirring was continued for 26 hours more, and during this time 989 mg (0.989-1.483 mmol) more of tetrabutyl ammonium fluoride on SiO₂ were added in several portions as the reaction was being controlled by HPLC-MS.

The reaction mixture was filtered, the solid was washed with tetrahydrofuran and with a mixture of chloroform/methanol/ammonium hydroxide 90:10:1, the filtrates were combined and concentrated under reduced pressure. The obtained residue was purified by column chromatography on silica gel, eluting with chloroform/methanol (15:1)→chloroform/methanol/ammonium hydroxide (90:10:0.1→90:10:1). Appropriate fractions were combined and concentrated. The title compound was obtained as an oil (374 mg, 74%).

### Example 10

### 3-[(3-{[6-({(2R)-2-hydroxy-2-[4-hydroxy-3-(hydroxymethyl)phenyl]ethyl}amino)hexyl] oxy}propyl)(methyl)amino]benzamide

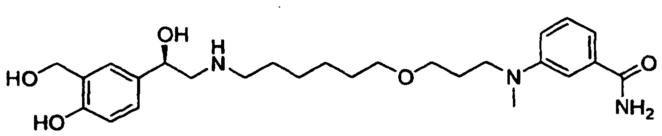

A mixture of 369 mg (0.718 mmol) of Intermediate 48, 7.7 ml of glacial acetic acid and 3.3 ml of water was stirred at 80°C for 30 min. The reaction mixture was cooled and concentrated under reduced pressure. The obtained residue was diluted with cyclohexane and concentrated (x2), the residue was diluted with chloroform and concentrated again (x2). The oil obtained was purified by column chromatography on silica gel eluting with chloroform/methanol/ammonium hydroxide 90:10:1. The title compound was obtained as an oil (213 mg, 63%).

¹H-NMR (300 MHz, d₆-DMSO) δ ppm: 1.24-1.36 (m, 4H), 1.36-1.45 (m, 2H), 1.45-1.56 (m, 2H), 1.66-1.78 (m, 2H), 2.46-2.63 (m, 4H), 2.89 (s, 3H), 3.19-3.50 (m, 6H), 4.46 (s, 2H), 4.52 (t, *J*=6.3 Hz, 1H), 4.79-5.24 (bs, 2H), 6.69 (d, *J*=8.2 Hz, 1H), 6.82 (dd, *J*=8.2, 2.5 Hz, 1H), 6.98 (dd, *J*=8.1, 2.1 Hz, 1H), 7.09 (d, *J*=6.9 Hz, 1H), 7.13-7.16 (m, 1H), 7.18 (d, *J*=8.2 Hz, 1H), 7.22 (bs, 1H), 7.24-7.28 (d, 1H), 7.85 (bs, 1H), 8.73-9.56 (bs, 1H)
MS: 474 [M+1]⁺

### Intermediate 49

### 3-[(methylamino)methyl]benzonitrile

To a commercially available solution of methylamine 2M in tetrahydrofuran (200 ml, 400 mmol), 11.2 g (57.1 mmol) of 3-(bromomethyl)benzonitrile dissolved in 112 ml of tetrahydrofuran were added dropwise. The reaction mixture was stirred at room temperature for 24 hours. The solvent was removed under reduced pressure. The obtained residue was diluted with ethyl ether and extracted with 1N HCl. The acidic extracts were basified with solid potassium carbonate and the basic solution extracted with dichloromethane. The organic phase was dried (MgSO₄) and concentrated under reduced pressure. The title compound was obtained as an oil (7.98g, 96%).

### Intermediate 50

### 3-[(methylamino)methyl]benzamide

To a solution of 7.98 g (54.6 mmol) of Intermediate 49 in 80 ml of isopropanol, 7.98 g (142.2 mmol) of powdered KOH (85%) were added. The mixture was stirred for 2 hours at reflux temperature. The solvent was reduced under reduced pressure. The obtained residue was dissolved in a small volume of water and extracted several times with dichloromethane. The organic extracts were combined, dried (MgSO₄), and concentrated. The residue was treated with hexane and concentrated (x3). The obtained residue was treated with chloroform and concentrated (x3), and finally the residual solvents were eliminated using an oil vacuum pump. The title compound was obtained as an oil that slowly solidified at room temperature. (5.14 g, 57%).

### Intermediate 51

### 3-{[(2-{[6-(1,3-dioxo-1,3-dihydro-2H-isoindol-2-yl)hexyl]oxy}ethyl)(methyl)amino] methyl}benzamide

A mixture of 1.5 g (9.13 mmol) of Intermediate 50, 3.07 g (8.30 mmol) of Intermediate 4 and 0.8 g (9.55 mmol) of solid sodium bicarbonate in 15 ml of anhydrous dimethylformamide was heated at 70°C for 48 hours. The solvent was removed under reduced pressure. The residue was treated with water and extracted with ethyl acetate (x2). The organic extracts were combined, washed with water (x2) and brine, dried (MgSO₄), and concentrated. The residue was purified by column chromatography on silica gel eluting with chloroform/methanol 15:1 → 9:1. Appropriate fractions were combined and concentrated. The title compound was obtained as an oil. (2.81g , 77%).

### Intermediate 52

### 3-{[{2-[(6-aminohexyl)oxy]ethyl}(methyl)amino]methyl}benzamide

To a solution of 2.80 g (6.40 mmol) of Intermediate 62 in 33 ml of ethanol, 0.47 ml (9.60 mmol) of hydrazine monohydrate were added. The mixture was refluxed for 3 hours. A white solid was formed during the process. The reaction mixture was filtered and the solid was washed with ethanol and with ethyl ether. The solid was discarded. The filtrates were combined and concentrated under reduced pressure. The obtained residue was dissolved in 80 ml of 1 N NaOH and extracted with dichloromethane (5 x 50 ml). The organic extracts were combined, dried (MgSO₄), and concentrated. The title compound was obtained as an oil (1.78 g, 90%).

### Intermediate 53

### 3-{[(2-{[6-({2-[8-(benzyloxy)-2-oxo-1,2-dihydroquinolin-5-yl]-2-hydroxyethyl}amino)hexyl]oxy}ethyl)(methyl)amino]methyl}benzamide

A solution of 1.06 g (3.25 mmol) of 8-(benzyloxy)-5-(dihydroxyacetyl)quinolin-2(1H)-one (EP 0147719, Example 2(1)) and 1.0 g (3.25 mmol) of Intermediate 52 in 10 ml of dimethylsulfoxide was stirred at room temperature for 2 hours. The reaction mixture was diluted with 10 ml of methanol, cooled to 0-5°C and 0.37 g (9.76 mmol) of NaBH₄ were added in several portions. The mixture was stirred at 0-5°C for 15 min and then at room temperature for 19 hours. The reaction mixture was diluted with 150 ml of tetrahydrofuran, 150 ml of saturated NH₄Cl solution were added and the mixture was stirred at room temperature for 15 min. The organic layer was separated and the aqueous layer was extracted with tetrahydrofuran (x2). The organic extracts were combined, dried (MgSO₄), and concentrated under reduced pressure. The obtained residue was diluted with ethanol and concentrated. The oil obtained was purified by column chromatography on silica gel performing a gradient elution using with chloroform/methanol (9:1)→ chloroform/methanol/ammonium hydroxide (90:10:1→80:20:2) as eluents . Appropriate fractions were combined and concentrated. The title compound was obtained as an oil (1.23 g, 63%).

### Example 11

### 3-{[{2-[(6-{[2-hydroxy-2-(8-hydroxy-2-oxo-1,2-dihydroquinolin-5-yl)ethyl]amino} hexyl)oxy]ethyl}(methyl)amino]methyl}benzamide

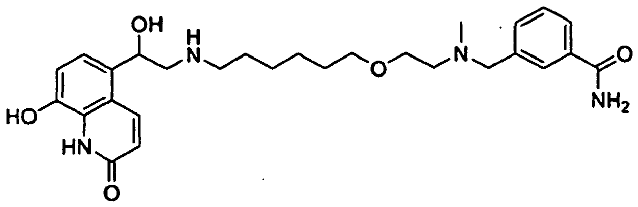

To a solution of 1230 mg (2.047 mmol) of Intermediate 53 in 123 ml of MeOH, 32 mg of Pd/C (10%) were added. The mixture was hydrogenated (H₂ balloon) at room temperature for 22 hours. After this time 64 mg more of Pd/C (10%) were added and the hydrogenation continued for 22 hours in the same conditions. The catalyst was filtered and the solvent was removed under reduced pressure. The resulting oil was purified by column chromatography on silica gel eluting with chloroform/methanol/ammonium hydroxide (80:20:2→80:20:3). The title product was obtained as a dry foam (670 mg, 64%).

¹H-NMR (300 MHz, d₆-DMSO) δ ppm: 1.20-1.31 (m, 4H), 1.33-1.41 (m, 2H), 1.41-1.52 (m, 2H), 2.15 (s, 3H), 2.45-2.58 (m, 4H), 2.61-2.73 (m, 2H), 3.33 (t, *J*=6.5 Hz, 2H), 3.48 (t, *J*=5.9 Hz, 2H), 3.53 (s, 2H), 4.97-5.05 (m, 1H), 6.49 (d, *J*=9.9 Hz, 1H), 6.90 (d, *J*=8.2 Hz, 1H), 7.06 (d, *J*=8.2 Hz, 1H), 7.32-7.47 (m, 3H), 7.74 (d, *J*=7.4 Hz, 1H), 7.79 (s, 1H), 7.96 (bs, 1H), 8.17 (d, *J*=10.2 Hz, 1H).
MS: 511 [M+1]⁺

### Intermediate 54

### 3-{[(2-{[6-({2-[4-(benzyloxy)-3-(hydroxymethyl)phenyl]-2-hydroxyethyl}amino)hexyl]oxy}ethyl)(methyl)amino]methyl}benzamide

To a suspension of 614 mg (2.272 mmol) of 4-benzyloxy-3-hydroxymethylphenylglioxal (US 4,753,962; description 54) in tetrahydrofuran, a solution of 705 mg (2.293 mmol) of Intermediate 52 in methanol was added. The obtained solution was stirred at room temperature for 1 hour. After this time, the reaction mixture was cooled to 0-5°C and 205 mg (5.419 mmol) of NaBH₄ were added in several portions. The mixture was warmed to room temperature and stirred for 2 hours. Solvents were removed under reduced pressure. The residue was treated with water and extracted with ethyl acetate (x2). The organic extracts were combined and washed with 2N NaOH, water and brine, dried (MgSO₄), and concentrated. The resulting oil was purified by column chromatography on silica gel, using chloroform/methanol/ammonium hydroxide (90:10:1→80:20:2) as eluent. Appropriate fractions were combined and concentrated. The obtained residue was purified again by column chromatography on silica gel using chloroform/methanol/ammonium hydroxide (90:10:0.3→90:15:1.5) as eluent. The title compound was obtained as an oil (830 mg, 65%).

### Example 12

### 3-{[(2-{[6-({2-hydroxy-2-[4-hydroxy-3-(hydroxymethyl)phenyl]ethyl}amino)hexyl]oxy} ethyl)(methyl)amino]methyl}benzamide

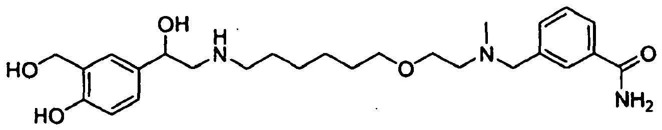

To a solution of 648 mg (1.150 mmol) of Intermediate 54 in 78 ml of methanol, 13 mg of Pd/C (10%) were added and the mixture was hydrogenated (H₂ balloon) at room temperature for 23 hours. The catalyst was filtered and the solvent was removed under reduced pressure. The resulting oil was purified by column chromatography on silica gel performing a gradient elution using chloroform/methanol (15:1)→ chloroform/methanol/ammonium hydroxide (90:10:0.1→80:20:4) as eluents. Appropriate fractions were combined and concentrated. The resulting oil (196 mg) was purified again by column chromatography on silica gel eluting with chloroform/methanol/ammonium hydroxide (85:15:1). The title product was obtained as an oil (97 mg, 25.2%, HPLC: 75%)

¹H-NMR (300 MHz, CDCl3 + 2 drops of d₆-DMSO) δ ppm: mixture of compounds. HPLC-MS: 75.2% title compound, 474 [M+1]⁺; 23.0% impurity , 431 [M+1]⁺

### Intermediate 55

### Benzyl [3-(2,4-dioxo-3,4-dihydropyrimidin-1(2H)-yl)benzyl]methylcarbamate

To a suspension of 2.40 g (7.66 mmol) of Intermediate 28 in 48 ml of dioxane, 0.46 g (11:50 mmol) of NaH (60% dispersion in mineral oil) were added in several portions. The reaction mixture was stirred at 60°C for 1.5 hours (until not H₂ formation was observed). After this time, the mixture was cooled to room temperature and 1.63 ml (1.70 g, 11.50 mmol) of methyl 3,3-dimethoxypropanoate were added. The mixture was stirred at reflux temperature, under N₂ atmosphere, for 1.5 hours and then was cooled to room temperature, 76.8 ml of acetic acid (40% aqueous solution) were added and the stirring continued for 1.5 hours. The crude reaction was treated with water and extracted with dichloromethane (x3). The organic extracts were combined, washed with water, 4% solution of NaHCO₃, water, dried (MgSO₄) and concentrated. The obtained residue was purified by column chromatography on silica gel performing a gradient elution using mixtures of chloroform/methanol (75:1→ 25:1) as eluents. Appropriate fractions were combined and concentrated. The title compound was obtained as an oil (607 mg, 22%).

### Intermediate 56

### 1-{3-[(methylamino)methyl]phenyl}pyrimidine-2,4(1H,3H)-dione

A solution of 602 mg (1.648 mmol) of Intermediate 55 in 18.6 ml of saturated ethanol/HCl(g) solution was stirred at reflux temperature for 30 min. The reaction mixture was cooled to room temperature and the solvent was removed under reduced pressure. The obtained residue was diluted with ethanol and concentrated (x2). The resulting product was purified by column chromatography on silica gel eluting with chloroform/methanol/ammonium hydroxide (90:10:1). The title compound was obtained as an oil. (271 mg, 71%).

### Intermediate 57

### 2-(6-{2-[[3-(2,4-dioxo-3,4-dihydropyrimidin-1(2H)-yl)benzyl](methyl)amino]ethoxy} hexyl)-1H-isoindole-1,3(2H)-dione

To a solution of 453 mg (1.226 mmol) of Intermediate 4 and 312 mg (1.349 mmol) of Intermediate 56 in 3 ml of dimethylformamide, 118 mg (1.405 mmol) of sodium bicarbonate were added. The reaction mixture was stirred at 70°C for 21 hours. After this time was cooled to room temperature and the solvent was removed under reduced pressure. The obtained residue was treated with water and extracted with ethyl acetate (x2). The organic extracts were combined, washed with water (x2) and brine, dried (MgSO₄) and concentrated. The title compound was obtained as an oil which was used in the next step without further purification. (595 mg, 96%).

### Intermediate 58

### 1-(3-{[{2-[(6-aminohexyl)oxy]ethyl}(methyl)amino]methyl}phenyl)pyrimidine-2,4(1H,3H)-dione

To a solution of 0.582 g (1.15 mmol) of Intermediate 57 in 6 ml of ethanol, 0.07 ml (1.44 mmol) of hydrazine monohydrate were added. The mixture was stirred at room temperature for 18 hours. The solvent was removed under reduced pressure. The obtained residue was treated with chloroform to obtain a solid which was filtered, washed with chloroform and discarded. The filtrates were combined and concentrated. The resulting product was purified by column chromatography on silica gel performing a gradient elution using chloroform/methanol (15:1) → chloroform/methanol/ammonium hydroxide (90:10:0.1→80:20:3) as eluents. Appropriate fractions were combined and concentrated. The title compound was obtained as an oil (247 mg, 57%).

### Intermediate 59

### 5-acetyl-8-[(4-methoxybenzyl)oxy]quinolin-2(1H)-one

To a mixture of 14.40 g (0.071 mol) of 5-acetyl-8-hydroxyquinolin-2(1*H*)-one (US 2004 0059116), 6.6 g (0.078 mol) of NaHCO₃ and a catalytic amount of sodium iodide in 360 ml of anhydrous dimethylformamide, stirred at 40°C, a solution of 10.7 ml (12.41 g, 0.079 mol) of 1-(chloromethyl)-4-methoxybenzene in 47 ml of anhydrous dimethylformamide was added in a period of 4 hours. The obtained mixture was stirred at 40°C overnight. After this reaction time, 3.3 g (0.039 mol) of NaHCO₃ were added and then a solution of 5.35 ml (6.21 g, 0.040 mol) of 1-(chloromethyl)-4-methoxybenzene in 23.5 ml of anhydrous dimethylformamide was added, at 40°C, in a period of 4 hours. The stirring was continued overnight maintaining the temperature. The solvent was removed under reduced pressure. The residue was treated with water and the obtained solid was filtered and washed with ethyl acetate. The solid was dissolved in dichloromethane and the obtained solution washed with water, dried (Na₂SO₄, MgSO₄) and concentrated. The title compound was obtained as a solid which was treated with diethyl ether and filtered. (18.5 g, 80%).

### Intermediate 60

### {8-[(4-methoxybenzyl)oxy]-2-oxo-1,2-dihydroquinolin-5-yl}(oxo)acetaldehyde

A mixture of 2.5 g (7.73 mmol) of Intermediate 59, 39 ml of dioxane, 1.7 ml of water and 1.28 g (11.60 mmol) of selenium dioxide was stirred overnight at reflux temperature. The warm solution was then filtered trough celite®. The first filtrate was discarded. The solids were washed with hot dioxane several times and the filtrates were combined and concentrated under reduced pressure. The title compound was obtained as a solid. (2.01 g, 73%).

### Intermediate 61

### 1-(3-{[[2-({6-[(2-hydroxy-2-{8-[(4-methoxybenzyl)oxy]-2-oxo-1,2-dihydroquinolin-5-yl}ethyl)amino]hexyl}oxy)ethyl](methyl)amino]methyl}phenyl)pyrimidine-2,4(1H,3H)-dione

A solution of 213 mg (0.569 mmol) of Intermediate 60 and 263 mg (0.740 mmol) of Intermediate 58 in 3 ml of dimethylsulfoxide was stirred at room temperature for 3 hours under N₂ atmosphere. The reaction mixture was diluted with 3 ml of methanol, cooled to 0-5°C and 86.1 mg of NaBH₄ (2.276 mmol) were added in two portions. The reaction mixture was stirred 15 minutes at 0-5°C and 16 hours at room temperature under N₂ atmosphere. The reaction was diluted with 30 ml of tetrahydrofuran, 30 ml of saturated NH₄Cl solution were added and the mixture was stirred at room temperature for 15 min. The organic layer was separated and the aqueous layer was extracted with tetrahydrofuran (x2). The organic extracts were combined, dried (MgSO₄), and concentrated under reduced pressure. The obtained residue was diluted with ethanol and concentrated (x2). The obtained residue was diluted with chloroform and methanol and concentrated. The oil obtained was purified by column chromatography on silica gel performing a gradient elution using chloroform/methanol (9:1)→ chloroform/methanol/ammonium hydroxide (90:10:1→80:20:2) as eluents. Appropriate fractions were combined and concentrated. The title compound was obtained as a dry foam (253 mg, 64%).

### Example 13

### 1-(3-{[{2-[(6-{[2-hydroxy-2-(8-hydroxy-2-oxo-1,2-dihydroquinolin-5-yl)ethyl]amino} hexyl)oxy]ethyl}(methyl)amino]methyl}phenyl)pyrimidine-2,4(1H,3H)-dione

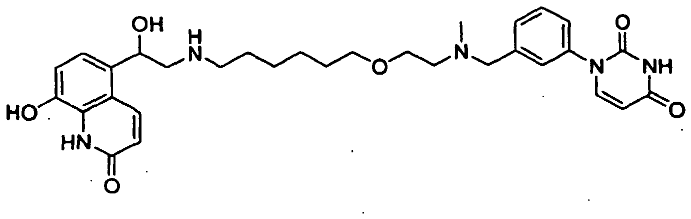

To a solution of 245 mg (0.351 mmol) of Intermediate 61 in 5 ml of dichloromethane, 0.270 ml (3.634 mmol) of trifluoroacetic acid were added. The mixture was stirred at room temperature, under N₂, for 3.5 hours. After this time 0.136 ml (1.831 mmol) more of trifluoroacetic acid were added and the mixture was stirred 17 hours at room temperature. The mixture was diluted with chloroform and solvents were removed under reduced pressure. The obtained residue was diluted with a mixture chloroform/methanol/ammonium hydroxide (80:20:2) and concentrated (x2) and the resulting product diluted with chloroform and concentrated again. The residue was purified by column chromatography on silica gel performing a gradient elution using chloroform/methanol (9:1)→ chloroform/methanol/ammonium hydroxide (90:10:1→80:20:2) as eluents . Appropriate fractions were combined and concentrated. The title compound was obtained as an oil (66 mg, 33%).

¹H-NMR (400 MHz, d₆-DMSO) δ ppm: 1.21-1.32 (m, 4H); 1.33-1.41 (m, 2H); 1.41-1.50 (m, 2H); 2.18 (s, 3H); 2.46-2.58 (m, 4H); 2.61-2.74 (m, 2H); 3.34. (t, *J*=6.7 Hz, 2H); 3.48 (t, *J*=5.9 Hz, 2H); 3.55 (s, 2H); 4.98- 5.05 (m, 1H); 5.66 (d, *J*=7.8 Hz, 1H); 6.49 (d, *J*=9.8 Hz, 1H); 6.90 (d, *J*=8.2 Hz, 1H); 7.06 (d, *J*=8.2 Hz, 1H); 7.26-7.30 (m, 1H); 7.31-7.36 (m, 2H); 7.39-7.45 (m, 1H); 7.69 (d, *J*=7.8 Hz, 1H); 8.16 (d, *J*=10.2 Hz, 1H).
MS: 578 [M+1]⁺

### Intermediate 62

### Methyl 3-mercaptobenzoate

To a solution of 3 g (0.01946 mol) of 3-mercaptobenzoic acid in 30 ml of methanol, cooled to 0-5°C, 0.53 ml of concentrated sulphuric acid (95-98%) were carefully added. The reaction mixture was warmed to room temperature and then refluxed for 2.5 hours. After this time, the mixture was cooled to 0-5°C and 0.13 ml more of concentrated sulphuric acid (95-98%) were carefully added. The reaction mixture was refluxed for 1.5 hours and then stirred at room temperature for 16 hours. The solvent was removed under reduced pressure and the residue was dissolved in ethyl acetate. The obtained solution was washed with 4% aqueous solution of NaHCO₃ (x2), water and brine. The organic layer was separated, dried (MgSO₄), and concentrated. The title compound was obtained as an oil. (2.81 g, 85.9%).

### Intermediate 63

### Methyl 3-(cyclopentylthio)benzoate

A solution of 2.35 g of Intermediate 62 (0.0140 mol), 1.50 ml of bromocyclopentane (2.08 g, 0.0140 mol) and 4.55 g of Cs₂CO₃ (0.0140 mol) in 95 ml of acetonitrile was stirred for 3 hours at room temperature. After this reaction time, the solvent was removed by concentration under reduced pressure. The obtained residue was treated with water and extracted with ethyl acetate (x2). The organic extracts were combined, washed with water and brine, dried (MgSO₄), and concentrated. The residue was purified by column chromatography on silica gel performing a gradient elution using
chloroform/hexane (1:1) → chloroform → chloroform/methanol (50:1 to 15:1) as eluents. Appropiate fractions were combined and concentrated. The title compound was obtained as an oil (2.18 g, 65.9%).

### Intermediate 64

### Methyl 3-(cyclopentylsulfonyl)benzoate

To a solution of 2.16 g of Intermediate 63 (0.00914 mol) in 54 ml of dichloromethane, cooled using an ice-water bath, 4.10 g of 3-chloroperoxybenzoic acid (0.01829 mol) were added in several portions. The reaction mixture was warmed to room temperature and stirred for 64 hours. A white solid was formed during the process. The reaction mixture was diluted with dichloromethane (the white solid was dissolved) and the obtained solution was washed with 4% aqueous solution of NaHCO₃ (checking the basic pH of the aqueous layer) and with water. The organic layer was separated, dried (MgSO₄), and concentrated. The obtained residue was purified by column chromatography on silica gel using chloroform as eluent. The title compound was obtained as an oil. (2.14 g, 87.3%).

### Intermediate 65

### 3-(Cyclopentylsulfonyl)benzoic acid

To a solution of 1945 mg (7.249 mmol) of Intermediate 64 in 95 ml of tetrahydrofuran and 95 ml of water, 346 mg (14.447 mmol) of lithium hydroxide were added. The reaction mixture was vigourously stirred at room temperature for 16 hours. The tetrahydrofuran was removed by concentration under reduced pressure. The obtained residue was diluted with 150 ml of water and the aqueous solution acidified (pH=3) using HCl 2N. The acidic aqueous solution was extracted with chloroform (2 x 100 ml). The organic extracts were combined, dried (MgSO₄), and concentrated under reduced pressure to obtain the title compound as a white solid (1570 mg, 85.2%).

### Intermediate 66

### 3-(Cyclopentylsulfonyl)-N-methylbenzamide

To a solution of 1410 mg (5.545 mmol) of Intermediate 65 in 47 ml of chloroform (ethanol free), cooled to 0-5°C, three drops of anhydrous dimethylformamide and 0.75 ml (1128 mg, 8.887 mmol) of oxalyl chloride were added. The reaction mixture was stirred 15 minutes at 0-5°C and 2 hours at room temperature, then was concentrated at reduced pressure. Chloroform (ethanol free) was added to the residue and the solution was concentrated again (x2). The obtained residue was dissolved in 28 ml of anhydrous tetrahydrofuran and cooled to -30°C. A solution of 19 ml (38 mmol) of commercially available 2M solution of methyl amine in tetrahydrofuran, diluted with 20 ml of tetrahydrofuran, was slowly added. The reaction mixture was left to warm to room temperature and the stirring was continued for 16 hours. After this reaction time, the reaction mixture was concentrated under reduced pressure. The obtained residue was dissolved in chloroform and the organic solution was washed with water (x2). The organic phase was separated, dried (MgSO₄), and concentrated under reduced pressure. Chloroform was added to the residue and the solution was concentrated to dryness (x2).The title product was obtained as an oil (1467 mg, 99%).

### Intermediate 67

### [3-(Cyclopentylsulfonyl)benzyl]methylamine

To a solution of 1467 mg (5.487 mmol) of Intermediate 66 in 130 ml of anhydrous tetrahydrofuran, under N₂ atmosphere, 19.2 ml (19.20 mmol) of a Borane-Tetrahydrofuran complex (1M solution in tetrahydrofuran) were added slowly at room temperature. The reaction mixture was refluxed for 2.5 hours. After this reaction time, the mixture was cooled to room temperature and a new amount, 9.6 ml (9.60 mmol), of a Borane-Tetrahydrofuran complex (1M solution in tetrahydrofuran) was slowly added. The reaction mixture was refluxed for 1.5 hours and stirred at room temperature overnight. After this reaction time, methanol (6.7 ml) was added drop by drop, and the obtained mixture was refluxed for 30 minutes. Solvents were removed by concentration under reduced pressure. The obtained residue was dissolved in 175 ml of methanol, and 2 ml of concentrated HCl were added. The mixture was refluxed for 30 min. After cooling, the solvent was removed under reduced pressure. The obtained residue was treated with 300 ml of water and extracted with diethyl ether (1 x 60 ml). The aqueous phase was separated and basified with solid K₂CO₃. The basic solution was extracted with chloroform (3 x 75 ml). The organic extracts were combined, dried (MgSO₄), and concentrated. The obtained residue was purified by column chromatography on silica gel performing a gradient elution using chloroform/methanol (25:1 → 4:1) as eluents. Appropiate fractions were combined and concentrated to dryness to give the title compound as an oil. (1102 mg, 79.3%).

### Intermediate 68

### 2-(6-{2-[[3-(Cyclopentylsulfonyl)benzyl](methyl)amino]ethoxy}hexyl)-1H-isoindole-1,3(2H)-dione

A mixture of 1102 mg (4.350 mmol) of Intermediate 67, 1459 mg (3.949 mmol) of Intermediate 4 and 387 mg (4.607 mmol) of NaHCO₃, in 10 ml of anhydrous dimethylformamide, was stirred for 22 hours, at 70°C , under N₂ atmosphere. The reaction mixture was cooled to room temperature and the solvent was removed under reduced pressure. The residue was treated with water and extracted with ethyl acetate (x3). The organic extracts were combined, washed with water (x2) and brine, dried (MgSO₄), and concentrated. The obtained residue was purified by column chromatography on silica, gel performing a gradient elution using chloroform/methanol (100:1 → 4:1) as eluents. Appropiate fractions were combined and concentrated. The title product was obtained as an oil. (1438 mg, 69.1%).

### Intermediate 69

### 6-{2-[[3-(Cyclopentylsulfonyl)benzyl](methyl)amino]ethoxy}hexan-1-amine

To a solution of 929 mg (1.764 mmol) of Intermediate 68 in 19 ml of ethanol, 0.32 ml (0.33 g, 6.59 mmol) of hydrazine monohydrate were added in several portions following the reaction advance by HPLC-MS. The reaction mixture was stirred 8 hours at reflux temperature and 78 hours at room temperature. A white solid was formed during the process. The reaction mixture was diluted with 5 ml of ethanol and filtered. The solid was washed with a mixture of diethyl ether/ethanol 4:1 and discarded. The filtrates were combined and concentrated. The obtained residue was treated with 60 ml of chloroform to obtain a solid which was filtered, washed with chloroform and discarded. The filtrates were combined and concentrated. The obtained residue was purified by column chromatography on silica gel performing a gradient elution using chloroform/methanol (15:1) → chloroform/methanol/ammonium hydroxide (90:10:1→ 80:20:2) as eluents. Appropiate fractions were combined and concentrated. The title compound was obtained as an oil (382 mg, 54.6%).

### Intermediate 70

### 8-(Benzyloxy)-5-{(1R)-1-{[tert-butyl(dimethyl)silyl]oxy}-2-[(6-{2-[[3-(cyclopentyl-sulfonyl)benzyl](methyl)amino]ethoxy}hexyl)amino]ethyl}quinolin-2(1H)-one

To a solution of 538 mg (1.10 mmol) of 8-(benzyloxy)-5-((1R)-2-bromo-1-{[tert-butyl(dimethyl)silyl]oxy}ethyl)quinolin-2(1H)-one (US20040059116) and 437 mg (1.10 mmol) of Intermediate 69 in 1.31 ml of anhydrous dimethyl sulfoxide, 278 mg (3.31 mmol) of NaHCO₃ and 247 mg (1.65 mmol) of Nal were added. The mixture was stirred at 120 °C for 2 hours under N₂ atmosphere. The reaction mixture was cooled, treated with water, and extracted with ethyl acetate (x 2). The organic extracts were combined and washed with water and brine, dried over MgSO₄, and concentrated to dryness. The residue was purified by column chromatography on silica gel performing a gradient elution using chloroform/methanol (75:1 → 15:1) as eluents. Appropiate fractions were combined and concentrated to dryness. The title compound was obtained as an oil. (399 mg, 45%).

### Intermediate 71

### 8-(Benzyloxy)-5-{(1R)-2-[(6-{2-[[3-(cyclopentylsulfonyl)benzyl](methyl)amino]ethoxy} hexyl)amino]-1-hydroxyethyl}quinotin-2(1H)-one

To a solution of 427 mg (0.531 mmol) of Intermediate 70 in 8.5 ml of tetrahydrofuran, 671 mg (0.67-1.0 mmol) of tetrabutylammonium fluoride on SiO₂ (capacity (F⁻:1.0-1.5 mmol /g) were added. The mixture was stirred at room temperature, under N₂ atmosphere, for 22 hours. The reaction mixture was diluted with tetrahydrofuran and filtered. The solid was washed with tetrahydrofuran and with a mixture of chloroform/methanol/ammonium hydroxide (90:10:1). The filtrates were combined and concentrated under reduced pressure. The obtained residue was purified by column chromatography on silica gel, performing a gradient elution using chloroform/methanol (15:1 to 4:1) → chloroform/methanol/ammonium hydroxide (90:10:1) as eluents. The title compound was obtained as an oil (328 mg, 89.6%).

### Example 14

### 5-{(1R)-2-[(6-{2-[[3-(Cyclopentylsulfonyl)benzyl](methyl)amino]ethoxy}hexyl)amino]-1-hydroxyethyl}-8-hydroxyquinolin-2(1H)-one

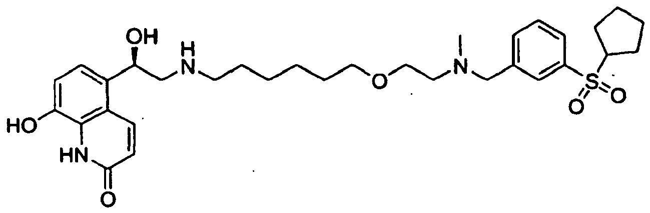

To a solution of 322 mg (0.467 mmol) of Intermediate 71 in 32 ml of methanol, 15 mg of Pd/C (10%) were added, and the mixture was hydrogenated (H₂ balloon pressure) at room temperature following the reaction advance by HPLC-MS. Two more portions of Pd/C (10%), 7 mg and 10 mg, were added to the reaction mixture at 19 hours and 91 hours repectively. The total reaction time was 140 hours. The catalyst was filtered and the solvent was removed under reduced pressure. The resulting oil was purified by column chromatography on silica gel using chloroform/methanol/ammonium hydroxide (90:10:1) as eluents. The title compound was obtained as an oil (112 mg, 40%).

¹H-NMR (300 MHz, DMSO-D6) δ ppm: 1.26-1:37 (m, 4H); 1.37-1.71 (m, 8H); 1.75-1.97 (m, 4H); 2.23 (s, 3H); 2.51-2.63 (m, 4H); 2.65-2.80 (m, 2H); 3.35-3.44 (t, *J*=6.5 Hz, 2H); 3.49-3.60 (t, *J*=5.8 Hz, 2H); 3.68 (s, 2H); 3.72-3.87 (m, 1H); 5.01-5.11 (dd, *J*=7.4 Hz, *J*=4.4 Hz, 1H); 6.54 (d, *J*=9.9 Hz, 1H); 6.95 (d, *J*=8.0 Hz, 1H); 7.11 (d, *J*=8.0 Hz, 1H); 7.60-7.74 (m, 2H); 7.77-7.84 (m, 1H); 7.86 (s, 1H); 8.21 (d, *J*=9.9 Hz, 1H).
MS: 600 [M+1]⁺

### Intermediate 72

### tert-Butyl benzyl(2-hydroxyethyl)carbamate

To a solution of 4.7 ml (5.0 g, 33.1 mmol) of 2-(benzylamino)ethanol in 100 ml of dioxane, cooled to 8-10°C, a solution of 1.32 g (33.1 mmol) of NaOH in 12 ml of water was slowly added. The mixture was maintained at 5°C and a solution of 7.22 g (33.1 mmol) of di-tert-butyl dicarbonate in 50 ml of dioxane was added drop by drop. The reaction mixture was stirred 1 hour at 5°C and at room temperature for 72 hours. After this reaction time, the solvent was removed under reduced pressure. The obtained residue was treated with water and extracted with ethyl acetate several times. The organic extracts were combined, dried (MgSO₄), and concentrated to dryness. The resulting product was purified by column chromatography on silica gel performing a gradient elution using hexane/ethyl acetate (90:10 → 50:50) as eluents. Appropiate fractions were combined and concentrated. The title compound was obtained as an oil. (6.51 g, 78.4%).

### Intermediate 73

### tert-Butyl benzyl{2-[(6-bromohexyl)oxy]ethyl}carbamate

A mixture of 6.51 g (25.9 mmol) of Intermediate 72, 11.9 ml (18.9 g , 77.7 mmol) of 1,6-dibromohexane, 250 mg (0.78 mmol) of tetrabutylammonium bromide and 19.5 ml of a 32% (w/v) NaOH solution, were mechanically stirred at room temperature for 16 hours. After this reaction time, the mixture was treated with 100 ml of water. The organic phase was separated and the aqueous phase was extracted with diethyl ether (2 x 125 ml). All the organic phases were combined, dried (MgSO₄), and concentrated. The obtained residue was purified by column chromatography. Initially, hexane was used as eluent to separate the residual 1,6-dibromohexane. The column chromatography was continued using hexane/ethyl acetate (95:5) to elute the title compound which was obtained as an oil. (7.5 g, 69.8%).

### Intermediate 74

### tert-Butyl benzyl(2-{[6-(1,3-dioxo-1,3-dihydro-2H-isoindol-2-yl)hexyl]oxy}ethyl) carbamate

To a solution of 7.5 g (18.19 mmol) of Intermediate 73 in 33 ml of anhydrous dimethylformamide, 3.88 g (20.92 mmol) of potassium phtalimide were added. The mixture was stirred at 75°C for 6 hours. The solvent was removed under reduced pressure. The residue was treated with water (250 ml) and extracted with diethyl ether (3 x 125 ml). The organic extracts were combined, washed with water (100 ml) and brine (100 ml), dried (MgSO₄), and concentrated. The obtained residue was purified by column chromatography on silica gel using hexane/ethyl acetate (80:20) as eluents. The title product was obtained as an oil (7.27 g, 83.6%).

### Intermediate 75

### tert-Butyl (2-[(6-aminohexyl)oxy]ethyl}benzylcarbamate

To a solution of 7.27 g (15.14 mmol) of Intermediate 74 in 91 ml of ethanol, 1.29 ml (25.73 mmol) of hydrazine monohydrate were added. The mixture was stirred at 100°C for 5 hours and at room temperature overnight. The solvent was removed under reduced pressure. The obtained residue was treated with chloroform and the solid obtained was filtered, washed with chloroform and discarded. The filtrates were combined and concentrated. Chloroform was added to the residue and the treatment was repeated (x3). The obtained residue was purified by column chromatography on silica gel using chloroform/methanol/ammonium hydroxide (90:10:1) as eluents. The title compound was obtained as an oil. (4.13 g, 77.9%).

### Intermediate 76

### tert-Butyl benzyl[2-({6-[(2-hydroxy-2-{8-[(4-methoxybenzyl)oxy]-2-oxo-1,2-dihydroquinolin-5-yl}ethyl)amino]hexyl}oxy)ethyl]carbamate

A solution of 1200 mg (3.42 mmol) of Intermediate 75, and 1582 mg (4.45 mmol) of Intermediate 60, in 26.7 ml of anhydrous dimethyl sulfoxide, was stirred at room temperature for 5 hours under N₂ atmosphere. After this reaction time, 16 ml of methanol were added, and the obtained mixture cooled to 0-5°C. Sodium borohydride, 388 mg (10.26 mmol), was added in several portions. The reaction mixture was stirred 20 minutes at 0-5°C and at room temperature overnight (under N₂). The reaction mixture was diluted with 200 ml of tetrahydrofuran and the obtained solution was washed with 200 ml of saturated aqueous NH₄Cl solution. The aqueous solution was separated and extracted with tetrahydrofuran (x2). The tetrahydrofuran extracts were combined and concentrated under reduced pressure. Ethanol was added to the residue and concentrated. The obtained residue was diluted with chloroform and concentrated again. The obtained oil was purified by column chromatography on silica gel performing a gradient elution using chloroform/methanol (75:1 → 50:1) as eluents. The title compound was obtained as an oil. (600 mg, 26%).

### Example 15

### 5-[2-({6-[2-(benzylamino)ethoxyl]hexyl}amino)-1-hydroxyethyl]-8-hydroxyquinolin-2(1H)-one

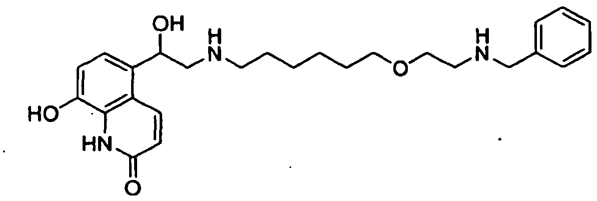

To a solution of 480 mg (0.71 mmol) of intermediate 76 in 15 ml of dried dichloromethane, 0.547 ml (7.1 mmol) of trifluoroacetic acid were added. The mixture was stirred at room temperature for 2 hours and 0.547 ml (7.1 mmol) of trifluoroacetic acid were added again. The stirring was continued for 1 hour. After this reaction time, the solvent was removed under reduced pressure. The residue was diluted with dichloromethane and concentrated (x2). The obtained residue was diluted with a mixture chloroform/methanol/ammonium hydroxide (80:20:2) and concentrated (x3). The residue was diluted with dichloromethane and concentrated again (x2). The obtained residue was purified by column chromatography on silica gel (25 g) performing a gradient elution using chloroform/methanol (15:1 to 9:1) → chloroform/methanol/ammonium hydroxide (90:10:1) as eluents. Appropiate fractions were combined and concentrated. The resulting product was purified again by column chromatography on silica gel (Varian Bond Elut Si, 10 g) using chloroform/methanol/ammonium hydroxide (95:5:0.5) → (90:10:1) as eluents. The title product was obtained as an oil. (88 mg, 27.2%).

¹H-NMR (300 MHz, DMSO-D6) δ ppm: 1.27-1.38 (m, 4H); 1.39-1.49 (m, 2H); 1.49-1.60 (m, 2H); 2.53-2.65 (m, 2H); 2.65-2.82 (m, 4H); 3.36-3.44 (t, *J* =6.5 Hz, 2H); 3.45-3.53 (t, *J* =5.8 Hz, 2H); 3.76 (s, 2H); 5.04-5.13 (dd, *J* =7.6, *J* =4.3 Hz, 1H); 6.52-6.60 (m, 1H); 6.93-7.01 (m, 1H); 7.09-7.16 (m, 1H); 7.23-7.32 (m, 1H); 7.32-7.41 (m, 4H); 8.20-8.28 (m, 1H).
MS: 454 [M+1]+

### Intermediate 77

### 3-[{(13R)-13-[4-(benzyloxy)-3-(formylamino)phenyl]-15,15,16,16-tetramethyl-4,14-dioxa-11-aza-15-silaheptadec-1-yl}(methyl)amino]benzamide

A mixture of 2.78 g (5.99 mmol) of [2-(Benzyloxy)-5-((1R)-2-bromo-1-{[tert-butyl(dimethyl)silyl]oxy}ethyl)phenyl]formamide (WO 2004/011416 Intermediate 4), 2.03 g (6.59 mmol) of Intermediate 45, 2.5 g (17.94 mmol) of K₂CO₃ and 1.0 g (6.60 mmol) of Nal in 7.8 ml of dried dimethyl sulfoxide, was stirred 1 hour at 120°C. After this time, the reaction mixture was cooled, treated with 100 ml of water and extracted with ethyl acetate (2x100 ml). The organic extracts were combined, washed with water (100 ml) and brine (100 ml), dried (MgSO₄), and concentrated under reduced pressure. The obtained residue was purified by column chromatography on silica gel, eluting with chloroform/methanol (98:2 → 95:5). The yield was 2.24 g (54%) of the title compound.

### Intermediate 78

### 3-[(3-{[6-({(2R)-2-[4-(Benzyloxy)-3-(formylamino)phenyl]-2-hydroxyethyl}amino) hexyl]oxy}propyl)(methyl)amino]benzamide

To a solution of 1.61 g (2.32 mmol) of Intermediate 77 in 29 ml of dried tetrahydrofuran, 1.11 g (3.50 mmol) of tetrabutylammonium fluoride trihydrate were added. The mixture was stirred 17 hours at room temperature under N₂ atmosphere. After this reaction time, the solvent was removed under reduced pressure. The obtained residue was diluted with ethyl acetate and the obtained solution was washed with NaHCO₃ (4% aqueous solution), water and brine, dried (MgSO₄), and concentrated. The obtained residue was purified by column chromatography on silica gel eluting with chloroform/methanol (95:5 → 90:10). The yield was 989 mg (73.7%) of the title compound.

### Example 16

### 3-[(3-{[6-({(2R)-2-[3-(Formylamino)-4-hydroxyphenyl]-2-hydroxyethyl}amino)hexyl] oxy}propyl)(methyl)amino]benzamide

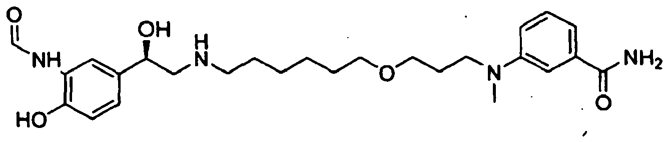

To a solution of 989 mg (1.71 mmol) of Intermediate 78 in 20 ml of ethanol, 98 mg of Pd/C (10%) were added. The mixture was hydrogenated at room temperature at 0,207 MPa for 17 hours. The catalyst was filtered and the filtrate was concentrated under reduced pressure. The obtained residue was purified by column chromatography on silica gel performing a gradient elution using chloroform → chloroform/methanol (90:10) → chloroform/methanol/ammonium hydroxide (90:10:1) as eluents. Appropiate fractions were combined and concentrated. The title compound was obtained as a dry foam (562 mg, 67.4%).

¹H-NMR (300 MHz, METHANOL-D4) δ ppm: 1.28-1.47 (m, 4H); 1.48-1.67 (m, 4H); 1.74-1.91 (m, 2H); 2.62-2.73 (m, 2H); 2.75-2.87 (m, 2H); 2.97 (s, 3H); 3.37-3.55 (m, 6H); 4.66-4.73 (m, 1H); 6.80-6.85 (m, 1H); 6.88-6.94 (m, 1H); 6.96-7.03 (m, 1H); 7.06-7.13 (m, 1H); 7.18-7.31 (m, 3H); 8.05 (s, 1H); 8.29 (s, 1H).
MS: 487 [M+1]+

### Intermediate 79

### tert-Butyl benzyl{2-[(6-{[2-(2,2-dimethyl-4H-1,3-benzodioxin-6-yl)-2-hydroxyethyl] amino}hexyl)oxy]ethyl}carbamate

To a solution of 0.68 g (1.64 mmol) of Intermediate 73 and 0.43 g (1.92 mmol) of 2-amino-1-(2,2-dimethyl-4H-1,3-benzodioxin-6-yl)ethanol (WO 2006/122788) in 30 ml of acetonitrile, 0.25 g (1.81 mmol) of K₂CO₃ were added. The mixture was stirred at 85-90°C for 6.5 hours and the stirring was continued at room temperature overnight. The solvent was removed under reduced pressure and the obtained residue was treated with water. The aqueous solution was extracted with ethyl acetate (x3). The organic extracts were combined and washed with water and brine, dried (MgSO₄), and concentrated. The residue was purified by column chromatography on silica gel performing a gradient elution using chloroform → chloroform/methanol (90:10) → chloroform/methanol/ammonium hydroxide (90:10:1) (only at the end) as eluents. Appropiate fractions were combined and concentrated. The title compound was obtained as an oil (468 mg, 51.2%).

### Example 17

### 4-[2-({6-[2-(Benzylamino)ethoxy]hexyl}amino)-1-hydroxyethyo-2-(hydroxymethyl) phenol

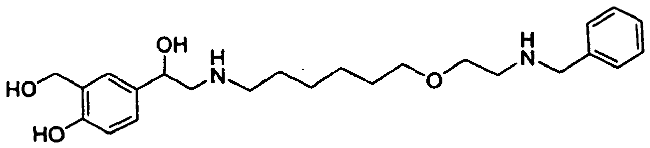

To a solution of 650 mg (1.17 mmol) of Intermediate 79 in 7 ml of ethyl acetate, 7 ml of a 2N HCl aqueous solution were added. The reaction mixture was vigourously stirred at room temperature for 20 hours. After this reaction time, the mixture was diluted with water and solid K₂CO₃ was added (to pH=10) while stirring. The obtained mixture was extracted with ethyl acetate (x2). The extracts were combined, dried (MgSO₄), and evaporated.The obtained residue was purified by column chromatography on silica gel eluting with chloroform/methanol/ammonium hydroxide (97:3:0.3).
The obtained product (217 mg) was dissolved in 25 ml of 1 N HCl, and the acidic solution was extracted with diethyl ether (10 ml) and with ethyl acetate (10 ml). The aqueous solution was neutralized (pH=7-8) using solid K₂CO₃, and extracted with a small volume of diethyl ether and with a small volume of ethyl acetate. The resulting aqueous solution was saturated with NaCl and exhaustively extracted with ethyl acetate. The ethyl acetate extracts were combined, dried (MgSO₄), and concentrated to dryness, 65 mg (13.4%) of the title product were obtained.

¹H-NMR (300 MHz, METHANOL-d4) δ ppm: 1.27-1.46 (m, 4H); 1.46-1.64 (m, 4H); 2.60-2.71 (m, 2H); 2.71-2.85 (m, 4H); 3.40-3.48 (m, 2H); 3.50-3.59 (m, 2H); 3.77 (s, 2H); 4.65 (s, 2H); 4.68-4.75 (m, 1H); 6.72-6.79 (m, 1H); 7.08-7.14 (m, 1H); 7.23-7.38 (m, 6H).
MS: 417 [M+1]+

### Intermediate 80

### 1-(3-{(14R)-14-[8-(Benzyloxy)-2-oxo-1,2-dihydroquinolin-5-yl]-2,16,16,17,17-penta-methyl-5,15-dioxa.2,12-diaza-16-silaoctadec-1-yl}phenyl)pyrimidine-2,4(1H,3H)-dione

To a solution of 2086 mg (4.270 mmol) of 8-(benzyloxy)-5-((1R)-2-bromo-1-{[tert-butyl(dimethyl)silyl]oxy}ethyl)quinolin-2(1H)-one (US20040059116) and 1605 mg (4.285 mmol) of Intermediate 58, in 5.1 ml of anhydrous dimethyl sulfoxide, 401 mg (4.773 mmol) of NaHCO₃ and 64 mg (0.427 mmol) of Nal were added. The mixture was stirred at 140°C for 1 hour, under N₂ atmosphere. The reaction mixture was treated with water (100 ml) and extracted with ethyl acetate (2 x 75 ml). The organic extracts were combined and washed with water (x2) and brine, dried over MgSO₄, and concentrated to dryness. The residue was purified by column chromatography on silica gel performing a gradient elution using chloroform/methanol (25:1 to 10:1) as eluents. Appropiate fractions were combined and concentrated to dryness. The tile compound was obtained as an oil. (1448 mg, 43.4%).

### Intermediate 81

### 1-(3-{[(2-{[6-({(2R)-2-[8-(Benzyloxy)-2-oxo-1,2-dihydroquinolin-5-yl]-2-hydroxyethyl} amino)hexyl]oxy}ethyl)(methyl)amino]methyl}phenyl)pyrimidine-2,4(1H,3H)-dione

To a solution of 1442 mg (1.844 mmol) of Intermediate 80 in 13.1 ml of tetrahydrofuran, 873 mg (2.767 mmol) of tetrabutylammonium fluoride trihydrate were added. The mixture was stirred at 40°C, under nitrogen atmosphere, for 1 hour. The solvent was removed under reduced pressure. The residue was treated with water and extracted with ethyl acetate (x2). The organic extracts were combined and washed with water (x2) and brine, dried over MgSO₄, and concentrated to dryness. The title compound was obtained as a dry foam (1095 mg, 88.9%) and was used in the following step without further purification.

### Example 18

### 1-(3-{[{2-[(6-{[(2R)-2-hydroxy-2-(8-hydroxy-2-oxo-1,2-dihydroquinolin-5-yl)ethyl] amino}hexyl)oxy]ethyl}(methyl)amino]methyl}phenyl)pyrimidine=2,4(1H,3H)-dione

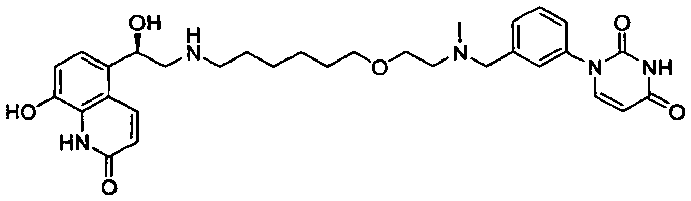

To a solution of 1089 mg (1.631 mmol) of Intermediate 81 in 110 ml of methanol, 54 mg of Pd/C (10%) were added, and the mixture was hydrogenated (H₂ balloon pressure) at room temperature following the reaction advance by HPLC-MS. Two more portions of 27 mg of Pd/C (10%), were added to the reaction mixture at 40 hours and 112 hours repectively. The total reaction time was 156 hours. The catalyst was filtered and the solvent was removed under reduced pressure. The resulting oil was purified by column chromatography on silica gel using chloroform/methanol/ammonium hydroxide (90:10:1 → 85:15:1.5 → 80:20:2) as eluents. Appropiate fractions were combined and evaporated. The title compound was obtained as dry foam (557 mg, 59.1%).

¹H-NMR (300 MHz, DMSO-d₆) δ ppm: 1.21-1.32 (m, 4H); 1.36-1.40 (m, 2H); 1.43-1.48 (m, 2H); 2.18 (s, 3H); 2.46-2.58 (m, 4H); 2.64-2.74 (m, 2H); 3.32-3.36 (t, 2H); 3.46-3.49 (t, 2H); 3.55 (s, 2H); 4.99-5.06 (m, 1H); 5.66 (d, *J* =7.9 Hz, 1H); 6.49 (d, *J* =9.9 Hz, 1H); 6.91 (d, *J* =8.2 Hz, 1H); 7.06 (d, *J* =8.0 Hz, 1H); 7.25-7.31 (m, 1H); 7.31-7.37 (m, 2H); 7.38-7.46 (m, 1H); 7.69 (d, *J* =7.7 Hz, 1H); 8.17 (d, *J* =9.9 Hz, 1H).
MS: 578 [M+1]+

### Intermediate 82

### 3-[(tert-Butylamino)sulfonyl]benzoic acid

To a solution of 5.0 g (0.0227 mol) of 3-(chlorosulfonyl)benzoic acid in 80 ml of dichloromethane at 0°C, 8.4 ml (0.0799 mol) of tert-butilamine dissolved in 20 ml of dichloromethane were added. The mixture was stirred at 0°C for 30 minutes and at room temperature for 2 hours. A white solid was formed during the reaction. The solid was filtered and washed with 10 ml of dichloromethane. The filtrate was discarded. The solid was poured into 100 ml of water and 5N aqueous HCl was added slowly (while stirring) until acidic pH of the solution. The mixture was stirred at room temperature for 30 minutes. A new solid was formed which was filtered, washed with water and dried in a vacuum oven at 45°C (P₂O₅). 4.21 g (72.1%) of the title compound were obtained. (Product described in WO 99/24461).

### Intermediate 83

### 3-[(tert-Butylamino)sulfonyl]-N-methylbenzamide

To a solution of 4.2 g (0.0163 mol) of Intermediate 82 in 135 ml of chloroform (ethanol free), at 0-5°C, four drops of anhydrous dimethylformamide and 2.24 ml (0.0265 mol) of oxalyl chloride were added. The reaction mixture was stirred 15 minutes at 0-5°C and 2 hours at room temperature. After this reaction time, the mixture was concentrated under reduced pressure. Chloroform (ethanol free) was added to the residue and concentrated again (x2). The obtained residue was dissolved in 82 ml of anhydrous tetrahydrofuran and cooled to -30°C. At this temperature, 56 ml (0.1120 mol) of commercially available 2M solution of methyl amine in tetrahydrofuran, were slowly added. The reaction mixture was left to warm to room temperature and stirred for 16 hours. The solvent was evaporated. The obtained residue was dissolved in chloroform and the solution was washed with water (x2), dried (MgSO₄), and concentrated. Chloroform was added to the residue and concentrated again (x2). The title product was obtained as solid (3.94 g, 89.3%).

### Intermediate 84

### N-(tert-Butyl)-3-[(methylamino)methyl]benzenesulfonamide

To a solution of 396 mg (1.465 mmol) of Intermediate 83 in 34 ml of anhydrous tetrahydrofuran, under N₂ atmosphere, 5.12 ml (5.12 mmol) of Borane-Tetrahydrofuran complex (1M solution in tetrahydrofuran) were added slowly at room temperature. The reaction mixture was refluxed for 16 hours. After this reaction time, the mixture was cooled to room temperature and a new amount, 5.12 ml (5.12 mmol), of a Borane-Tetrahydrofuran complex (1M solution in tetrahydrofuran) was added slowly. The reaction mixture was refluxed for 5 hours and the stirring was continued at room temperature 16 hours. Methanol (2.38 ml) was added drop by drop and the mixture was refluxed for 30 minutes. After cooling to room temperature, solvents were evaporated. The obtained residue was dissolved in 67 ml of methanol, 0.53 ml of concentrated aqueous HCl were slowly added, and the mixture was refluxed for 30 minutes. Solvents were evaporated. The obtained residue was treated with 38 ml of water and extracted with diethyl ether (26 ml) to remove some impurities. The acidic aqueous phase was separated and basified with solid K₂CO₃. The basic solution was extracted with chloroform (x3). The organic extracts were combined, dried (MgSO₄), and concentrated. The title compound was obtained as an oil (320 mg, 85.3%).

### Intermediate 85

### N-(tert-Butyl)-3-{[(2-{[6-(1,3-dioxo-1,3-dihydro-2H-isoindol-2-yl)hexyl]oxy}ethyl) (methyl)amino]methyl}benzenesulfonamide

To a solution of 675 mg (1.83 mmol) of Intermediate 4 and 469 mg (1.83 mmol) of Intermediate 84, in 4.5 ml of anhydrous dimethylformamide, 178 mg (2.12 mmol) of NaHCO₃ were added and the mixture was vigorously stirred at 70°C for 20 hours (under N₂ atmosphere). A new amount of NaHCO₃ (178 mg, 2.12 mmol) was added, and the mixture was stirred at 70°C for 6 hours more. The reaction mixture was cooled to room temperature, the solvent was removed under reduced pressure and the residue was treated with water and extracted with ethyl acetate (x2). The extracts were combined, washed with water and brine, dried (MgSO₄), and concentrated.

The obtained product was dissolved in 180 ml of diethyl ether and the ether solution was extracted with 2N HCl (3x180 ml). The acidic extracts were combined and basified (pH>12) with solid K₂CO₃. The basic solution was extracted with ethyl, acetate (x3). The organic extracts were combined, washed with brine, dried (MgSO₄), and concentrated to dryness. The title product was obtained as an oil (716 mg, 73.9%)

### Intermediate 86

### 3-{[{2-[(6-Aminohexyl)oxy]ethyl}(methyl)amino]methyl}-N-(tert-butyl)benzene-sulfonamide

To a solution of 709 mg (1.338 mmol) of Intermediate 85 in 7 ml of ethanol, 0.097 ml (1.996 mmol) of hydrazine monohydrate were added. The reaction mixture was stirred at room temperature for 20 hours. The reaction advance was followed by HPLC-MS and 0.175 ml (3.60 mmol) more of hydrazine monohydrate were added, in several portions, in a period of 116 hours. The total reaction time was 136 hours at room temperature. A white solid was formed during the process. The reaction mixture was filtered, the solid was washed with ethanol and discarded. The filtrates were combined and concentrated under reduced pressure. The residue was treated with chloroform and the solid obtained was filtered. The solid was discarded, the filtrate was concentrated, and the obtained residue was purified by column chromatography on silica gel performing a gradient elution using chloroform/methanol (15:1) → chloroform/methanol/ammonium hydroxide (90:10:1 to 80:20:2) as eluents. The title compound was obtained as an oil (286 mg, 53.4%).

### Intermediate 87

### 3-{(14R)-14-[8-(Benzyloxy)-2-oxo-1,2-dihydroquinolin-5-yl]-2,16,16,17,17-penta-methyl-5,15-dioxa-2,12-diaza-16-silaoctadec-1-yl}-N-(tert-butyl)benzenesulfonamide

To a solution of 342 mg (0.701 mmol) of 8-(benzyloxy)-5-((1R)-2-bromo-1-{[tert-butyl(dimethyl)silyl]oxy}ethyl)quinolin-2(1H)-one (US20040059116), and 280 mg (0.701 mmol) of Intermediate 86, in 1 ml of anhydrous dimethyl sulfoxide, 65 mg (0.771 mmol) of NaHCO₃ and 11 mg (0.07 mmol) of Nal were added. The mixture was stirred at 140°C for 1 hour, under N₂ atmosphere. The reaction mixture was cooled to room temperature, treated with water and extracted with ethyl acetate (x2). The organic extracts were combined and washed with water (x2) and brine, dried (MgSO₄), and concentrated to dryness. The residue was purified by column chromatography on silica gel performing a gradient elution using chloroform/methanol (50:1 to 15:1) as eluents. Appropiate fractions were combined and concentrated to dryness. The title compound was obtained as an oil. (253 mg, 44.8%).

### Intermediate 88

### 3-{[(2-{[6-({(2R)-2-[8-(benzyloxy)-2-oxo-1,2-dihydroquinolin-5-yl]-2-hydroxyethyl} amino)hexyl]oxy}ethyl)(methyl)amino]methyl}-N-(tert-butyl)benzenesulfonamide

To a solution of 247 mg (0.306 mmol) of Intermediate 87 in 2.2 ml of tetrahydrofuran, 145 mg (0.459 mmol) of tetrabutylammonium fluoride trihydrate were added. The mixture was stirred at 40°C, under nitrogen atmosphere, for 1 hour. The solvent was removed under reduced pressure. The residue was treated with water and extracted with ethyl acetate (x2). The organic extracts were combined and washed with water (x2) and brine, dried (MgSO₄), and concentrated to dryness. The residue was purified by column chromatography on silica gel performing a gradient elution using chloroform → chloroform/methanol (50:1 to 4:1) as eluents. The title product was obtained as an oil (144 mg, 67.9%).

### Example 19

### N-(tert-Butyl)-3-{[{2-[(6-{[(2R)-2-hydroxy-2-(8-hydroxy-2-oxo-1,2-dihydroquinolin-5-yl)ethyl]amino}hexyl)oxy]ethyl}(methyl)amino]methyl}benzenesulfonamide

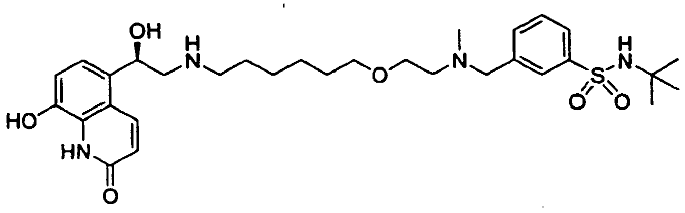

To a solution of 143 mg (0.2064 mmol) of Intermediate 88 in 14 ml of methanol, 7 mg of Pd/C (10%) were added, and the mixture was hydrogenated (H₂ balloon pressure) at room temperature following the reaction advance by HPLC-MS. One more portion of 7 mg of Pd/C (10%), was added to the reaction mixture at 17 hours from the begining. The total reaction time was 85 hours. The catalyst was filtered and the solvent was removed under reduced pressure. The resulting oil was purified by column chromatography on silica gel using chloroform → chloroform/methanol (50:1 to 4:1) → chloroform/methanol/ammonium hydroxide (90:10:1 to 80:20:2) as eluents. The title compound was obtained as dry foam (52 mg, 41.9%).

¹H-NMR (300 MHz, DMSO-d6) δ ppm: 1.06 (s, 9H); 1.21-1.31 (m, 4H); 1.33-1.41 (m, 2H); 1.41-1.51 (m, 2H); 2.16 (s, 3H); 2.45-2.57 (m, 4H); 2.60-2.73 (m, 2H); 3.33 (t, *J* =6.4 Hz, 2H); 3.48 (t, *J* =5.9 Hz, 2H); 3.58 (s, 2H); 4.97-5.04 (m, 1H); 6.49 (d, *J* =9.6 Hz, 1H); 6.90 (d, *J* =8.2 Hz, 1H); 7.06 (d, *J* =8.2 Hz, 1H); 7.46-7.52 (m, 2H); 7.66-7.73 (m, 1H); 7.78 (s, 1H); 8.16 (d, *J* =10.2 Hz, 1H).
MS: 578 [M+1]⁺

### Intermediate 89

### ({2-[(5-Bromopentyl)oxy]ethoxy}methyl)benzene

A mixture of 2-(benzyloxy)etanol (10.0 g, 0.0657 mol), 1,5-dibromopentane (33.3 ml, 0.2447 mol), tetrabutylammonium bromide (1.3 g, 0.0040 mol) and 50 ml (0.40 mol) of aqueous 8N NaOH solution, was vigorously stirred at room temperature for 17 hours. The reaction mixture was partitioned between water and hexane. The hexane phase was separated and washed with water, methanol (small volume), water and brine, dried (MgSO₄), and concentrated. The excess of 1,5-dibromopentane was eliminated by distillation under reduced pressure. The title compound was obtained as a colourless oil (14.74 g, 74.5%).

### Intermediate 90

### 2-{5-[2-(Benzyloxy)ethoxy]pentyl}-1H-isoindole-1,3(2H)-dione

To a solution of 16.118 g (0.0535 mol) of Intermediate 89 in 76 ml of dimethylformamide, 11.894 g (0.0642 mol) of potassium phtalimide and a catalytic amount of trihexyltetradecylphosphonium bromide were added. The mixture was stirred at 75°C for 3 hours and at room temperature overnight. After this reaction time the solvent was removed under reduced pressure. The obtained residue was diluted with water and the obtained solution was extracted with diethyl ether (x3). The organic extracts were combined, washed with water (x2) and brine, dried (MgSO₄), and concentrated. The title compound was obtained as an oil. (17.70g, 90.03%).

### Intermediate 91

### 2-[5-(2-Hydroxyethoxy)pentyl]-1H-isoindole-1,3(2H)-dione

To a solution of 17.70 g (0.0482 mol) of Intermediate 90 in 90 ml of methanol and 70 ml of ethyl acetate , 0.9 g of Pd/C (10%) were added. The mixture was hydrogenated at room temperature at 0,262 MPa for 26 hours. The catalyst was filtered and the filtrate was concentrated under reduced pressure. The title compound was obtained as an oil (13.61 g, quantitative yield).

### Intermediate 92

### 2-{[5-(1,3-Dioxo-1,3-dihydro-2H-isoindol-2-yl)pentyl]oxy}ethyl methanesulfonate

A solution of 4 ml (0.0515 mol) of methanesulfonyl chloride in 57 ml of dichloromethane was added slowly to a solution of 13.61 g (0.0491 mol) of Intermediate 91 and 7.5 ml (0.0540 mol) of triethylamine in 100 ml of dichloromethane at 0-5°C. The mixture was stirred 15 minutes at 0-5°C and at room temperature for 4 hours. After this reaction time, was diluted with dichloromethane and the obtained solution was washed with NaHCO₃ (4% aqueous solution) (x2) and with water, dried (MgSO₄), and concentrated under reduced pressure. The obtained residue was purified by column chromatography on silica gel using hexane/ethylacetate (1:1 to 1:2) as eluents. The title compound was obtained as an oil. (12.51 g, 71.7%).

### Intermediate 93'

### 2-(5-{2-[Methyl(2-phenylethyl)amino]ethoxy}pentyl)-1H-isoindole-1,3(2H)-dione

To a solution of 3.0 g (0.00844 mol) of Intermediate 92 and 1.35 ml (0.00928 mol) of N-methyl-phenethylamine in 21 ml of dried dimethylformamide, 0.82 g (0.00976 mol) of NaHCO₃ were added. The reaction mixture was stirred at 70°C for 17 hours. After this time, the reaction mixture was cooled to room temperature and the solvent was removed under reduced pressure. The obtained residue was treated with water and extracted with ethyl acetate (x2). The organic extracts were combined, washed with water (x2) and brine, dried (MgSO₄), and concentrated. The title compound was obtained as an oil which was purified by column chromatography on silica gel using chloroform/methanol (50:1 to 15:1) as eluents. Te title compound was obtained as an oil (2.22 g, 66.7%).

### Intermediate 94

### 5-{2-[methyl(2-phenylethyl)amino]ethoxy}pentan-1-amine

To a solution of 2214 mg (5.612 mmol) of Intermediate 93 in 21.3 ml of ethanol, 0.407 ml (8.374 mmol) of hydrazine monohydrate were added. The reaction mixture was stirred at room temperature for 6 hours and then at 60°C for 16 hours. The reaction mixture was cooled to room temperature and 0.204 ml (4.187 mmol) of hydrazine monohydrate were added. The stirring was continued at room temperature for 68 hours. The solvent was removed under reduced pressure. The residue was treated with chloroform and filtered. The solid was discarded, the filtrate was concentrated and the oil obtained was dissolved in a mixture of diethyl ether/dichloromethane 5:1. The obtained solution was washed with 1 N NaOH (x2), water and brine, then was extracted with 2N HCl (x3). The acidic extracts were combined and basified with solid K₂CO₃. The basic solution was extracted with chloroform (x3). The organic extracts were combined, dried (MgSO₄), and concentrated. The obtained residue was purified by column chromatography on silica gel performing a gradient elution using chloroform/methanol (15:1) → chloroform/methanol/ammonium hydroxide (90:10:1 to 80:20:2) as eluents. The title compound was obtained as an oil (635 mg, 42.8%).

### Intermediate 95

### 8-(Benzyloxy)-5-{(1R)-1-{[tert-butyl(dimethyl)silyl]oxy}-2-[(5-{2-[methyl(2-phenyl-ethyl)amino]ethoxy}pentyl)amino]ethyl}quinolin-2(1H)-one

To a solution of 1164 mg (2.381 mmol) of 8-(benzyloxy)-5-((1R)-2-bromo-1-{[tert-butyl(dimethyl)silyl]oxy}ethyl)quinolin-2(1H)-one (US20040059116) and 629 mg (2.378 mmol) of Intermediate 105 in 3.4 ml of anhydrous dimetyl sulfoxide, 400 mg (4.75 mmol) of NaHCO₃ and 70.6 mg (0.4712 mmol) of Nal were added. The mixture was stirred at 120°C for 1 hour, under N₂ atmosphere. The reaction mixture was cooled to room temperature, treated with water and extracted with ethyl acetate (x2). The organic extracts were combined, washed with water (x2) and brine, dried (MgSO₄), and concentrated to dryness. The residue was purified by column chromatography on silica gel performing a gradient elution using chloroform/methanol (15:1 → 10:1 → 4:1) as eluents. Appropiate fractions were combined and concentrated to dryness. The title compound was obtained as an oil. (646 mg, 40.4%).

### Intermediate 96

### 8-(Benzyloxy)-5-{(1R)-1-hydroxy-2-[(5-{2-[methyl(2-phenylethyl)amino]ethoxy}pentyl)amino]ethyl}quinolin-2(H)-one

To a solution of 640 mg (0.952 mmol) of Intermediate 95 in 6.8 ml of tetrahydrofuran, 451 mg (1.429 mmol) of tetrabutylammonium fluoride trihydrate were added. The mixture was stirred at 40°C, under nitrogen atmosphere, for 1 hour. The solvent was removed under reduced pressure. The residue was treated with water and extracted with ethyl acetate (x2). The organic extracts were combined and washed with water (x2) and brine, dried (MgSO₄), and concentrated to dryness. The residue was purified by column chromatography on silica gel performing a gradient elution using chloroform/methanol (15:1 to 4:1) → chloroform/methanol-ammonium hydroxide 90:10:1 to 80:20:2) as eluents. The title compound was obtained as an oil. (468 mg, 88.1%).

### Example 20

### 8-Hydroxy-5-{(1R)-1-hydroxy-2-[(5-{2-[methyl(2-phenylethyl)amino]ethoxy}pentyl) amino]ethyl}quinolin-2(1H)-one

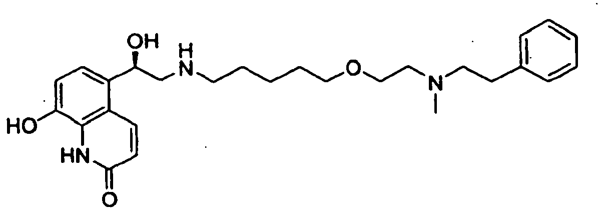

To a solution of 462 mg (0.828 mmol) of Intermediate 96 in 56 ml of methanol, 46 mg of . Pd/C (10%) were added. The mixture was hydrogenated (H₂ balloon pressure) at room temperature following the reaction advance by HPLC-MS. The total reaction time was 151 hours (31 hours with magnetical stirring and 120 hours in stand-by under the H₂ balloon pressure). The catalyst was filtered and the solvent was removed by concentration under reduced pressure. The obtained oil was purified by column chromatography on silica gel using chloroform/methanol/ammonium hydroxide (80:20:1 to 80:20:2) as eluents. The title compound was obtained as dry foam (298 mg, 77%).

¹H-NMR (300 MHz, DMSO-*d*₆) δ ppm: 1.19-1.51 (m, 6H); 2.22 (s, 3H); 2.45-2.60 (m, 6H); 2.60-2.73 (m, 4H); 3.31 (t, *J* =6.3 Hz, 2H); 3.4 (t, *J* =5.9 Hz, 2H); 5.00 (dd, *J* =7.5, 4.5 Hz, 1H); 6.48 (d, *J* =9.9 Hz, 1H); 6.89 (d, *J* =8.2 Hz, 1H); 7.05 (d, *J* =8.0 Hz, 1H); 7.11-7.29 (m, 5H); 8.16 (d, *J* =10.2 Hz, 1H).
MS: 468 [M+1]⁺

### Intermediate 97

### 3-(Cyclopentylthio)benzoic acid

To a solution of 2830 mg (11.97 mmol) of Intermediate 63 in 100 ml of tetrahydrofuran and 100 ml of water, 590 mg (24.63 mmol) of LiOH were added. The mixture was stirred for 4 hours at room temperature. The solvent was evaporated. The obtained residue was treated with 50 ml of water and the aqueous solution was acidified (pH=3) with 2N HCl. The acidic solution was extracted with chloroform (x2). The extracts were combined, washed with water and brine, dried (MgSO₄), and concentrated to dryness to give the title product. (2.56 g, 96.3%).

### Intermediate 98

### 3-(Cyclopentylthio)-N-methylbenzamide

To a solution of 2.564 g (11.53 mmol) of Intermediate 97 in 100 ml of chloroform (ethanol free), three drops of anhydrous dimethylformamide were added. The solution was cooled to 0-5°C and 1.56 ml (18.44 mmol) of oxalyl chloride dissolved in 10 ml of chloroform (ethanol free) were added. The reaction mixture was stirred 15 minutes at 0-5°C and 2 hours at room temperature. A new amount of oxalyl chloride (0.50 ml, 5.91 mmol) was added at 0-5°C, and the reaction mixture was stirred at room temperature for 72 hours. After this reaction time, solvents were evaporated under reduced pressure. Chloroform (ethanol free) was added to the residue and concentrated again (x2). The obtained residue was dissolved in 60 ml of anhydrous tetrahydrofuran and cooled to -30°C. At this temperature, 40 ml (80 mmol) of commercially available 2M solution of methyl amine in tetrahydrofuran, diluted with 40 ml of anhydrous tetrahydrofuran, were slowly added. The reaction mixture was left to warm to room temperature and stirred for 2 hours. The solvent was evaporated. The obtained residue was dissolved in chloroform and the solution was washed with water (x2), dried (MgSO₄), and concentrated. Chloroform was added to the residue and concentrated again (x2) to obtain the title product (2.72 g, 100%).

### Intermediate 99

### [3-(Cyclopentylthio)benzyl]methylamine

To a solution of 2010 mg (8.540 mmol) of Intermediate 98 in 171 ml of anhydrous tetrahydrofuran, under N₂ atmosphere, 25.7 ml (25.7 mmol) of Borane-Tetrahydrofuran complex (1M solution in tetrahydrofuran) were added slowly at room temperature. The reaction mixture was stirred 30 minutes at room temperature and refluxed for 3 hours. After this reaction time, the mixture was cooled to room temperature and stirred overnight. Methanol (13.7 ml) was added drop by drop. The mixture was refluxed for 30 minutes. After cooling to room temperature solvents were evaporated. The obtained residue was dissolved in 342 ml of methanol, 2.85 ml of concentrated aqueous HCl were slowly added, and the mixture was refluxed for 30 minutes. Solvents were evaporated. The obtained residue was treated with 427 ml of water and extracted with diethyl ether (142 ml) to remove some impurities. The acidic aqueous phase was separated and basified with solid K₂CO₃. The basic solution was extracted with chloroform (x3). The organic extracts were combined, dried (MgSO₄), and concentrated. The title compound was obtained as an oil (1613 mg, 85.3%).

### Intermediate 100

### 2-(6-{2-[(3-(Cyclopentylthio)benzyl](methyl)amino]ethoxy}hexyl)-1H-isoindole-1,3(2H)-dione

To a solution of 3.175 g (8.596 mmol) of Intermediate 4 and 2.093 g (9.455 mmol) of Intermediate 99, in 21 ml of dried dimethylformamide, 830 mg (9.885 mmol) of NaHCO₃ were added and the mixture was vigorously stirred at 70°C for 17 hours (under N₂ atmosphere). The reaction mixture was cooled to room temperature, the solvent was removed under reduced pressure and the residue was treated with water and extracted with ethyl acetate (x2). The organic extracts were combined, washed with water (x2) and brine, dried (MgSO₄), and concentrated. The obtained product was purified by column chromatography on silica gel using chloroform → chloroform/methanol (50:1) as eluents. Appropriate fractions were combined and concentrated. The title compound was obtained as an oil (3.594 g, 84.5%).

### Intermediate 101

### 2-(6-{2-[[3-(Cyclopentylsulfinyl)benzyl](methyl)amino]ethoxy}hexyl)-1H-isoindole-1,3(2H)-dione

To a stirred solution of 3.588 g of Intermediate 100 (purity 87%, 6.310 mmol) in 31.4 ml of MeOH, a solution of 1.352 g (6.321 mmol) of NalO₄ in 31.4 ml of water was added drop by drop. A white solid was formed during the addition. The reaction mixture was stirred at room temperature for 18 hours. After this reaction time, the mixture was diluted with water and extracted with ethyl acetate (x2). The organic extracts were combined and washed with water (x2) and brine, dried (MgSO₄), and concentrated. The obtained residue was purified by column chromatography on silica gel using chloroform/methanol (60:1 → 75:2) as eluents. The title product was obtained as an oil. (1.698 g, 52.7%).

### Intermediate 102

### 6-{2-[[3-(cyclopentylsulfinyl)benzyl](methyl)amino]ethoxy}hexan-1-amine

To a solution of 1.692 g (3.313 mmol) of Intermediate 101 in 12.5 ml of ethanol, 0.50 ml (10.308 mmol) of hydrazine monohydrate were added. The mixture was stirred at 60°C for 4 hours. A white solid was formed during the process. After this reaction time, the mixture was cooled to room temperature and 123 ml of chloroform were added. The mixture was stirred at room temperature for 1 hour. The solid was filtered and washed with diethyl ether. The solid was discarded and the filtrates were combined. The filtrates solution was slowly stirred overnight and the solid formed was filtered. The solid was discarded and the filtrate concentrated to dryness to give the title product (1065 mg, 84.5%) which was used in the next step without further purification.

### Intermediate 103

### 8-(Benzyloxy)-5-{(1R)-1-{[tert-butyl(dimethyl)silyl]oxy}-2-[(6-{2-[[3-(cyclopentyl-sulfinyl)benzyl](methyl)amino]ethoxy}hexyl)amino]ethyl}quinolin-2(1H)-one

To a solution of 1064 mg (2.178 mmol) of 8-(benzyloxy)-5-((1R)-2-bromo-1-{[tert-butyl(dimethyl)silyl]oxy}ethyl)quinolin-2(1H)-one (US20040059116) and 891 mg (purity 93%, 2.177 mmol) of Intermediate 102 in 3.1 ml of anhydrous dimethyl sulfoxide, 366 mg (4.356 mmol) of NaHCO₃ and 65 mg (0.433 mmol) of Nal were added. The mixture was stirred at 120°C for 1 hour, under N₂ atmosphere. The reaction mixture was cooled to room temperature, treated with water and extracted with ethyl acetate (x2). The organic extracts were combined, washed with water (x2) and brine, dried (MgSO₄) and concentrated to dryness. The residue was purified by column chromatography on silica gel using chloroform/methanol (15:1) as eluents. Appropiate fractions were combined and concentrated to dryness. The title compound was obtained as an oil. (990 mg, 57.7%).

### Intermediate 104

### 8-(Benzyloxy)-5-{(1R)-2-[(6-{2-[[3-(cyclopentylsulfinyl)benzyl](methyl)amino]ethoxy} hexyl)amino]-1-hydroxyethyl}quinolin-2(1H)-one

To a solution of 984 mg (purity 92%, 1.148 mmol) of Intermediate 103 in 9 ml of tetrahydrofuran, 541 mg (1.715 mmol) of tetrabutylammonium fluoride trihydrate were added. The mixture was stirred at 40°C, under nitrogen atmosphere, for 1 hour. The solvent was removed under reduced pressure. The residue was treated with water and extracted with ethyl acetate (x2). The organic extracts were combined and washed with water (x2) and brine, dried (MgSO₄), and concentrated to dryness. The residue was purified by column chromatography on silica gel performing a gradient elution using chloroform/methanol (9:1 to 4:1) → chloroform/methanol/ammonium hydroxide (90:10:1) as eluents. The title compound was obtained as an oil. (491 mg, 63.4 %).

### Example 21

### 5-{(1R)-2-[(6-{2-[[3-(Cyclopentylsulfinyl)benzyl](methyl)amino]ethoxy}hexyl)amino]-1-hydroxyethyl}-8-hydroxyquinolin-2(1H)-one

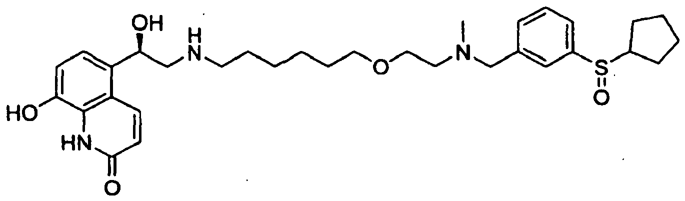

To a solution of 345 mg (0.512 mmol) of Intermediate 104 in 17 ml of dried dichloromethane, under N₂ atmosphere, 1 ml (1 mmol) of Boron Trichloride (1M solution in dichloromethane) was added at 0-5°C. The reaction mixture was stirred 5 minutes at 0-5°C, and 30 minutes at room temperature. The reaction advance was monitored by HPLC-MS. A new amount, 0.5 ml (0.5 mmol) of Boron Trichloride (1 M solution in dichloromethane) was added at 0-5°C. The reaction mixture was stirred 5 minutes at 0-5°C, and 30 minutes at room temperature. The reaction advance was monitored by HPLC-MS.

The reaction mixture was cooled to 0-5°C and 17 ml of NaHCO₃ (4% aqueous solution) were slowly added. The mixture was stirred 5 minutes at 0-5°C and 5 minutes at room temperature. The organic phase was separated and the aqueous phase was extracted with dichloromethane. The organic extracts were combined, dried (MgSO₄), and concentrated. The residue was purified by column chromatography on silica gel performing a gradient elution using chloroform/methanol (9:1 to 4:1) → chloroform/methanol/ammonium hydroxide (90:10:0.5 to 80:20:2) as eluents. A fraction of 37 mg enriched in the title compound (HPLC-MS: 55%) was obtained.

Several fractions, obtained in equivalent assays, enriched in the title compound, were combined to obtain 100 mg of a residue which was purified by column chromatography on silica gel (Varian Bond Elut Si 5g) performing a gradient elution using chloroform → chloroform/methanol (75:1 to 15:1) → chloroform/methanol/ammonium hydroxide (90:10:0.5 to 80:20:3). Appropiate fractions were combined and concentrated, 31 mg of the title compound were obtained.

¹H-NMR (300 MHz, METHANOL-*d*4) δ ppm: 1.31-1.44 (m, 4H), 1.48-1.86 (m, 11H), 1.91-2.08 (m, 1H), 2.25 (s, 3H), 2.56-2.64 (m, 2H), 2.68-2.78 (m, 2H), 2.83-3.0 (m, 2H), 3.25-3.35 (m, 1H), 3.39-3.46 (m, 2H), 3.53-3.60 (m, 2H), 3.67 (s, 2H), 5.23 (dd, *J* =9.20, 3.71 Hz, 1H), 6.63 (d, *J* =9.89 Hz, 1H), 6.92 (d, *J* =8.24 Hz, 1H), 7.10-7.23 (m, 1H), 7.19 (d, *J* =8.24 Hz, 1H) , 7.46-7.60 (m, 3H), 8.36 (d, *J* =9.89 Hz, 1H).
HPLC-MS: 78% title compound (MS: 584 [M+1]⁺ ); 22% impurity (MS: 568 [M+1]⁺)

### Intermediate 105

### Ethyl N-{[(3-{[[(benzyloxy)carbonyl](methyl)amino]methyl}phenyl)amino]carbonyl} glycinate

To a solution of 7.52 g (27.82 mmol) of Intermediate 27 in 100 ml of dried dichloromethane, 3.65 ml (31.95 mmol) of ethyl 2-isocyanatoacetate were added drop by drop. The mixture was stirred at room temperature for 1 hour. After this reaction time 6.25 ml of methanol were carefully added and the mixture was stirred at room temperature for 30 minutes. Solvents were evaporated and the residue was purified by column chromatography on silica gel using dichloromethane/diethyl ether (1:1) as eluent. Appropiate fractions were combined to give the title compound (10.65 g, 95.8%).

### Intermediate 106

### N-{[(3-{[[(Benzyloxy)carbonyl](methyl)amino]methyl}phenyl)amino]carbonyl}glycine

To a solution of 5.168 g (12.94 mmol) of Intermediate 116 in 35 ml of ethanol, 18 ml of 2N aqueous NaOH solution (36.0 mmol) were added. The mixture was stirred at room temperature for 4.5 hours. After this reaction time solvents were evaporated. The residue was treated with 100 ml of water and the obtained solution was acidified with 2N HCl solution. The acidic solution was extracted with ethyl acetate. The organic extracts were combined, washed with brine, dried (MgSO₄), and concentrated to dryness. The title product was obtained as a yellow dry foam (4.63 g, 96.2%).

### Intermediate 107

### 3-{3-[(Methylamino)methyl]phenyl}imidazolidine-2,4-dione

A mixture of 2.96 g (7.98 mmol) of Intermediate 106 in 13 ml of water and 4.15 ml (47.4 mmol) of aqueous 35% HCl (density 1.19 g/ml) was stirred at 140°C for 18 hours. After this reaction time, the mixture was cooled to room temperature and washed with a small volume of diethyl ether. The acidic solution was neutralized (pH=7) with 2N NaOH and the neutral solution was washed with dichloromethane. The aqueous solution was concentrated to dryness under reduced pressure. The obtained residue was treated with ethanol and filtered. The filtrate was concentrated and the residue was dissolved in ethanol and concentrated again. The obtained residue was dissolved in chloroform (ethanol free) and concentrated to dryness to obtain a dry foam. This product was purified by column chromatography on silica gel using chloroform/methanol/ammonium hydroxide (90:10:1) as eluent. The title product was obtained as a white solid (free base, 1.17g, 66.8%).

### Intermediate 108

### 2-{[tert-Butyl(dimethyl)silyl]oxy}ethanol

To a solution of 2 g (32.22 mmol) of ethane-1,2-diol in 50 ml of dried tetrahydrofuran, 1.29 g (32.25 mmol) of sodium hydride (60% in mineral oil) were added in several portions. The mixture was stirred for 1 hour at room temperature. After this time a solution of tert-butylchlorodimethylsilane (4.9 g, 32.51 mmol) in 15 ml of dried tetrahydrofuran was slowly added. The reaction mixture was stirred at room temperature for 2 hours. After this time, the reaction mixture was diluted with 120 ml of diethyl ether and treated with 120 ml of NaHCO₃ (4% aqueous solution). The organic phase was separated, washed with NaHCO₃ (4% aqueous solution), water and brine, dried (MgSO₄), and concentrated to dryness. The title product was obtained as an oil (5.51, 97%).

### Intermediate 109

### {2-[(6-Bromohexyl)oxy]ethoxy}(tert-butyl)dimethylsilane

A mixture of 5.51 g (31.25 mmol) of Intermediate 108, 15 ml (97.5 mmol) of 1,6-dibromohexane, 23.4 ml of 32% (w/v) NaOH aqueous solution and 0.2 g (0.62 mmol) of tetrabutylammonium bromide, was vigorously stirred for 7 days at room temperature. After this reaction time the reaction mixture was partitioned between hexane and water. The organic phase was washed with water, dried (MgSO₄), and concentrated. The residue was purified by column chromatography on silica gel performing a gradient elution using mixtures of increasing polarity of hexane/chloroform, starting from hexane 100% (to elute the excces of 1,6-dibromohexane) and finishing with chloroform 100%. Appropiate fractions were combined and concentrated to obtain the title product as an oil. (5.33 g, 50.3%).

### Intermediate 110

### 13,13,14,14-tetramethyl-1-phenyl-2,9,12-trioxa-13-silapentadecane

A mixture of 0.85 g (7.86 mmol) of benzyl alcohol, 5.13 g (15.12 mmol) of Intermediate 109, 5.9 ml of 32% (w/v) NaOH aqueous solution and 76 mg (0.24 mmol) of tetrabutylammonium bromide, was vigorously stirred for 72 hours at room temperature. After this reaction time the mixture was treated with water and extracted with diethyl ether. The ether phase was dried (MgSO₄), and concentrated. The obtained residue was purified by column chromatography on silica gel using hexane/ethyl acetate (10:0.5) as eluent. Appropiate fractions were combined and concentrated to obtain the title product as an oil (0.941 g, 32.7%).

### Intermediate 111

### 2-{[6-(Benzyloxy)hexyl]oxy}ethanol

To a solution of 0.462 g (1.26 mmol) of intermediate 110 in 25 ml of dry tetrahydrofuran, 676 mg (2.14 mmol) of tetrabutylammonium fluoride trihydrate were added. The mixture was stirred for 1.5 hours at 40°C and 72 hours at room temperature. After this reaction time the solvent was evaporated and the residue was treated with water and extracted with ethyl acetate (x2). The organic phases were combined, washed with water and brine, dried (MgSO₄) and concentrated. The obtained residue was purified by column chromatography on silica gel eluting with hexane/ethyl acetate (6:1 → 4:1). The title product was obtained as an oil (0.225 g, 70.8%).

**Intermediate 112**

### 2-{[6-(Benzyloxy)hexyl]oxy}ethyl methanesulfonate

To a solution of 1.017 g (4.03 mmol) of Intermediate 111 and 0.76 ml (5.48 mmol) of triethylamine in 13 ml of dried dichloromethane, at 0-5°C, a solution of 0.41 ml (5.25 mmol) of methanesulfonyl chloride in 4.5 ml of dried dichloromethane was slowly added. The mixture was stirred 15 minutes at 0-5°C and 6 hours at room temperature. After this reaction time, the mixture was diluted with 50 ml of dichloromethane. The obtained solution was washed with NaHCO₃ (4% aqueous solution) (2 x 50 ml) and water, dried (MgSO₄), and concentrated to dryness. The title product was obtained as an oil (1.232 g, 92.5%).

### Intermediate 113

### 3-(3-{[(2-{[6-(Benzyloxy)hexyl]oxy}ethyl)(methyl)amino]methyl}phenyl)imidazoildine-2,4-dione

To a solution of 1.232 g (3.73 mmol) of Intermediate 112 in 7 ml of dried dimethylformamide, 0.902 g (4.11 mmol) of Intermediate 107 and 0.36 g (4.29 mmol) of NaHCO₃ were added. The reaction mixture was stirred 18 hours at 70°C under N₂ atmosphere. After this reaction time the solvent was removed by concentration under reduced pressure. The residue was treated with water and the obtained aqueous solution extracted with ethyl acetate (x2). The organic extracts were combined, washed with water and brine, dried (MgSO₄), and concentrated. The obtained residue was purified by column chromatography on silica gel using dichloromethane /methanol (95:5) as eluent. Appropiate fractions were combined and concentrated to obtain the title product (1.08 g, 63.9%).

### Intermediate 114

### 3-(3-{[{2-[(6-Hydroxyhexyl)oxy]ethyl}(methyl)amino]methyl}phenyl)imidazolidine-2,4-dione

A solution of 590 mg (1.30 mmol) of Intermediate 113 in 27 ml of ethanol, was acidified by adding 3 ml of a solution 1.25 M of HCl (gas) in ethanol. After addition of 60 mg of Pd/C (10%) the mixture was hydrogenated at room temperature (H₂ balloon pressure) for 5 hours. After this reaction time, the catalyst was filtered and the filtrate was concentrated to dryness under reduced pressure to obtain the title product (576 mg) as a clorhidrate salt in quantitative yield.

### Intermediate 115

### 6-{2-[[3-(2,5-dioxoimidazolidin-1-yl)benzyl](methyl)amino]ethoxy}hexyl methanesulfonate

To a solution of 950 mg (2.6 mmol) of Intermediate 114 and 0.91 ml (6.5 mmol) of triethylamine in 18 ml of dried dichloromethane, 0.23 ml (3.0 mmol) of methanesulfonyl chloride were added drop by drop. The reaction mixture was stirred at room temperature for 24 hours. After this reaction time, the mixture was diluted with dichloromethane and the obtained solution was washed with NaHCO₃ (4% aqueous solution). The organic phase was separated using an ISOLUTE^{®} Phase Separator and concentrated. The obtained residue was purified by column chromatography on silica gel using dichloromethane/methanol (30:1 → 15:1) as eluents. Appropiate fractions were combined and concentrated to obtain the title product. (355 mg, 30.8%).

### Intermediate 116

### 3-(3-{[{2-[(6-{[(2R)-2-(2,2-dimethyl-4H-1,3-benzodioxin-6-yl)-2-hydroxyethyl]amino} hexyl)oxy]ethyl}(methyl)amino]methyl}phenyl)imidazolidine-2,4-dione

To a solution of 355 mg (0.80 mmol) of Intermediate 115 in 4.5 ml of dried dimethylformamide, 242 mg (1.08 mmol) of (1R)-2-amino-1-(2,2-dimethyl-4H-1,3-benzodioxin-6-yl)ethanol (WO 02/070490) and 259 mg (0.80 mmol) of tetrabutylammonium bromide were added. The mixture was stirred at room temperature for 72 hours. After this reaction time, the major part of the solvent was removed by concentration under reduced pressure. The obtained residue was diluted with dichloromethane and the obtained solution was washed with water. The organic phase was separated using an ISOLUTE^{®} Phase Separator and concentrated. The obtained residue was purified by column chromatography on silica gel (using a Varian Bond Elut Si 10g) using dichloromethane/methanol/ammonium hydroxide (100:6:1) as eluents. Appropiate fractions were combined and concentrated to obtain the title product. (171 mg, 37.4 %).

### Example 22

### 3-(3-{[(2-{[6-({(2R)-2-Hydroxy-2-[4-hydroxy-3-(hydroxymethyl)phenyl]ethyl}amino) hexyl]oxy}ethyl)(methyl)aminolmethyl}phenyl)imidazolidine-2,4-dione

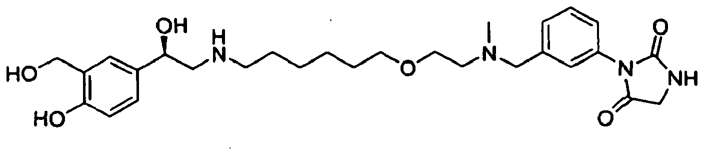

A solution of 198 mg (0.348 mmol) of Intermediate 116 in 9.27 ml of glacial acetic acid and 2.55 mol of water is heated at 70°C for 60 minutes. After this reaction time, solvents were evaporated. Cyclohexane was added to the residue and concentrated (x3). Chloroform (ethanol free) was added to the residue and concentrated again. The obtained residue was purified by column chromatography on silica gel (Varian Bond Elut Si 10 g) using chloroform/methanol/ammonium hydroxide (90:10:1) as eluents. Appropiate fractions were combined and concentrated to dryness to obtain the title compound (free base, 88 mg, 47.8%).

¹H-NMR (400 MHz, DMSO-d6) δ ppm: 1.22-1.32 (m, 4H); 1.34-1.42 (m, 2H); 1.42-1.52 (m, 2H); 2.17 (s, 3H); 2.47-2.60 (m, 6H); 3.28-3.44 (m, 2H); 3.45-3.51 (t, 2H); 3.54 (s, 2H); 4.06 (s, 2H); 4.46 (s, 2H); 4.47-4.53 (m, 1H); 4.89-4.98 (br. s., 1H); 6.67-6.69 (d, 1H); 6.95-7.0 (m, 1H); 7.18-7.24 (m, 1H); 7.24-7.32 (m, 3H); 7.37-7.42 (m, 1H).
MS: 529 [M+1]⁺

### Pharmaceutical Compositions

The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.
A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.
Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitably powder base (carrier substance) such as lactose or starch. Use of lactose is preferred.

Each capsule or cartridge may generally contain between 2µg and 150 µg of each therapeutically active ingredient. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92/09322) and rotatable frustums (e. g. WO 92/00771), all having cavities which have to be filled with powder from the container. Other multi dose devices have measuring slides (e. g.US 5201308 and WO 97/00703) or measuring plungers with a local or circumferential recess to displace a certain volume of powder from the container to a delivery chamber or an air conduit e. g. EP 0505321, WO 92/04068 and WO 92/04928.

Reproducible dose measuring is one of the major concerns for multi dose inhaler devices. The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (e. g. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with.
Such atomisers are described, for example, in PCT Patent Application No. WO 91/14468 and International Patent Application No. WO 97/12687, reference here being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetrafluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants eg oleic acid or lecithin and cosolvens eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µ, preferably 2-5µ. Particles having a size above 20µ are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving a high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate. Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

Each dosage unit contains suitably from 1 µg to 100 µg, and preferably from 5 µg to 50 µg of a β2-agonist according to the invention.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day.

The compositions of the invention can optionally comprise one or more additional active substances which are known to be useful in the treatment of respiratory disorders, such as PDE4 inhibitors, corticosteroids or glucocorticoids and/or anticholinergics.

Examples of suitable PDE4 inhibitors that can be combined with β2-agonists are denbufylline, rolipram, cipamfylline, arofylline, filaminast, piclamilast, mesopram, drotaverine hydrochloride, lirimilast, roflumilast, cilomilast, 6-[2-(3,4-Diethoxyphenyl)thiazol-4-yl]pyridine-2-carboxylic acid, (R)-(+)-4-[2-(3-Cyclopentyloxy-4-methoxyphenyl)-2-phenylethyl]pyridine, N-(3,5-Dichloro-4-pyridinyl)-2-[1-(4-fluorobenzyl)-5-hydroxy-1H-indol-3-yl]-2-oxoacetamide, 9-(2-Fluorobenzyl)-N6-methyl-2-(trifluoromethyl)adenine, N-(3,5-Dichloro-4-pyridinyl)-8-methoxyquinoline-5-carboxamide, N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride, 4-[6,7-Diethoxy-2,3-bis(hydroxymethyl)naphthalen-1-yl]-1-(2-methoxyethyl)pyridin-2(1H)-one, 2-carbomethoxy-4-cyano-4-(3-cyclopropylmethoxy-4-difluroromethoxyphenyl)cyclohexan1-one, *cis* [4-cyano-4-(3-cyclopropylmethoxy-4-difluoromethoxyphenyl)cyclohexan-1-ol, ONO-6126 (Eur Respir J 2003, 22(Suppl. 45): Abst 2557) and the compounds claimed in the PCT patent applications number WO03/097613 and PCT/EP03/14722 and in the Spanish patent application number P200302613.

Examples of suitable corticosteroids and glucocorticoids that can be combined with β2-agonists are prednisolone, methylprednisolone, dexamethasone, naflocort, deflazacort, halopredone acetate, budesonide, bectomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, deprodone propionate, fluticasone propionate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Examples of suitable M3 antagonists (anticholinergics) that can be combined with β2-agonists are tiotropium salts, oxitropium salts, flutropium salts, ipratropium salts, glycopyrronium salts, trospium salts, revatropate, espatropate, 3-[2-Hydroxy-2,2-bis(2-thienyl)acetoxy]-1-(3-phenoxypropyl)-1-azoniabicyclo[2.2.2]octane salts, 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoeihyl)piperidin-1-yl]-2(R)-[3,3-difluorocyclopent-1 (R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

The combinations of the invention may be used in the treatment of respiratory diseases, wherein the use of bronchodilating agents is expected to have a beneficial effect, for example asthma, acute or chronic bronchitis, emphysema, or Chronic Obstructive Pulmonary Disease (COPD).

The active compounds in the combination, i.e. the β2-agonist of the invention and the PDE4 inhibitors, corticosteroids or glucocorticoids and/or anticholinergics may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers, however, any other form or parenteral or oral application is possible. Here, the application of inhaled compositions embodies the preferred application form, especially in the therapy of obstructive lung diseases or for the treatment of asthma.

Additional suitable carriers for formulations of the active compounds of the present invention can be found in Remington: The Science and Practice of Pharmacy, 20th Edition, Lippincott Williams & Wilkins, Philadelphia, Pa., 2000. The following non-limiting examples illustrate representative pharmaceutical compositions of the invention.

### Formulation Example 1 (Oral suspension)

| **Ingredient** | **Amount** |
|---|---|
| β2 adrenergic receptor agonist | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| β2 adrenergic receptor agonist | 1 mg |
| Lactose | 150 mg |
| Magnesium stearate | 3 mg |

### Formulation Example 3 (Gelatin cartridge for inhalation)

| **Ingredient** | **Amount** |
|---|---|
| β2 adrenergic receptor agonist (micronized) | 0,2 mg |
| Lactose | 25 mg |

### Formulation Example 4 (Formulation for inhalation with a DPI)

| **Ingredient** | **Amount** |
|---|---|
| β2 adrenergic receptor agonist (micronized) | 15 mg |
| Lactose | 3000 mg |

### Formulation Example 5 (Formulation for a MDI)

| **Ingredient** | **Amount** |
|---|---|
| β2 adrenergic receptor agonist (micronized) | 10 g |
| 1,1,1,2,3,3,3-heptafluoro-n-propane | q.s. to 200 ml |

### Biological Assays

The compounds of this invention, and their pharmaceutically-acceptable salts, exhibit biological activity and are useful for medical treatment. The ability of a compound to bind to the β adrenergic receptors, as well as its selectivity, agonist potency, and intrinsic activity can be demonstrated using Tests A to E below, or can be demonstrated using other tests that are known in the art.

### TEST A

### Human Adrenergic ß1 and ß2 Receptor Binding Assays

The study of binding to human adrenergic β1 and β2 receptors was performed using commercial membranes prepared from Sf9 cells where they are overexpressed (Perkin Elmer).
The membrane suspensions (16 µg/well for beta1 and 5µg/well for beta2) in assay buffer, 75mM Tris/HCl with 12.5mM MgCl₂ and 2mM EDTA pH=7.4, were incubated with 0.14nM ³H-CGP12177 (Amersham) and different concentrations of the test compounds, in a final volume of 250 µl, in GFC Multiscreen 96 well plates (Millipore) pretreated with + 0.3% PEI. Non specific binding was measured in the presence of 1µM propanolol. Incubation was for 60 minutes at room temperature and with gentle shaking. The binding reactions were terminated by filtration and washing with 2.5 volumes of Tris/HCl 50mM pH=7.4. The affinity of each test compound to the receptor was determined by using at least six different concentrations ran in duplicate. IC₅₀ values were obtained by non-linear regression using SAS.
Selected compounds of this invention were found to have IC₅₀ values less than 25 nM for β₂ receptor and more than 140 nM for β₁ receptor

### TEST B

### Human Adrenergic ß3 Receptor Binding Assay

Membranes prepared from Human SK-N-MC neurotumor cells from the American Type Culture Collection were used as the source of β3 receptor. The cells were grown, and the membranes prepared following the methods described in P.K. Curran and P.H. Fishman, Cell. Signal, 1996, 8 (5), 355-364.

The assay procedure as detailed in the mentioned publication can be summarized as follows: the SK-N-MC cell line expresses both β1 and β3 receptor and for that reason, for selective binding to β3, membranes were incubated with 1nM ¹²⁵I-CYP ((-)-3-[¹²⁵I]lodocyanopindolol (Amersham)) and 0.3µM CGP20712A (a β1 antagonist) in 50mM HEPES, 4mM MgCl2 and 0.4% bovine serum albumin, pH=7.5 (assay buffer), and different concentrations of the test compounds. The final volume of the assay was 250µl. Non specific binding was defined by 100µM alprenolol. The samples were incubated 90 minutes at 30°C with shaking.
The binding reactions were terminated by filtration through Whatman GF/C membranes, prewet in assay buffer at 4°C, using a BRANDEL M-24 harvester. The filters were washed three times with 4 ml each of 50 mM Tris/HCl and 4mM MgCl₂ pH 7.4, and the radioactivity, retained in the filters, measured.
The affinity of each test compound to the receptor was determined by using at least eight different concentrations ran in duplicate. IC₅₀ values were obtained by non-linear regression using SAS. Exemplified compounds of this invention were found to have IC₅₀ values more than 1200 nM for β3 receptor.

### TEST C

### ACTIVITY AND DURATION OF ACTION OF ß₂ AGONISTS ON ISOLATED GUINEA-PIG TRACHEAL RINGS

### Test compounds and products

The test compounds were dissolved in distilled water. Some of them needed to be dissolved using 10% polyethylene glycol 300 and a few drops of HCl 0.1 N. Isoprenaline hemisulfate was supplied by Sigma (code 15752) and dissolved in distilled water. Stock solutions were then diluted in Krebs Henseleit solution (NaCl 118mM, KCl 4.7mM, CaCl₂ 2.52mM, MgSO₄ 1.66 mM, NaHCO₃ 24.9mM, KH₂PO₄ 1.18mM, glucose 5.55 mM, sodium pyruvate 2mM) to prepare different concentration ranges per each compound.

### Experimental procedure

The activity of compounds in tracheal ring was assessed according a previously described procedure (Cortijo et al., Eur J Pharmacol. 1991, 198, 171-176). Briefly, adult, male guinea pigs (400-500g) were sacrificed by a blow to the head with immediate exsanguinations (abdominal aorta). Tracheas were excised and placed into Krebs solution in a Petri dish. The adherent connective tissue was dissected away and the lumen gently flushed with Krebs solution. Each trachea was dissected into single rings. First, cotton thread was attached to the cartilage at both sides of the smooth muscle. The rings were opened by cutting through the cartilage on the side opposite to the smooth muscle band. Then, one end of the ring was attached to the strain gauge and the other end was attached to the organ-bath under a resting tension of 1g and changes in tension of the rings were measured using an isometric transducer. The bath contained Krebs solution gassed with 5% CO₂ in oxygen at 37°C. Tissues were then left for one hour to stabilize.

At the beginning of the experience, isoprenaline was administered at a concentration of 0.1µM to test ring relaxation. Rings were then washed twice with Krebs solution and left to recover for 15-30 min. For each compound, a range of increasing and accumulative concentrations (0.01 nM to 0.1 µM) was administered with a maximum waiting time of 30 min between each administration. After the maximum concentration (achievement of complete relaxation), ring preparations were washed every 15 min during 1 hour. At the end of the experiment, 0.1 µM of isoprenaline was administered to each preparation to produce maximum relaxation back.

### Determination of agonist activity

Agonist activity was determined by assaying accumulative increasing concentrations of test compounds prepared in the Krebs solution. The magnitude of each response was measured and expressed as a percentage versus the maximum relaxation induced by isoprenaline. Potency values for the test compounds were expressed in absolute terms (concentration required to induce a 50% relaxation, EC₅₀). Selected compounds of this invention were found to have EC₅₀ values less than 1.0 nM.

### TEST D

### HISTAMINE-INDUCED BRONCHOSPASM IN CONSCIOUS GUINEA PIGS

### Test compounds and products

The test compounds were dissolved in distilled water. Some of them need to be dissolved using 10% polyethylene glycol 300. Histamine HCl was supplied by Sigma (code H 7250) and dissolved in distilled water.

### Experimental procedure

Male guinea-pigs (325-450g) were supplied by Harlan (Netherlands), and maintained at a constant temperature of 22±2 °C, humidity 40-70% with 10 cycles of room air per hour. They were illuminated with artificial light in 12 hour cycles (from 7h am to 7h pm). A minimum of 5 days acclimatization period was left before animals were dosed with test compounds. The animals were fasted for 18 hours before the experiment with water *ad libitum.*

Five animals per session were placed in a methacrylate box (47x27x27 cm) which was connected to an ultrasonic nebuliser (Devilbiss Ultraneb 2000, Somerset, PA, USA). The test compounds (β₂-adrenergic agonists) were administered by aerosol during 30 seconds at concentrations between 0.1 and 1000 µg/ml. Either 5 or 180 min after test compounds administration, 250 ug/ml of histamine were nebulized during 30 s to induce a bronchospasm. The time elapsed from histamine administration to first bronchospasm was recorded up to a maximum of 5 min.

### Determination of activity, duration of action and calculations

For each treatment and dosage the percentage of delay of bronchospasm was calculated using the following formula: % delay bronchospasm = [(t'- t) / (tₘₐₓ - t)] x 100, where tₘₐₓ = maximum observation time (5 min), t= time elapsed to first bronchospasm in the animals of control group, and t'= time elapsed to first bronchospasm in compound-treated animals. The EC₅₀ was defined as the concentration dose causing a 50 % delay of bronchospasm. An EC₅₀ was calculated for compounds administered 5 minutes or 180 min before histamine challenge and were named EC₅₀ at 5 min and EC₅₀ at 180 min, respectively.

Duration of action of test compounds was determined by the ratio EC₅₀ 5 min/ EC₅₀ 180 min. The compounds exhibiting a ratio EC₅₀5 min/ EC₅₀180 min below 100 were considered long-acting.

A selected group of exemplified compounds of this invention show EC₅₀ values lower than 10 µg/ml at 5 min and the ratios between EC₅₀ at 5 min and EC₅₀ at 180 min are below 100.

### TEST E

### ACETYLCHOLINE INDUCED BRONCHOCONSTRICTION IN GUINEA PIG.

### Test compounds and products

The test compounds were dissolved in distilled water. Some of them need to be dissolved using a maximum of 10% polyethylene glycol 300. Acetylcholine HCl was supplied by Sigma (code A 6625) and dissolved in saline solution.

### Experimental procedure

Male guinea-pigs (450-600g) were supplied by Harlan (Netherlands), and maintained at a constant temperature of 22±2 °C, humidity 40-70% with 10 cycles of room air per hour. They were illuminated with artificial light in 12 hour cycles (from 7h am to 7h pm). A minimum of 5 days acclimatization period was left before animals were dosed with test compounds. The animals were fasted 18 hours before the experiment with water *ad libitum*

Guinea pigs were exposed to an aerosol of a test compound or vehicle. These aerosols were generated from aqueous solutions using a Devilbiss nebuliser (Model Ultraneb 2000, Somerset, PA, SA). A mixture of gases (CO₂=5%, O₂=21%, N₂=74%) was flown through the nebuliser at 3 Uminute. This nebuliser was connected to a methacrylate box (17x17x25 cm) where the animals were placed one per session. Every guinea pig remained in the box for a total of 10 minutes. Aerosols were generated at 0 and 5 minutes during 60 seconds each one (approximately 5 mL of solution was nebulised).

Aerosol concentrations between 0.1 and 300 µg/ml of the compounds were administered. The bronchoprotective effects of test compounds were evaluated one hour or twenty four hours post-dose with a Mumed PR 800 system.

### Determination of bronchoprotective effect and calculations

The guinea pigs were anesthetized with an intramuscular injection of ketamine (43.75 mg/kg), xylazine (83.5 mg/kg), and acepromazine (1.05 mg/kg) at a volume of 1 ml/kg. After the surgical site was shaved, a 2-3 cm midline incision of the neck was made. The jugular vein was isolated and cannulated with a polyethylene catheter (Portex Ld.) to allow an intravenous bolus of acetylcoline (10 and 30 µg/kg iv) at 4-min intervals. The carotid artery was cannulated and the blood pressure was measured by a Bentley Tracer transducer. The trachea was dissected and cannulated with a teflon tube and connected at a pneumotachograph Fleisch for measuring the airflow. Animal was ventilated using an Ugo Basile pump, with a volume of 10 ml/kg at a rate of 60 breaths/min. The transpulmonary pressure was measured with an esophageal cannula (Venocath-14, Venisystems) connected to Celesco transducer. Once the cannulations were completed a Mumed pulmonary measurement computer program enabled the collection of pulmonary values. The baseline values were within the range of 0.3-0.9 mL/cm H₂O for compliance and within the range of 0.1-0.199 cm H₂O/mL per second for lung resistance (R_{L}).

The bronchocoprotective effect of inhaled compounds was determined with the concentration of the test compound causing a 50 % of inhibition of bronchoconstriction (EC₅₀) induced by acetylcholine at 30 µg/kg iv

### Determination of duration of action

A selected group of exemplified compounds of this invention show ratios between EC₅₀ at 24hr and EC₅₀ at 1 hr below 10.

## Claims

1. A compound of formula (I): 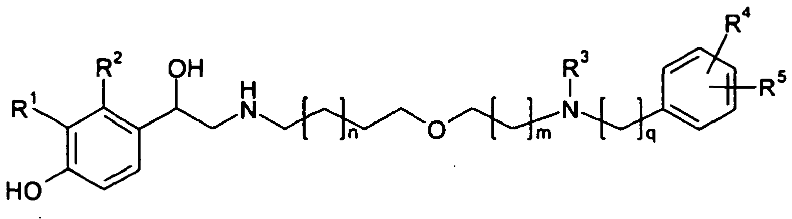 wherein:
• R¹ is a group selected from -CH₂OH, -NHC(O)H and
• R² is a hydrogen atom; or
• R¹ together with R² form the group -NH-C(O)-CH=CH- wherein the nitrogen atom is bound to the carbon atom in the phenyl ring holding R¹ and the carbon atom is bound to the carbon atom in the phenyl ring holding R²
• R³ is selected from hydrogen atoms and C₁₋₄alkyl groups
• R⁴ is selected from hydrogen and halogen atoms or groups selected from -SO-R⁶, -SO₂-R⁶, -NR⁷-CO-NHR⁸, -CO-NHR⁷, hydantoino, C₁₋₄alkyl, C₁₋₄alkoxy and -SO₂NR⁹R⁸
• R⁵ is selected from hydrogen atoms, halogen atoms and C₁₋₄alkyl groups
• R⁶ is a C₁₋₄alkyl group or C₃₋₈ cycloalkyl group
• R⁷ is independently selected from hydrogen atoms and C₁₋₄alkyl groups
• R⁸ is independently selected from hydrogen atoms and C₁₋₄alkyl groups
• or R⁷ and R⁸ form the group -CH=CH-C(O)- wherein the carbon atom forming part of the ethylenic bond is bound to the nitrogen atom which is also bound to the carbon atom in the phenyl ring and the carbon atom of the carbonyl group is bound to the nitrogen atom which is bound to the hydrogen atom
• R⁹ is independently selected from hydrogen atoms and C₁₋₄alkyl groups
• m is 1 or 2
• n, is 0, 1, 2, 3 or 4
• q is 0, 1 or 2
or a pharmaceutically-acceptable salt, solvate or stereoisomer thereof.

2. A compound according to claim 1 wherein m+n is 4, 5 or 6.

3. A compound according to any preceding claim wherein n is 3.

4. A compound according to any preceding claim wherein q is 0 or 1.

5. A compound according to any preceding claim wherein R¹ is a group -CH₂OH and R² is a hydrogen atom; or R¹ together with R² form the group -NH-C(O)-CH=CH- wherein the nitrogen atom is bound to the carbon atom in the phenyl ring holding R¹ and the carbon atom is bound to the carbon atom in the phenyl ring holding R²

6. A compound according to any preceding claim wherein R³ is a methyl group.

7. A compound according to any preceding claim wherein R⁴ is selected from halogen atoms or groups selected from -SO-R⁶, -SO₂-R⁶, -NR⁷-CO-NHR⁸, -CO-NHR⁷, hydantoino and -SO₂NR⁹R⁸_{.}

8. A compound according to any preceding claim wherein R⁵ is a hydrogen atom.

9. A compound selected from the group consisting of:
3-[{2-[(6-{[2-hydroxy-2-(8-hydroxy-2-oxo-1,2-dihydroquinolin-5-yl)ethyl]amino}hexyl)oxy]ethyl}(methyl)amino]benzamide
4-{2-[(6-{2-[(2,6-dichlorobenzyl)(methyl)amino]ethoxy}hexyl)amino]-1-hydroxyethyl}-2-(hydroxymethyl)phenol
3-[(2-{[6-({2-hydroxy-2-[4-hydroxy-3-(hydroxymethyl)phenyl]ethyl}amino)hexyl]oxy}ethyl)(methyl) amino]benzamide
5-{2-[(6-{2-[(2,6-dichlorobenzyl)(methyl)amino]ethoxy}hexyl)amino]-1-hydroxyethyl}-8-hydroxyquinolin-2(1H)-one
N-(3-{[{2-[(6-{[2-hydroxy-2-(8-hydroxy-2-oxo-1,2-dihydroquinolin-5-yl)ethyl]amino}hexyl)oxy]ethyl}(methyl)amino]methyl} phenyl)urea
N-(3-{[{2-[(6-{[(2R)-2-hydroxy-2-(8-hydroxy-2-oxo-1,2-dihydroquinolin-5-yl)ethyl]amino}hexyl)oxy]ethyl}(methyl)amino]methyl}phenyl)urea
N-(3-{[(2-{[6-({2-hydroxy-2-[4-hydroxy-3-(hydroxymethyl)phenyl]ethyl}amino)hexyl]oxy}ethyl)(methyl)amino]methyl}phenyl) urea
4-{2-[(6-{3-[(2,6-dichlorobenzyl)(methyl)amino]propoxy}hexyl)amino]-1-hydroxyethyl}-2-(hydroxymethyl)phenol
3-[(3-{[6-({2-hydroxy-2-[4-hydroxy-3-(hydroxymethyl)phenyl]ethyl}amino) hexyl]oxy}propyl)(methyl)amino]benzamide
3-[(3-{[6-({(2R)-2-hydroxy-2-[4-hydroxy-3-(hydroxymethyl)phenyl]ethyl}amino)hexyl]oxy}propyl)(methyl)amino]benzamide
3-{[{2-[(6-{[2-hydroxy-2-(8-hydroxy-2-oxo-1,2-dihydroquinolin-5-yl)ethyl]amino}hexyl)oxy]ethyl}(methyl)amino]methyl}benzamide
3-{[(2-{[6-({2-hydroxy-2-[4-hydroxy-3-(hydroxymethyl)phenyl]ethyl}amino)hexyl]oxy}ethyl)(methyl)amino]methyl}benzami de
1-(3-{[{2-[(6-{[2-hydroxy-2-(8-hydroxy-2-oxo-1,2-dihydroquinolin-5-yl)ethyl]amino}hexyl)oxy]ethyl}(methyl)amino]methyl}phenyl)pyrimidine-2,4(1H,3H)-dione
5-{(1R)-2-[(6-{2-[[3-(Cyclopentylsulfonyl)benzyl](methyl)amino]ethoxy}hexyl)amino]-1-hydroxyethyl}-8-hydroxyquinolin-2(1H)-one
5-[2-((6-[2-(benzylamino)ethoxy]hexyl)amino)-1-hydroxyethyl]-8-hydroxyquinolin-2(1H)-one
3-[(3-{[6-({(2Rr2-[3-(Formylamino)-4-hydroxyphenyl]-2-hydroxyethyl}amino)hexyl]oxy}propyl)(methyl)amino]benzamide
4-[2-({6-[2-(Benzylamino)ethoxy]hexyl}amino)-1-hydroxyethyl]-2-(hydroxymethyl)phenol
1-(3-{[{2-[(6-{[(2R)-2-hydroxy-2-(8-hydroxy-2-oxo-1,2-dihydroquinolin-5-yl)ethyl]amino}hexyl)oxy]ethyl}(methyl)amino]methyl}phenyl)pyrimidine-2,4(1H,3H)-dione
N-(tert-Butyl)-3-{[{2-[(6-{[(2R)-2-hydroxy-2-(8-hydroxy-2-oxo-1,2-dihydroquinolin-5-yl)ethyl]amino}hexyl)oxy]ethyl}(methyl)amino]methyl}benzenesulfonamide 8-Hydroxy-5-{(1R)-1-hydroxy-2-[(5-{2-[methyl(2-phenylethyl)amino]ethoxy}pentyl)amino]ethyl}quinolin-2(H)-one
5-{(1R)-2-[(6-{2-[[3-(Cyclopentylsulfinyl)benzyl](methyl)amino]ethoxy}hexyl)amino]-1-hydroxyethyl}-8-hydroxyquinolin-2(1H)-one
3-(3-{[(2-{[6-({(2R)-2-Hydroxy-2-[4-hydroxy-3-(hydroxymethyl)phenyl]ethyl}amino)hexyl]oxy}ethyl)(methyl)amino]methyl}phenyl)i midazolidine-2,4-dione
and pharmaceutically-acceptable salts and solvates thereof.

10. A pharmaceutical composition comprising a therapeutically effective amount of a compound according to anyone of claims 1 to 9 and a pharmaceutically acceptable carrier and optionally further comprising a therapeutically effective amount of one or more other therapeutic agents.

11. The pharmaceutical composition of claim 10 wherein the other therapeutic agent is a corticosteroid, an anticholinergic agent, or a PDE4 inhibitor

12. A combination comprising a compound according to anyone of claims 1 to 9 and one or more other therapeutic agents, as defined in claim 10 or 11.

13. A compound according to any one of claims 1 to 9 for use in treating a disease or pathological condition in a mammal associated with β2 adrenergic receptor activity.

14. A compound according to claim 13 wherein the disease or pathological condition is a pulmonary disease, pre-term labor, glaucoma, neurological disorders, cardiac disorders or inflammation, and preferably wherein said pulmonary disease is asthma or chronic obstructive pulmonary disease.

15. Use of a compound according to any one of claims 1 to 9, a composition according to any one of claims 10 or 11 or a combination according to claim 12 in the manufacture of a medicament for the treatment of a disease or pathological condition according to claim 13 or 14.

## Patentansprüche

1. Verbindung der Formel (I): 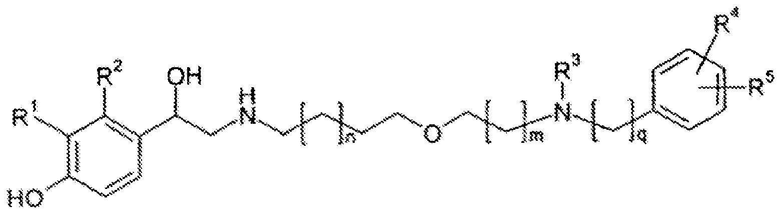 wobei
• R¹ eine Gruppe ist, ausgewählt aus -CH₂OH, -NHC(O)H und
• R² ein Wasserstoffatom ist; oder
• R¹ bildet gemeinsam mit R² die Gruppe -NH-C(O)-CH=CH-, wobei das Stickstoffatom an das Kohlenstoffatom im Phenylring, der R¹ trägt, gebunden ist, und das Kohlenstoffatom an das Kohlenstoffatom in dem Phenylring, der R² trägt, gebunden ist
• R³ ist ausgewählt aus Wasserstoffatomen und C₁₋₄-Alkylgruppen
• R⁴ ist ausgewählt aus Wasserstoff- und Halogenatomen, oder einer Gruppe, ausgewählt aus -SO-R⁶, -SO₂-R⁶, -NR⁷-CO-NHR⁸, -CO-NHR⁷, Hydantoino, C₁₋₄-Alkyl, C₁₋₄-Alkoxy und -SO₂NR⁹R⁸
• R⁵ ist ausgewählt aus Wasserstoffatomen, Halogenatomen und C₁₋₄-Alkylgruppen
• R⁶ ist eine C₁₋₄-Alkylgruppe oder C₃₋₈-Cycloalkylgruppe
• R⁷ ist unabhängig ausgewählt aus Wasserstoffatomen und C₁₋₄-Alkylgruppen
• R⁸ ist unabhängig ausgewählt aus Wasserstoffatomen und C₁₋₄-Alkylgruppen
• oder R⁷ und R⁸ bilden die Gruppe -CH=CH-C(0)-, wobei das Teil der ethylenischen Bindung bildende Kohlenstoffatom an das Stickstoffatom gebunden ist, welches ebenfalls an das Kohlenstoffatom in dem Phenylring gebunden ist, und das Kohlenstoffatom der Carbonylgruppe ist an das Stickstoffatom gebunden, welches an das Wasserstoffatom gebunden ist.
• R⁹ ist unabhängig ausgewählt aus Wasserstoffatomen und C₁₋₄-Alkylgruppen
• m ist 1 oder 2
• n ist 0, 1, 2, 3 oder 4
• q ist 0, 1 oder 2
oder ein pharmazeutisch verträgliches Salz, Solvat oder Stereoisomer davon.

2. Verbindung gemäß Anspruch 1, wobei m+n 4, 5 oder 6 ist.

3. Verbindung gemäß einem beliebigen vorstehenden Anspruch, wobei n 3 ist.

4. Verbindung gemäß einem beliebigen vorstehenden Anspruch, wobei q 0 oder 1 ist.

5. Verbindung gemäß einem beliebigen vorstehenden Anspruch, wobei R¹ eine Gruppe -CH₂OH ist und R² ein Wasserstoffatom ist; oder R¹ gemeinsam mit R² bildet die Gruppe -NH-C(O)-CH=CH-, wobei das Stickstoffatom an das Kohlenstoffatom im Phenylring, der R¹ trägt, gebunden ist, und das Kohlenstoffatom ist an das Kohlenstoffatom in dem Phenylring, der R² trägt, gebunden.

6. Verbindung gemäß einem beliebigen vorstehenden Anspruch, wobei R³ eine Methylgruppe ist.

7. Verbindung gemäß einem beliebigen vorstehenden Anspruch, wobei R⁴ ausgewählt ist aus Halogenatomen oder Gruppen, ausgewählt aus -SO-R⁶, -SO₂-R⁶, -NR⁷-CO-NHR⁸, -CO-NHR⁷, Hydantoino und -SO₂NR⁹R⁸.

8. Verbindung gemäß einem beliebigen vorstehenden Anspruch, wobei R⁵ ein Wasserstoffatom ist.

9. Verbindung, ausgewählt aus der Gruppe, bestehend aus:
3-[{2-[(6-{[2-Hydroxy-2-(8-hydroxy-2-oxo-1,2-dihydrochinolin-5-yl)ethyl]amino}hexyl)oxy]ethyl}(methyl)amino]benzamid
4-{2-[(6-{2-[(2,6-Dichlorbenzyl)(methyl)amino]ethoxy}hexyl)amino]-1-hydroxyethyl}-2-(hydroxymethyl)phenol
3-[(2-{[6-({2-Hydroxy-2-[4-hydroxy-3-(hydroxymethyl)phenyl]ethyl}amino)hexyl]oxy}ethyl)(methyl)amino]benzamid
5-{2-[(6-{2-[(2,6-Dichlorbenzyl)(methyl)amino]ethoxy}hexyl)amino]-1-hydroxyethyl}-8-hydroxychinolin-2(1H)-on
N-(3-{[{2-[(6-{[2-Hydroxy-2-(8-hydroxy-2-oxo-1,2-dihydrochinolin-5-yl)ethyl]amino}hexyl)oxy]ethyl}(methyl)amino]methyl}phenyl)ha rnstoff
N-(3-{[{2-[(6-{[(2R)-2-Hydroxy-2-(8-hydroxy-2-oxo-1,2-dihydrochinolin-5-yl)ethyl]amino}hexyl)oxy]ethyl}(methyl)amino]methyl}phenyl)ha rnstoff
N-(3-{[(2-{[6-({2-Hydroxy-2-[4-hydroxy-3-(hydroxymethyl)phenyl]ethyl}amino)hexyl]oxy}ethyl)(methyl)amino]-methyl}phenyl)harnstoff
4-{2-[(6-{3-[(2,6-Dichlorbenzyl)(methyl)amino]propoxy}hexyl)amino]-1-hydroxyethyl}-2-(hydroxymethyl)phenol
3-[(3-{[6-({2-Hydroxy-2-[4-hydroxy-3-(hydroxymethyl)phenyl]ethyl}amino)hexyl]oxy}propyl)-(methyl)amino]benzamid
3-[(3-{[6-({(2R)-2-Hydroxy-2-[4-hydroxy-3-(hydroxymethyl)phenyl]ethyl}amino)hexyl]oxy}propyl)-(methyl)amino]benzamid
3-{[{2-[(6-{[2-Hydroxy-2-(8-hydroxy-2-oxo-1,2-dihydrochinolin-5-yl)ethyl]amino}hexyl)oxy]ethyl}(methyl)amino]methyl}benzamid
3-{[(2-{[6-({2-Hydroxy-2-[4-hydroxy-3-(hydroxymethyl)phenyl]ethyl}amino)hexyl]oxy}ethyl)-(methyl)amino]methyl}benzamid
1-(3-{[{2-[(6-{[2-Hydroxy-2-(8-hydroxy-2-oxo-1,2-dihydrochinolin-5-yl)ethyl]amino}hexyl)oxy]ethyl}(methyl)-amino]methyl}phenyl)pyrimidin-2,4(1H,3H)-dion
5-{(1R)-2-[(6-{2-[[3-(Cyclopentylsulfonyl)benzyl](methyl)amino]ethoxy}hexyl)amino] -1-hydroxyethyl}-8-hydroxychinolin-2(1H)-on
5-[2-({6-[2-(Benzylamino)ethoxy]hexyl}amino)-1-hydroxyethyl]-8-hydroxychinolin-2(1H)-on
3-[(3-{[6-({(2R)-2-[3-(Formylamino)-4-hydroxyphenyl]-2-hydroxyethyl}amino)hexyl]oxy}propyl)(methyl)amino]benzamid
4-[2-({6-[2-(Benzylamino)ethoxy]hexyl}amino)-1-hydroxyethyl]-2-(hydroxymethyl)phenol
1-(3-{[{2-[(6-{[(2R)-2-Hydroxy-2-(8-hydroxy-2-oxo-1,2-dihydrochinolin-5-yl)ethyl]amino}hexyl)oxy]ethyl}-(methyl)amino]methyl}phenyl)pyrimidin-2,4(1H,3H)-dion
N-(tert-Butyl)-3-{[{2-[(6-{[(2R)-2-hydroxy-2-(8-hydroxy-2-oxo-1,2-dihydrochinolin-5-yl)ethyl]amino}hexyl)oxy]ethyl}-(methyl)amino]methyl}benzolsulfonamid
8-Hydroxy-5-{(1R)-1-hydroxy-2-[(5-{2-[methyl(2-phenylethyl)amino]ethoxy}pentyl)amino]ethyl}chinolin-2(1H)-on
5-{(1R)-2-[(6-{2-[[3-(Cyclopentylsulfinyl)benzyl](methyl)amino]ethoxy}-hexyl)amino]-1-hydroxyethyl}-8-hydroxychinolin-2(1H)-on
3-(3-{[(2-{[6-({(2R)-2-Hydroxy-2-[4-hydroxy-3-(hydroxymethyl)phenyl]ethyl}amino)hexyl]oxy}ethyl)(methyl)-amino]methyl}phenyl)imidazolidin-2,4-dion
und pharmazeutisch verträgliche Salze und Solvate davon.

10. Pharmazeutische Zusammensetzung, umfassend eine therapeutisch wirksame Menge einer Verbindung gemäß einem beliebigen der Ansprüche 1 bis 9 und einen pharmazeutisch verträglichen Träger sowie gegebenenfalls des weiteren umfassend eine therapeutisch wirksame Menge eines oder mehrerer anderer therapeutischer Mittel.

11. Pharmazeutische Zusammensetzung gemäß Anspruch 10, wobei das andere therapeutische Mittel ein Corticosteroid, ein Anticholinergikum oder ein PDE4-Inhibitor ist.

12. Kombination, umfassend eine Verbindung gemäß einem beliebigen der Ansprüche 1 bis 9 und ein oder mehrere andere therapeutische Mittel, wie in Anspruch 10 oder 11 definiert.

13. Verbindung gemäß einem beliebigen der Ansprüche 1 bis 9 zur Verwendung in der Behandlung einer Krankheit oder eines Krankheitszustands in einem Säuger, assoziiert mit β2-adrenerger Rezeptor-Aktivität.

14. Verbindung gemäß Anspruch 13, wobei die Krankheit oder der Krankheitszustand eine Lungenkrankheit, vorzeitige Wehen, Glaukom, neurologische Störungen, Herzstörungen oder -entzündung ist, und vorzugsweise wobei die Lungenkrankheit Asthma oder chronisch-obstruktive Lungenkrankheit ist.

15. Verwendung einer Verbindung gemäß einem beliebigen der Ansprüche 1 bis 9, einer Zusammensetzung gemäß einem beliebigen der Ansprüche 10 oder 11, oder einer Kombination gemäß Anspruch 12 in der Herstellung eines Medikaments zur Behandlung einer Krankheit oder eines Krankheitszustands gemäß Anspruch 13 oder 14.

## Revendications

1. Composé de formule (I) : 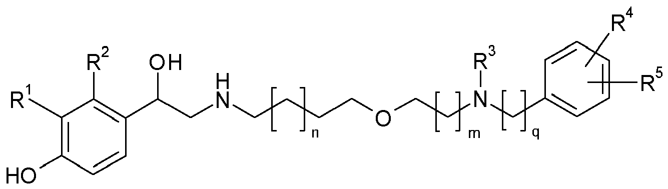 dans laquelle :
- R¹ représente un groupe choisi parmi ceux de formule -CH₂OH ou -NHC(O)H,
- et R² représente un atome d'hydrogène ;
- ou bien R¹ et R² représentent conjointement un groupe de formule -NH-C(O)-CH=CH- dont l'atome d'azote est lié à l'atome de carbone du groupe phényle qui porte l'entité symbolisée par R¹ et dont l'atome de carbone est lié à l'atome de carbone du groupe phényle qui porte l'entité symbolisée par R² ;
- R³ représente une entité choisie parmi un atome d'hydrogène et les groupes alkyle en C₁₋₄ ;
- R⁴ représente une entité choisie parmi les atomes d'hydrogène et d'halogène, les groupes symbolisés par -SO-R⁶ -SO₂-R⁶, -NR⁷-CO-NHR⁸, -CO-NHR⁷ ou -SO₂-NR⁹R⁸, et les groupes hydantoïno, alkyle en C₁₋₄ et alcoxy en C₁₋₄ ;
- R⁵ représente une entité choisie parmi les atomes d'hydrogène et d'halogène et les groupes alkyle en C₁₋₄ ;
- R⁶ représente un groupe alkyle en C₁₋₄ ou cycloalkyle en C₃₋₈ ;
- R⁷ représente indépendamment une entité choisie parmi un atome d'hydrogène et les groupes alkyle en C₁₋₄,
- et R⁸ représente indépendamment une entité choisie parmi un atome d'hydrogène et les groupes alkyle en C₁₋₄ ;
- ou bien R⁷ et R⁸ représentent conjointement un groupe de formule -CH=CH-C(O)- dont l'atome de carbone engagé dans la liaison éthylénique est lié à l'atome d'azote qui est aussi lié à un atome de carbone du groupe phényle et dont l'atome de carbone du groupe carbonyle est lié à l'atome d'azote lié à un atome d'hydrogène;
- R⁹ représente indépendamment une entité choisie parmi un atome d'hydrogène et les groupes alkyle en C₁₋₄ ;
- l'indice m vaut 1 ou 2 ;
- l'indice n vaut 0, 1, 2, 3 ou 4 ;
- et l'indice q vaut 0, 1 ou 2 ;
ou sel, solvat ou stéréisomère, pharmacologiquement admissible, d'un tel composé.

2. Composé conforme à la revendication 1, dans lequel la somme des indices m et n vaut 4, 5 ou 6.

3. Composé conforme à l'une des revendications précédentes, dans lequel l'indice n vaut 3.

4. Composé conforme à l'une des revendications précédentes, dans lequel l'indice q vaut 0 ou 1.

5. Composé conforme à l'une des revendications précédentes, dans lequel R¹ représente un groupe de formule -CH₂OH et R² représente un atome d'hydrogène, ou bien R¹ et R² représentent conjointement un groupe de formule -NH-C(O)-CH=CH- dont l'atome d'azote est lié à l'atome de carbone du groupe phényle qui porte l'entité symbolisée par R¹ et dont l'atome de carbone est lié à l'atome de carbone du groupe phényle qui porte l'entité symbolisée par R².

6. Composé conforme à l'une des revendications précédentes, dans lequel R³ représente un groupe méthyle.

7. Composé conforme à l'une des revendications précédentes, dans lequel R⁴ représente une entité choisie parmi les atomes d'halogène, les groupes symbolisés par -SO-R⁶, -SO₂-R⁶, -NR-CO-NHR⁸, -CO-NHR⁷ ou -SO₂-NR⁹R⁸, et le groupe hydantoïno.

8. Composé conforme à l'une des revendications précédentes, dans lequel R⁵ représente un atome d'hydrogène.

9. Composé choisi dans l'ensemble formé par les suivants:
- 3-[{2-[(6-{[2-hydroxy-2-(8-hydroxy-2-oxo-1,2-dihydroquinoléin-5-yl)-éthyl]amino}-hexyl)oxy]-éthyl}-(méthyl)amino]-benzamide
- 4-{2-[(6-{2-[(2,6-dichloro-benzyl)-(méthyl)amino]-éthoxy}-hexyl)-amino]-1-hydroxy-éthyl}-2-(hydroxy-méthyl)-phénol
- 3-[(2-{[6-({2-hydroxy-2-[4-hydroxy-3-(hydroxy-méthyl)-phényl]-éthyl}amino)-hexyl]oxy}-éthyl)-(méthyl)amino]-benzamide
- 5-{2-[(6-{2-[(2,6-dichloro-benzyl)-(méthyl)amino]-éthoxy}-hexyl)-amino]-l-hydroxy-éthyl}-8-hydroxy-quinoléine-2(1H)-one
- N-(3-{[{2-[(6-{[2-hydroxy-2-(8-hydroxy-2-oxo-1,2-dihydroquinoléin-5-yl)-éthyl]amino}-hexyl)oxy]-éthyl}-(méthyl)amino]méthyl}-phényl)-urée
- N-(3-{[{2-[(6-{[(2R)-2-hydroxy-2-(8-hydroxy-2-oxo-1,2-dihydro-quinoléin-5-yl)-éthyl] amino}-hexyl)oxy]-éthyl}-(méthyl)amino]-méthyl}-phényl)-urée
- N-(3-{[(2-{[6-({2-hydroxy-2-[4-hydroxy-3-(hydroxy-méthyl)-phényl]-éthyl}amino)-hexyl]oxy}-éthyl)-(méthyl)amino]méthyl}-phényl)-urée
- 4-{2-[(6-{3-[(2,6-dichloro-benzyl)-(méthyl)amino]-propoxy}-hexyl)amino]-1-hydroxy-éthyl}-2-(hydroxy-méthyl)-phénol
- 3-[(3-{[6-({2-hydroxy-2-[4-hydroxy-3-(hydroxy-méthyl)-phényl]-éthyl}amino)-hexyl]oxy}-propyl)-(méthyl)amino]-benzamide
- 3-[(3-{[6-({(2R)-2-hydroxy-2-[4-hydroxy-3-(hydroxy-méthyl)-phényl]-éthyl}amino)-hexyl]oxy}-propyl)-(méthyl)amino]-benzamide
- 3-{[{2-[(6-{[2-hydroxy-2-(8-hydroxy-2-oxo-1,2-dihydroquinoléin-5-yl)-éthyl]amino}-hexyl)oxy]-éthyl}-(méthyl)amino]méthyl}-benzamide
- 3-{[(2-{[6-({2-hydroxy-2-[4-hydroxy-3-(hydroxy-méthyl)-phényl]-éthyl}amino)-hexyl]oxy}-éthyl)-(méthyl)amino]méthyl}-benzamide
- 1-(3-{[{2-[(6-{[2-hydroxy-2-(8-hydroxy-2-oxo-1,2-dihydroquinoléin-5-yl)-éthyl]amino}-hexyl)oxy]-éthyl}-(méthyl)amino]méthyl}-phényl)-pyrimidine-2,4(1H,3H)-dione
- 5-((1R)-2-{[6-(2-{[3-(cyclopentyl-sulfonyl)-benzyl]-(méthyl)-amino}-éthoxy)-hexyl]amino}-1-hydroxy-éthyl)-8-hydroxy-quinoléine-2(1H)-one
- 5-[2-({6-[2-(benzyl-amino)-éthoxy]-hexyl}amino)-1-hydroxy-éthyl]-8-hydroxy-quinoléine-2(1H)-one
- 3-[(3-{[6-({(2R)-2-[3-(formyl-amino)-4-hydroxy-phényl]-2-hydroxy-éthyl}amino)-hexyl]oxy}-propyl)-(méthyl)amino]-benzamide
- 4-[2-({6-[2-(benzyl-amino)-éthoxy]-hexyl}amino)-1-hydroxy-éthyl]-2-(hydroxy-méthyl)-phénol
- 1-(3-{[{2-[(6-{[(2R)-2-hydroxy-2-(8-hydroxy-2-oxo-1,2-dihydro-quinoléin-5-yl)-éthyl]amino}-hexyl)oxy]-éthyl}-(méthyl)amino]-méthyl}-phényl)-pyrimidine-2,4(1H,3H)-dione
- N-tertiobutyl-3-{[{2-[(6-{[(2R)-2-hydroxy-2-(8-hydroxy-2-oxo-1,2-dihydroquinoléin-5-yl)-éthyl]amino}-hexyl)oxy]-éthyl}-(méthyl)amino]méthyl}-benzènesulfonamide
- 8-hydroxy-5-{(1R)-1-hydroxy-2-[(5-{2-[méthyl-(2-phényl-éthyl)-amino]-éthoxy}-pentyl)amino]-éthyl}-quinoléine-2(1H)-one
- 5-((1R)-2-{[6-(2-{[3-(cyclopentyl-sulfinyl)-benzyl]-(méthyl)-amino}-éthoxy)-hexyl]amino}-1-hydroxy-éthyl)-8-hydroxy-quinoléine-2(1H)-one
- 3-(3-{[(2-{[6-({(2R)-2-hydroxy-2-(4-hydroxy-3-(hydroxy-méthyl)-phényl]-éthyl}amino)-hexyl]oxy}-éthyl)-(méthyl)amino]-méthyl}-phényl)-imidazolidine-2,4-dione
et les sels et les solvats pharmacologiquement admissibles de ces composés.

10. Composition pharmaceutique comprenant, en une quantité à effet thérapeutique, un composé conforme à l'une des revendications 1 à 9, ainsi qu'un véhicule pharmacologiquement admissible, et comprenant en outre, en option, un ou plusieurs autre(s) agent(s) thérapeutique(s), en quantité(s) à effet thérapeutique.

11. Composition pharmaceutique conforme à la revendication 10, dans laquelle l'autre agent thérapeutique est un corticostéroïde, un agent anticholinergique ou un inhibiteur de phosphodiesterase 4.

12. Combinaison comprenant un composé conforme à l'une des revendications 1 à 9, et un ou plusieurs autre(s) agent(s) thérapeutique(s), tel(s) que défini(s) dans la revendication 10 ou 11.

13. Composé conforme à l'une des revendications 1 à 9, pour utilisation dans le traitement, chez un mammifère, d'une maladie ou d'un état pathologique associé(e) à l'activité des récepteurs adrénergiques β2.

14. Composé conforme à la revendication 13, pour lequel la maladie ou l'état pathologique est une affection pulmonaire, un accouchement avant terme, un glaucome, des troubles neurologiques, des troubles cardiaques ou une inflammation, ladite affection pulmonaire étant de préférence un asthme ou une bronchopneumopathie chronique obstructive.

15. Utilisation d'un composé conforme à l'une des revendications 1 à 9, d'une composition conforme à la revendication 10 ou 11, ou d'une combinaison conforme à la revendication 12, dans la fabrication d'un médicament conçu pour le traitement d'une maladie ou d'un état pathologique défini dans la revendication 13 ou 14.
